# EUROPEAN PATENT APPLICATION

(11) **EP 4 378 952 A1**
(43) Date of publication of application: **05.06.2024**
(21) Application number: 22848724.5
(22) Date of filing: 01.08.2022
(51) Int. Cl.: C07K 14/705, C07K 14/715, C07K 16/30, C07K 16/08, C12N 15/85, A61K 35/17

(54) **UNIVERSAL T CELL AND APPLICATION THEREOF**

(30) Priority: 30.07.2021 CN 202110877843
(71) Applicant: ST Phi Therapeutics Co., Ltd, Hangzhou, Zhejiang 310056 (CN)
(72) Inventor: LIU, Lingfeng, Hangzhou, Zhejiang 310052 (CN); ZHONG, Wenting, Hangzhou, Zhejiang 310052 (CN)
(74) Representative: Hoffmann Eitle
(86) International application number: PCT/CN2022/109558
(87) International publication number: WO 2023/006120

(57) **Abstract**

Provided are a universal T cell (CNK-UT) and an application thereof. The CNK-UT has the broad-spectrum ability to recognize and kill tumor cells and virus-infected cells.

## Description

### Technical Field

The present disclosure relates to the field of cell therapy, more specifically, to a universal T cell and use thereof, in particular to chimeric natural killer receptor-universal T cell (CNK-UT) and use thereof.

### Background

In cell therapy, immune cells are modified by genetic engineering technology to present tumor-associated antigens or express receptors that specifically recognize disease cells, which are exponentially amplified in vitro and then transfused back into patients to activate the immune system in vivo to attack tumor cells or directly specifically recognize and kill disease cells. In 2012, CD19-targeted CAR-T cells achieved the first targeted elimination of tumor cells in patients with B-cell leukemia in the history of human medicine, becoming another treatment technology that can truly cure leukemia after bone marrow stem cell transplantation treatment technology, opening a new era of cell therapy in precision medicine. This technology is expected to be applied to the treatment of various hematological tumors and solid tumors. However, the current clinical efficacy of conventional CAR-T in the treatment of solid tumors is not good, and the reasons are as follows: (1) the killing function of CAR-T is highly dependent on the recognition of tumor-associated antigen (TAA) by CAR structure, but due to the heterogeneity of solid tumors, there are great differences in the expression of target proteins on the surface of tumor cells; at present, a single targeted CAR-T cannot completely recognize and kill all tumor cells, leading to immune escape and recurrence and metastasis of tumors; (2) direct immunosuppressive effect of solid tumors, expressing PD-L1, B3H7, etc., directly inhibiting the activation of T cells; (3) in the immunosuppressive microenvironment of solid tumors, there are a large number of immunosuppressive cells, e.g., TAM, Treg, MDSC, and the design of conventional CAR-T is difficult to break through the limitations of immunosuppressive cells inside the tumor; (4) conventional CAR-T is personalized cell therapy, that is, T cells source the patient itself, and if the patient has received a large number of radiotherapy and chemotherapy, etc., immune system function is impaired, and therefore it is difficult to isolate a sufficient number of T cells from the patient's peripheral blood, even if they are isolated and modified, the proliferation and killing function of T cells are still very weak, so it is difficult to exert the therapeutic effect.

The immune system is highly plastic and has a near-limitless ability to detect invading viruses, bacteria, xenologous cells, and diseased cells. This extraordinary immune monitoring capability is achieved primarily through humoral immunity and cellular immunity, which includes two important molecular structures: immunoglobulins and T cell receptors (TCRs). TCR, the defining structure of T cells, is a transmembrane heterodimer consisting of α and β chains or δ and γ chains linked by disulfide bonds. In these strands, the complementary determinant region (CDR) determines the antigen to which the TCR will bind. In TCR, the TCRα and TCRβ subunits (or TCRγ and TCRδ in γδT cells) are responsible for identifying major histocompatibility complexes (MHCs)/antigen ligands.

The human MHCI gene region includes alleles at HLA-A, B, and C sites, encoding classical class I antigens (molecules), for example, HLA-A antigen, B antigen and C antigen, which is referred to as HLA-A, HLA-B and HLA-C. These antigen molecules are present on the surface of all somatic cells and bind to intracellular protein epitope peptides for recognition by the immune system. If cells produce mutant proteins, or foreign bacteria or viruses invade, the cells present these mutant proteins or heterologous protein epitopes, and immune cells will carry out immune attack and kill after recognition, thereby removing the diseased cells, bacteria and viruses.

Since that MHC molecules are key factors to present viral antigens and trigger the attack of immune system against viral pathogens, viruses have gradually formed a strategy of targeted inhibition of MHC molecules to avoid immune surveillance and clearance for evolution in millions of years. A typical example is human cytomegalovirus (HCMV), which has evolved alongside its host and achieved immune escape through many genomic functional proteins. The unique short (US) genomic region of HCMV encodes at least five glycoproteins (US2, US3, US6, US10, US11). These glycoproteins use special mechanisms to down-regulate MHC class Imolecules, thus preventing viral antigen presentation to cytotoxic T lymphocytes (CTL).

CMVUS glycoprotein could hijack the endoplasmic reticulum associated degradation (ER-associated degradation, ERAD) mechanism to inhibit MHC molecule-mediated viral antigen presentation, thus evading the immune surveillance system. ER-related degradation (ERAD) is a protein scavenging system. Proteins misfolded, misassembled or metabolically regulated in the endoplasmic reticulum are selectively dislocated from the endoplasmic reticulum to the cytoplasmic sol through a specific membrane penetration mechanism, and then degraded by the cytoplasmic ubiquitin proteasome system (UPS).

Both HCMV US2 and US 11 proteins are ER-resident type I integral membrane glycoproteins that co-select this ERAD pathway to promote the degradation of MHC class I heavy chains, thereby inhibiting MHC class I antigen presentation. Expression of either protein results in rapid degradation of newly synthesized MHC class I heavy chains. US2 and US11 bind to heavy chains through their luminal domains and recruit host cell proteins that extract polypeptides from the endoplasmic reticulum membrane by "pulling" the cytoplasmic tails of the heavy chains. After translocation into the cytoplasm, MHC class I molecules are ubiquitinated and degraded by the proteasome.

In addition to class I molecules, US2 also leads to the degradation of two proteins of the class II pathway, DR-α and DM-α, as well as HFE, a non-canonical major histocompatibility complex (MHC) class I protein involved in iron regulation.

The luminal domain of US2 is responsible for binding MHC class I and II molecules, the transmembrane domain (TM) and cytoplasmic domain (CT) interact with cellular composition of ERAD pathways, and facilitate translocation and promotion of enzymatic digestion of both class I and class II proteins. The cytoplasmic tail of US2 is sufficient to interact with signal peptidase (SPP), and SPP is an essential component of US2-dependent MHC I dislocation complex. In addition, US2 interacts with the endoplasmic reticulum-resident RING-type E3 ligase TRC8 through its TM domain, which also contributes to the ubiquitination and proteasomal degradation of US2 tail-anchored MHC I and II molecules.

In contrast, US11-induced MHC-I molecular degradation requires Derlin1 rather than SPP. The ER luminal domain of US11 interacts with the luminal domain of the MHC-1 heavy chain, while the TM domain of US11 binds Derlin-1. Therefore, the main function of US11 may be to deliver MHC-1 molecules to Derlin-1, and then induce their translocation to the cytosol for proteasomal degradation. Furthermore, US11 activates unfolded proteins. Through Derlin-1, US11 associates with TMEM129 as an ERAD RING E3 ligase and recruits Ube2J2 to ubiquitinate MHC-I prior to US11-induced degradation.

Rather than promoting the degradation of MHC proteins, US3 glycoprotein physically binds to peptide-loaded MHC class I heterodimers so that the class I complex remains in ER and inhibits the binding of invariant chains to class II DR-αβ dimers in ER, resulting in mislocalization of class II complex and reduced peptide loading. Therefore, US3 is capable of interfering with the intracellular transport and maturation of MHC class I molecules during the early stages of HCMV infection. US3 is an endoplasmic reticulum-resident membrane protein. Domain swapping experiments revealed that the luminal domain of US3 is sufficient for US3 to maintain the ER by itself, whereas both the luminal and transmembrane domains are required for the retention of MHC class I molecules in the ER.

In addition to MHC I molecules, US2 and US3 glycoproteins inhibit class II antigen presentation by destroying or eliminating the function of class II proteins. US2 binds to class II DR and leads to rapid and effective proteasome-mediated degradation of only the α-chain of II-like DRαβ complexes. US2 also leads to the degradation of DM α chain, which is a MHC II complex needed to load antigenic peptides into class II DR complexes. HCMV US3 binding to class II DRαβ heterodimers inhibits the binding of invariant chains (Ii), leading to intracellular mislocalization and reduced the peptide loading of DR complexes.

HCMV US10 encodes an endoplasmic reticulum glycoprotein, which also interacts with molecules presented by MHC class I antigens. US10 binds free class I heavy chains and delays their transport from the ER. However, US 10 does not affect US2 or US11.

The adenovirus gene product E3max 19K (E19) can also retain class I molecules in the secretory pathway and interfere with antigen presentation. E19 is also an endoplasmic reticulum resident glycoprotein, which can eliminate the cell surface transport of major histocompatibility complex class I (MHC-1) and MHC-I-related chains A and B (MICA/B). E3/19K includes three functional modules: a luminal domain for interaction with MHC-I and MICA/B molecules, a transmembrane domain, and a dilysine motif in the cytoplasmic tail that may return the Golgi apparatus to the ER. Studies have shown that a transmembrane domain (TMD) and ER return signal are required to ensure efficient ER localization, transport-inhibition of MHC-1 and MICA/B molecules, and proteasomal degradation. Non-identical with US2, US3, US10, and US11, the HCMV L protein US6 affects antigen presentation through a completely different strategy. In contrast to interacting with free class I heavy chains or fully assembled class I complex, US6 inhibits translocation of cytosolic peptides through the TAP complex (TAP1/2). US6 binds to the ER luminal side of TAP1 and causes a conformational change that prevents ATP binding. Residues 89-108 in the ER-luminal domain of US6 contribute to the binding of US6 to TAP and which are necessary and sufficient for this inhibition. The inhibition of TAP activity affects not only the expression of the classical MHC class I alleles, but also the expression of the nonclassical alleles HLA-C and HLA-G in fetal cytotrophoblast cells.

Acting as a HCMV US6 protein, HSV ICP47 expressed early in the infection cycle is dispensable for replication in vitro, and the same strategy could be applied to prevent class I molecules assembly. ICP47 blocks TAP-mediated peptide transport and binds tightly to the TAP1-TAP2 complex. A clue to the mechanism by which ICP47 blocks TAP is that it exhibits high species selectivity. Both HSV1 and HSV2I CP47 inhibit the TAP in simian, monkey, pig, dog and bovine, and have little effect on the TAP in mouse, rat, guinea pig or rabbit. ICP47 has an approximately 100-fold higher affinity for human TAP than for mouse TAP. ICP47 inhibits peptide binding to TAP but does not affect ATP binding. ICP47 has a 10-1000 folds higher affinity for TAP than most peptides which can acts as a competitive inhibitor of peptide binding to TAP, and is thought to bind directly to the peptide-binding site.

α/βT lymphocytes recognize peptide-MHC ligands by means of a multimeric protein ensemble termed the αβT cell antigen receptor (TCR) CD3 complex. The structure is consisted of a variable αβTCR dimer that binds to antigens and three invariant dimers (CD3γε, δε, and ζζ) involved in TCR. CD3 surface transport, stabilization, and signal transduction. αβT cell receptors (αβTCR) are expressed on most (approximately 95%) T cells and play a key role in T cell activation by recognizing major histocompatibility complex (MHC)-anchored antigens. Therefore, TCR-mediated T cell activation is a critical step in the pathogenesis of graft-versus-host disease (GVHD) during allogeneic hematopoietic cell transplantation (allo-HCT) and allogeneic CAR-T cell therapy.

The human leukocyte antigen (HLA) system or complex is a group of related proteins that are encoded by the human major histocompatibility complex (MHC) gene complex. These cell-surface proteins are responsible for the regulation of the immune system. In therapeutic transplant setting, "HLA mismatch" happens when the donor HLA on the allograft differs from the recipient. HLA mismatch leads to the activation of alloreactive T cells, which can cause acute cellular rejection (ACR) within six months of transplantation. Mismatched donor HLA antigens are also targets for the development of nascent donor-specific HLA antibodies (dnDSA) which play augmented roles in both acute and chronic transplant T cell rejection. Therefore, to generate universal T cells for safe allogenic infusion and therapeutic purpose, it is advisable to effectively block graft-versus-host disease (GVHD) by genetically disrupting the TCR. In addition, it is necessary to suppress HLA expression on the allogeneic T cell to reduce rejection of the recipient immune system on allogeneic T cell TCRαβ and/or HLA class I of the allogeneic T cells.

Therefore, it is necessary to develop new cell therapy methods in order to overcome the differences in tumor targets, tumor immunosuppressive environment, personalized treatment cell sources, inability to standardize and large-scale production, and low killing efficiency in existing cell therapies.

### Summary

The purpose of the present disclosure is to overcome the problems faced by the existing tumor cell therapy, such as diversity of tumor targets, tumor immunosuppressive environment, source of personalized treatment cells, inability to standardize and large-scale production, low killing efficiency, etc. One of the purposes of the present disclosure is to provide a universal T (UT) module, which can hijack ERAD mechanism to block TCR and MHC molecules in ER, prevent its transport and promote its translocation to the cytoplasm for ubiquitin and degradation through proteasome. This design may target any protein, including endogenous or exogenous proteins, to effectively inhibit expression and rapidly degrade for therapeutic purposes.

The concept of UT module is based on the discovery that viral ER resident glycoproteins can hijack ERAD regulatory mechanisms to inhibit/block MHC molecules assembly, transport or promote its ubiquitination and proteasome degradation, thereby inhibiting MHC-mediated viral antigen presentation to evade immune surveillance. Effective down-regulation of TCR will significantly inhibit TCR-mediated immune attack and reduce GVHD in T cell allogeneic transfusion. Including natural viral endoplasmic reticulum resident glycoprotein can further inhibit MHC molecules, thereby preventing peptide presentation to recipient CD8+ T cells and inhibiting immune recognition by allogeneic T cells. Therefore, the UT module can improve the compatibility and long-term persistence of allogeneic T cells after infusion. For the purpose of cell therapy, the general T cells with defective expression on the surface of αβTCR and MHC molecules can be further genetically engineered for therapeutic purposes.

Another purpose of the present disclosure is to provide a method, in which by introducing NK elements, especially optimized recombinant NK elements, such that T cells can be as efficient as NK cells to identify all virus-infected cells and tumor cells, further by optimizing the transduction element, thereby CNK-T cells can be efficiently activated and kill tumor cells. Since that NK targets include family member proteins such as MICA, MICB and ULBP1-6, which can be widely expressed in various tumor cells and cover different stages of tumor progression, CNK technology can effectively solve the off-target effect of a single CAR-T and eliminate the probability of tumor immune escape. Meanwhile, the chimeric adaptor introduced by CNK in the design can effectively transduce and amplify NK signals, break the limitations of immune checkpoints e.g., PD1 signals, efficiently activate T cells, and achieve the killing of tumor cells. The chimeric adaptor amplifies CNK-T cell signals, and is capable of resisting immunosuppression in the tumor environment, realizing the activation of T cells, and realizing the killing of tumor cells. Then CNK-T cells recognize targets through NK, and can also clear immunosuppressive cells e.g., MDSC. After the virus infects cells and expressing specific functional proteins, the assembly or transport of MHCI or directly promotes the directional degradation of MHCI molecules, thereby inhibiting the presentation of viral epitopes and producing immune escape.

The present disclosure redesigns viral elements targeting TCR molecules to achieve retention and directional degradation of TCR molecular in endoplasmic reticulum. In addition, the present disclosure also realizes expression inhibition or directional degradation of MHCI molecules by introducing specific viral elements, thereby realizing transformation of universal T cells.

In the present disclosure, it is verified that CNK-UT has broad-spectrum tumor identification and killing capabilities. The present disclosure further designs composite specific target CAR/CNK-UT products, demonstrating that CAR/CNK-UT products have more powerful killing and activation functions on tumor cells than conventional CAR-T. In animal experiments, CAR/CNK-UT products also have more efficient tumor clearance capabilities. UT technology realizes the allogeneic universal transformation, T cells source healthy donors, so as to realize the standardization and large-scale production of CNK-UT products, which can be prepared in advance, and ensure the killing against tumor cells and activation ability of T cells.

### Brief description of the drawings

Fig. 1 illustrates the four structures of CNK-UT multi-functional complex, wherein, Fig. 1A shows a basic CNK-UT multi-functional complex, Fig. 1B shows a CNK-UT multi-functional complex further comprising an MHC I-targeted binding protein molecular domain, Fig. 1C shows a CNK-UT multi-functional complex further comprising an MHC I-targeted binding proterin molecular domain and a chimeric adaptor, Fig. 1D shows a CNK-UT multi-functional complex further comprising an MHC I-targeted binding protein molecular domain and a receptor such as CAR, TCR or etc., targeting and killing a tumor cell.

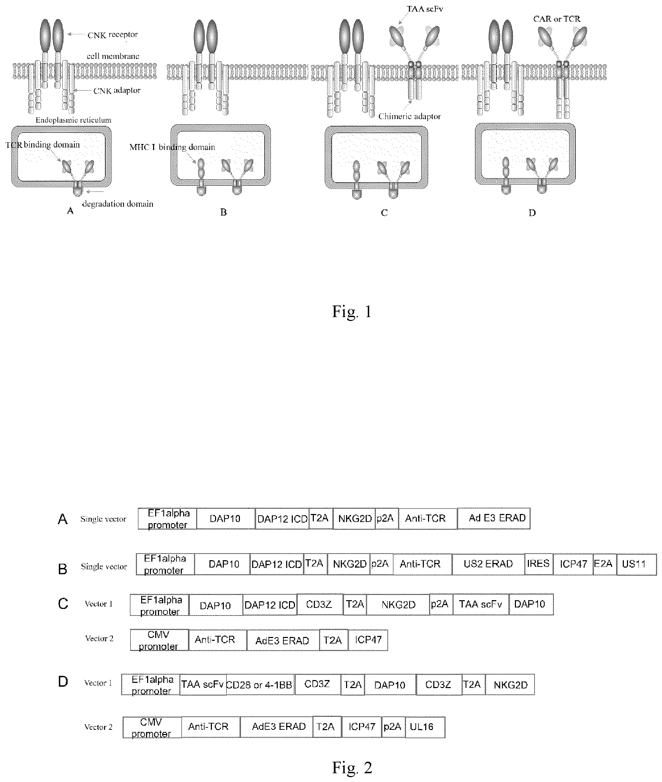

Fig. 2 illustrates the structures of four CNK-UT elements expressing CNK-UT multi-functional complexes. As shown in Fig. 2A, a single lentiviral expression vector with an EF1α promoter promotes the expression of one combination of CNK-UT elements: DAP10-DAP12 ICD-T2A-NKG2D-p2A-anti-TCR-AdE3 ERAD; as shown in Fig. 2B, a single lentiviral expression vector with an EF1α promoter promotes the expression of one combination of CNK-UT elements: DAP10-DAP12 ICD -T2A-NKG2D-p2A-anti-TCR-US2 ERAD; as shown in Fig. 2C, a single lentiviral expression vector using both EF1α and CMV promoters respectively regulate the multi-gene expressions of DAP10-CD3ζ-T2A-NKG2D-p2A-ant-TAA scFv-DAP10 and anti-TCR-AdE3 ERAD-E2A-AdE3, therby achieving the expression of multiple CNK-UT functional elements by a single vector; as shown in Fig. 2D, two different lentiviral expression vectors are used to respectively regulate the expression of anti-TAA scFv-CD28/4-1BB-CD3ζ-T2A-CD3ζ-p2A-NKG2D and anti-TCR-AdE3 ERAD-T2A-AdE3, by co-transfection of T cells, the expressions of multiple CNK-UT functional elements in one single T cell may be achieved.
Fig. 3 illustrates the results of flow cytometry detection of a basic phenotype of CNK-UT (DAP10-CD3ζ-T2A-NKG2D-p2A-anti-TCR scFv-AdE3) cells.
Fig. 4 illustrates the results of recognition and specific killing of CNK-UT cells against the human colonic adenoma cell line HT29, wherein Fig. 4A shows the expression of different NK target proteins in the human colonic adenoma cell line HT29, and Fig. 4B shows the effective killing against HT29 and activation ability of CNK-UT cells.
Fig. 5 illustrates the recognition and specific killing of MDA-MB453 by CNK-UT cells, wherein Fig. 5A shows the expression of different NK target proteins in the triple negative breast cancer cell line MDA-MB453, and Fig. 5B shows the effective killing against MDA-MB453 and activation ability of CNK-UT cells.
Fig. 6 illustrates that CNK-UT cells recognize and specifically kill THP1, wherein Fig. 6A shows the expression of different NK targets in acute myeloid leukemia cells THP1; Fig. 6B shows the effective killing against THP1 and activation ability of CNK-UT cells.
Fig. 7 illustrates that CNK-UT cells can upgrade conventional CAR-T techniques to achieve enhancement of targeted killing and activation ability. Fig. 7A shows the expression of GPC3, PD-L1 and different NK targets of HepG2 in hepatocellular carcinoma (HCC). Fig. 7B shows that
Fig. 8 illustrates that CNK-UT cells can upgrade conventional CAR-T techniques to achieve targeted killing against tumor cells with high PD-L1 expression and activation ability. Fig. 8A shows the expression of GPC3, PD-L1 and different NK targets of PLC in hepatocellular carcinoma (HCC). Fig. 8B shows that the more effective killing against PLC cells and activation ability of CAR/CNK-UT cells than conventional CAR-T cells.
Fig. 9 illustrates that GPC3 CAR/CNK-UT cells have higher tumor elimination ability in mice than GPC3 CAR-T cells.
Fig. 10 illustrates that GPC3 CAR/CNK-UT cells have higher tumor elimination ability than GPC3 CAR-T cells.
Fig. 11 illustrates that GPC3 CAR/CNK-UT can specifically recognize and kill the triple negative breast cancer cell MDA-MB453.
Fig. 12 illustrates that GPC3 CAR/CNK-UT can specifically recognize and kill acute myeloid leukemia cell line THP1.
Fig. 13 illustrates the recognition, specific killing against renal cell carcinoma 786-O cells and activation ability of CNK-UT cells.
Fig. 14 illustrates the recognition, specific killing against renal cell carcinoma ACHN cells and activation ability of CNK-UT cells.
Fig. 15 illustrates the recognition, specific killing against pulmonary epithelial adenoma A549 cells and activation ability of CNK-UT cells.
Fig. 16 illustrates the recognition, specific killing against AML cell line UL60 and activation ability of CNK-UT cells.
Fig. 17 illustrates the recognition, specific killing against AML cell line U937 (purchased from U937, item number CL-0239) and activation ability of CNK-UT cells.
Fig. 18 illustrates the recognition, specific killing against AML cell line U937 and activation ability of CNK-UT cells.
Fig. 19 illustrates the recognition, specific killing against pancreatic cancer cell line PANC-1 (purchased from Pnoxil, item number CL-0184) and activation ability of CNK-UT cells.

### Detailed description

In the present invention, unless otherwise stated, the scientific and technical terms used in this disclosure have a meaning generally understood by those skilled in the art. In addition, the protein and nucleic acid chemistry, molecular biology, cell and tissue culture, microbiology, immunology-related terms and laboratory procedures used in this disclosure are widely used terms and routine steps in the corresponding fields. Besides, in order to better understand the invention, definitions and explanations of related terms are provided below.

The term "chimeric natural killer receptor" (CNK) refers to a composition comprising a chimeric NK activating receptor module and a chimeric NK signal transduction module.

The term "CNK T cell" refers to a T cell containing a CNK construct.

The term "chimeric natural killer receptor-universal T cell" (CNK-UT) refers to the expression of NK activating receptor module constructs, CNK signal transduction module constructs, and UT module constructs of T cells.

The term "CAR/CNK-UT" is chemeric antigen receptor and chimeric natural killer receptor-universal T cell (CNK-UT) refers to a T cell expressing a CAR construct targeting a specific tumor antigen, a NK activating receptor module construct, a CNK signal transduction module construct, and a UT module construct.

As used herein, the term "about" refers to a measurable value such as an amount, a time duration, and the like, and encompasses variations of ±20%, ±10%, ±5%, ±1%, ±0.5% or ±0.1% from the specified value.

As used herein, the term "antibody" used in this disclosure refers to immunoglobulin molecules that specifically bind to antigens. Antibodies can be intact immunoglobulins derived from natural or recombinant sources and can be part of the immune response of intact immunoglobulins. Antibodies are usually tetramers of immunoglobulin molecules. The antibodies in the present disclosure may exist in a variety of forms, including, for example, polyclonal antibodies, monoclonal antibodies, Fv, Fab and F (ab), as well as scFv and humanized antibodies.

As used herein, the term "co-stimulatory ligand" includes a molecule on an antigen presenting cell (e.g., an APC, dendritic cell, B cell, and other immune cells) that specifically binds a cognate co-stimulatory molecule on a T cell, thereby providing a signal which, in addition to the primary signal provided by, for instance, binding of a TCR/CD3 complex with an MHC molecule loaded with peptide, mediates a T cell response, including, but not limited to, proliferation, activation, differentiation, etc. A co-stimulatory ligand can include, but not limited to, CD7, B7-1 (CD80), B7-2 (CD86), PD-L1, PD-L2, 4-1BBL, OX40L, inducible co-stimulatory ligand (ICOS-L), intercellular adhesion molecule (ICAM), CD30L, CD40, CD70, CD83, HLA-G, MICA, MICB, HVEM, lymphotoxin beta receptor, 3/TR6, ILT3, ILT4, HVEM, an agonist or antibody that binds Toll ligand receptor and a ligand that specifically binds with B7-H3. A co-stimulatory ligand also encompassesan antibody that specifically binds with a co-stimulatory molecule present on a T cell, such as, but not limited to, CD27, CD28, 4- 1BB, 0X40, CD30, CD40, PD-l, ICOS, lymphocyte function-associated antigen-l (LFA-l), CD2, CD7 LIGHT, NKG2C, B7-H3, and a ligand that specifically binds with CD83. The term "co-stimulatory molecule" or "co-stimulatory receptor" refers to the cognate binding partner on a T cell that specifically binds with a co-stimulatory ligand, thereby mediating a co-stimulatory response by the T cell, such as, but not limited to, proliferation. Co-stimulatory molecule includes, but not limited to, an MHC class I molecule, BTLA, a Toll ligand receptor. Co-stimulatory molecule also includes non-natural engineered protein(s).

As used herein, "co-stimulatory signal" refers to a signal, which in combination with a primary signal, such as TCR/CD3 ligation, leads to T cell proliferation and/or upregulation or down regulation of key molecules.

The term "stimulation" refers to a primary response induced by binding of a stimulatory molecule (e.g., a TCR/CD3 complex) with its cognate ligand thereby mediating a signal transduction event, such as, but not limited to, signal transduction via the TCR/CD3 complex. Stimulation can mediate altered expression of certain molecules, such as downregulation of TGF-B, and/or reorganization of cytoskeletal structures, and the like.

As used herein, the term "stimulatory molecule" refers to a molecule on a T cell that specifically binds with a cognate stimulatory ligand present on an antigen presenting cell.

As used herein, the term "stimulatory ligand" means a ligand that when present on an antigen presenting cell (e.g., an APC, a dendritic cell, a B-cell, and the like) can specifically bind with a cognate binding partner (referred to herein as a"stimulatory molecule') on a T cell, thereby mediating a primary response by the T cell, including, but not limited to, activation, initiation of an immune response, proliferation, and the like. Stimulatory ligands are well-known in the art and encompass, in particular, an MHC Class I molecule loaded with a peptide, an anti-CD3 antibody, a Superagonist anti-CD28 antibody, and a Superagonist anti-CD2 antibody.

The term "carrier" is a material composition that contains isolated nucleic acid and can be used to deliver isolated nucleic acid to the interior of the cell. Many vectors are known in the art, including, but not limited to, linear polynucleotides, polynucleotides associated with ions or amphoteric compounds, plasmids and viruses. Therefore, the term "vector" includes autonomous replication of plasmids or viruses. The term should also be interpreted to include non-plasmids and non-viral compounds that facilitate the transfer of nucleic acids into cells, such as polylysine compounds, liposomes, etc. Examples of viral vectors include, but not limited to, lentiviruses, adenoviral vectors, adeno-associated viral vectors, retroviral vectors and so on. Examples of non-viral vectors include, but not limited to, CRISPR vector systems, Sleeping Beauty transposon systems, etc. As used herein, the term "activation" refers to the state of a T cell that has been sufficiently stimulated to induce detectable cellular proliferation. Activation can also be associated with induced cytokine production, and detectable effector functions. The term "activated T cells" refers to in particular to T cells that are undergoing cell division.

In one aspect, the present disclosure provides a multi-functional complex including the following modules:
(1) a NK activating receptor module, comprising at least a NK-cell-activation receptor or a functional variant thereof, wherein the NK-cell-activation receptor comprises: (a) an extracellular domain (ED) of the NK-cell-activation receptor or a functional variant thereof, (b) a transmembrane domain (TMD) of the NK-cell-activation receptor or a functional variant thereof, and (c) an intracellular domain (ICD) of the NK-cell-activation receptor or a functional variant thereof; and optionally, a hinge or linker is included among the extracellular domain of the NK-cell-activation receptor or a functional variant thereof, the transmembrane domain of NK-cell-activation receptor or a functional variant thereof, and/or the intracellular domain of the NK-cell-activation receptor or a functional variant thereof;
(2) CNK signal transduction module, comprising at least (i) a NK cell signal adaptor or a functional variant thereof, wherein the NK cell signal adaptor comprises: (a) an extracellular domain (ED) of the NK cell signal adaptor or a functional variant thereof, (b) a transmembrane domain (TMD) of the NK cell signal adaptor or a functional variant thereof, and (c) an intracellular domain (ICD) of the NK cell signal adaptor or a functional variant thereof; and wherein, optionally, a hinge or linker is included among the extracellular domain of the NK cell signal adaptor or a functional variant thereof, the transmembrane domain of the NK cell signal adaptor or a functional variant thereof, and/or the intracellular domain of the NK cell signal adaptor or a functional variant thereof; and
(3) a UT module, comprising at least (i) a recombinant protein molecule for targeted degradation of TCR, MHC, and/or targets of a NK cell, or a functional variant thereof, wherein the recombinant protein molecule for targeted degradation of TCR, MHC, and/or targets of a NK cell, comprises: (a) a binding protein molecular domain targeting TCR, MHC, and/or targets of a NK cell, or a functional variant thereof, (b) a transmembrane domain of a viral endoplasmic reticulum (ER) resident glycoprotein or a functional variant thereof, and (c) a cytoplasmic domain of a viral endoplasmic reticulum resident glycoprotein or a functional variant thereof; the transmembrane domain of the viral endoplasmic reticulum resident glycoprotein or a functional variant thereof and the cytoplasmic domain of the viral endoplasmic reticulum resident glycoprotein or a functional variant thereof form an ERAD degradation domain; optionally, the molecular domain of the binding protein targeting TCR or a functional variant thereof, the transmembrane domain of the viral endoplasmic reticulum resident glycoprotein or a functional variant thereof and/or the cytoplasmic domain of the viral endoplasmic reticulum resident glycoprotein or a functional variant thereof comprise a hinge or linker. Optionally, the hinge or linker is included among the NK activating receptor module, the CNK signal transduction module, and/or the UT module.

In some embodiments, the NK-cell-activation receptor in the NK activating receptor module is selected from the group consisting of NKG2D, NKG2C, NKG2E, NKG2F, NKG2H, CD94, KIR2DL4, KIR2DS1, KIR2DS2, KIR2DS4, KIR3DS1, natural cytotoxic receptors, TRAIL, DNAM-1, CD 16a, 2B4, NTB-A, CRACC and NKp80; preferably, the natural cytotoxic receptors are selected from the group consisting of NKp46, NKp44 and NKp30.

In some preferred embodiments, the NK-cell-activation receptor is a mammalian derived NK-cell-activation receptor; preferably, the mammal is selected from the group consisting of human, primate, rat, horse, cattle, sheep, goat, cat, pig, dog, llama, alpacas, elephant, squirrel, guinea pig.

In some preferred embodiments, the NK-cell-activation receptor is a recombinant NK-cell-activation receptor comprising different original NK-cell-activation receptor domains.

In some preferred embodiments, the NK-cell-activation receptor is a human derived NK-cell-activation receptor; and preferably, the NK-cell-activation receptor is a recombinant NK-cell-activation receptor comprising different human derived NK-cell-activation receptor domains.

In some preferred embodiments, the NK-cell-activation receptor is a mouse derived NK-cell-activation receptor; and preferably, the NK-cell-activation receptor is a recombinant NK-cell-activation receptor comprising different murine derived NK-cell-activation receptor domains.

In some preferred embodiments, the NK-cell-activation receptor is a recombinant NK-cell-activation receptor comprising human derived NK-cell-activating receptor domain and mouse derived NK-cell-activation receptor domain.

In some preferred embodiments, the extracellular domain of the NK-cell-activation receptor is the extracellular domain of the human or murine NK-cell-activation receptor.

In some preferred embodiments, the transmembrane domain of the NK-cell-activation receptor is the transmembrane domain of the human or murine NK-cell-activation receptor.

In some preferred embodiments, the intracellular domain of the NK-cell-activation receptor is the intracellular domain of the human or murine NK-cell-activation receptor.

In some preferred embodiments, the functional variant of the NK-cell-activation receptor is selected from the group consisting of the NK-cell-activation receptor mutant, wild-type fusion protein, or wild-type and mutant fusion protein.

In some preferred embodiments, the extracellular domain of human NKG2D includes an amino acid sequence having 80% or more identity to the amino acid sequence shown in SEQ ID NO: 1, preferably an amino acid sequence having an identity of 85%, 90%, 95%, 96%, 97%, 98%, or 99% or more, and more preferably an amino acid sequence having an identity of 98%, or 99% or more; and the amino acid sequence of the extracellular domain of human NKG2D is shown as in SEQ ID NO: 1.

In some preferred embodiments, the full-length sequence of the human NKG2D includes an amino acid sequence having 80% or more identity to the amino acid sequence shown in SEQ ID NO: 2, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2D is shown as in SEQ ID NO: 2.

In some preferred embodiments, the extracellular domain of mouse NKG2D includes an amino acid sequence having 80% or more identity to the amino acid sequence shown in SEQ ID NO: 3, preferably an amino acid sequence having an identity of 85%, 90%, 95%, 96%, 97%, 98%, or 99% or more, and more preferably an amino acid sequence having an identity of 98%, or 99% or more; and the amino acid sequence of the extracellular domain of mouse NKG2D is shown as in SEQ ID NO: 3.

In some preferred embodiments, the full-length sequence of the mouse NKG2D includes an amino acid sequence having 80% or more identity to the amino acid sequence shown in SEQ ID NO: 4, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the mouse NKG2D is shown as in SEQ ID NO: 4.

In some preferred embodiments, the full-length sequence of the human-mouse recombinant NKG2D includes an amino acid sequence having 80% or more identity to the amino acid sequence shown in SEQ ID NO: 5, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human-mouse recombinant NKG2D is shown as in SEQ ID NO: 5.

In some preferred embodiments, the full-length sequence of the human NKG2C includes an amino acid sequence having 80% or more identity to the amino acid sequence shown in SEQ ID NO: 6, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2C is shown as in SEQ ID NO: 6.

In some preferred embodiments, the full-length sequence of the human NKG2E includes an amino acid sequence having 80% or more identity to the amino acid sequence shown in SEQ ID NO: 7, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2E is shown as in SEQ ID NO: 7.

In some preferred embodiments, the full-length sequence of the human NKG2F includes an amino acid sequence having 80% or more identity to the amino acid sequence shown in SEQ ID NO: 8, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKG2F is shown as in SEQ ID NO: 8.

In some preferred embodiments, the full-length sequence of the human CD94 includes an amino acid sequence having 80% or more identity to the amino acid sequence shown in SEQ ID NO: 9, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CD94 is shown as in SEQ ID NO: 9.

In some preferred embodiments, the full-length sequence of the human KIR2DL4 includes an amino acid sequence having 80% or more identity to the amino acid sequence shown in SEQ ID NO: 10, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DL4 is shown as in SEQ ID NO: 10.

In some preferred embodiments, the full-length sequence of the human KIR2DS1 includes an amino acid sequence having 80% or more identity to the amino acid sequence shown in SEQ ID NO: 11, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DS1 is shown as in SEQ ID NO: 11.

In some preferred embodiments, the full-length sequence of the human KIR2DS2 includes an amino acid sequence having 80% or more identity to the amino acid sequence shown in SEQ ID NO: 12, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DS2 is shown as in SEQ ID NO: 12.

In some preferred embodiments, the full-length sequence of the human KIR2DS4 includes an amino acid sequence having 80% or more identity to the amino acid sequence shown in SEQ ID NO: 13, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR2DS4 is shown as in SEQ ID NO: 13.

In some preferred embodiments, the full-length sequence of the human KIR3DS1 includes an amino acid sequence having 80% or more identity to the amino acid sequence shown in SEQ ID NO: 14, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human KIR3DS1 is shown as in SEQ ID NO: 14.

In some preferred embodiments, the full-length sequence of the human NKp46 includes an amino acid sequence having 80% or more identity to the amino acid sequence shown in SEQ ID NO: 15, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp46 is shown as in SEQ ID NO: 15.

In some preferred embodiments, the full-length sequence of the human NKp44 includes an amino acid sequence having 80% or more identity to the amino acid sequence shown in SEQ ID NO: 16, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp44 is shown as in SEQ ID NO: 16.

In some preferred embodiments, the full-length sequence of the human NKp30 includes an amino acid sequence having 80% or more identity to the amino acid sequence shown in SEQ ID NO: 17, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp30 is shown as in SEQ ID NO: 17.

In some preferred embodiments, the full-length sequence of the human DNAM1 includes an amino acid sequence having 80% or more identity to the amino acid sequence shown in SEQ ID NO: 18, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DNAM1 is shown as in SEQ ID NO: 18.

In some preferred embodiments, the full-length sequence of the human TRAIL includes an amino acid sequence having 80% or more identity to the amino acid sequence shown in SEQ ID NO: 19, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human TRAIL is shown as in SEQ ID NO: 19.

In some preferred embodiments, the full-length sequence of the human CD16a includes an amino acid sequence having 80% or more identity to the amino acid sequence shown in SEQ ID NO: 20, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CD16a is shown as in SEQ ID NO: 20.

In some preferred embodiments, the full-length sequence of the human 2B4 includes an amino acid sequence having 80% or more identity to the amino acid sequence shown in SEQ ID NO: 21, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human 2B4 is shown as in SEQ ID NO: 21.

In some preferred embodiments, the full-length sequence of the human NTB-A includes an amino acid sequence having 80% or more identity to the amino acid sequence shown in SEQ ID NO: 22, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NTB-A is shown as in SEQ ID NO: 22.

In some preferred embodiments, the full-length sequence of the human CRACC includes an amino acid sequence having 80% or more identity to the amino acid sequence shown in SEQ ID NO: 23, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CRACC is shown as in SEQ ID NO: 23.

In some preferred embodiments, the full-length sequence of the human NKp80 includes an amino acid sequence having 80% or more identity to the amino acid sequence shown in SEQ ID NO: 24, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp80 is shown as in SEQ ID NO: 24.

In some embodiments, the NK cell signal adaptor in the CNK signal transduction module is DAP10 or DAP12.

In some preferred embodiments, the NK cell signal adaptor is a mammalian derived NK cell signal adaptor; preferably, the mammal is selected from the group consisting of human, primate, rat, horse, cattle, sheep, goat, cat, pig, dog, llama, alpacas, elephant, squirrel, guinea pig.

In some preferred embodiments, the NK cell signal adaptor is a recombinant NK cell signal adaptor comprising different original NK cell signal adaptor domains.

In some preferred embodiments, the NK cell signal adaptor is a human derived NK cell signal adaptor; preferably, the NK cell signal adaptor is a recombinant NK cell signal adaptor comprising different human derivedNK cell signal adaptor domains.

In some preferred embodiments, the NK cell signal adaptor is a murine NK cell signal adaptor; preferably, the NK cell signal adaptor is a recombinant NK cell signal adaptor comprising different murine NK cell signal adaptor domains.

In some preferred embodiments, the NK cell signal adaptor is a recombinant NK cell signal adaptor comprising human derived, and murine derived NK cell signal adaptor domains.

In some preferred embodiments, the extracellular domain of the NK cell signal adaptor is the extracellular domain of the human or murine NK cell signal adaptor.

In some preferred embodiments, the transmembrane domain of the NK cell signal adaptor is the transmembrane domain of the human or murine NK cell signal adaptor.

In some preferred embodiments, the intracellular domain of the NK cell signal adaptor is the intracellular domain of the human or murine NK cell signal adaptor.

In some preferred embodiments, the functional variant of CNK cell signal adaptor is selected from the group consisting of a mutant of DAP10 or DAP12, or a fusion protein of DAP10 and DAP12, or a wild-type DAP 10 or DAP12 fusion protein with a mutant type DAP 10 or DAP12.

In some preferred embodiments, the CNK signal transduction module further includes (ii) immune receptor activation signal transduction domain (ITAM) and/or (iii) T cell co-stimulatory signal transduction domain.

In some preferred embodiments, a hinge or linker is included among the NK cell signal adaptor or a functional variant thereof, the immune receptor activation signal transduction domain (ITAM), and/or the T cell co-stimulatory signal transduction domain; preferably, the NK cell signal adaptor or a functional variant thereof is fused with the immune receptor activation signal transduction domain (ITAM).

In some preferred embodiments, the immune receptor activation signal transduction domain (ITAM) derives from the intracellular activation signal transduction domain of the immune receptor; preferably, the immune receptor is selected from the group consisting of TCRζ, CD2, CD3γ, CD3δ, CD3ε, CD3ζ, CD5, CD22, FcRy, CD66d, FcαRI, FcyRI, FcyRII, FcyRIII, Dectin-1, CLEC-1, CD72, CD79A, and CD79B; preferably, the immune receptor activated signal transdution domain (ITAM) is fused with the NK cell signal adaptor or its functional variant; and preferably, the immune receptor is CD3ζ.

In some preferred embodiments, the T cell co-stimulatory signal transduction domain is derived from the intracellular signal transduction domain of the co-stimulatory molecule; preferably, the co-stimulatory molecules are selected from the group consisting of MHCI molecules, TNF receptor proteins, immunoglobulin-like proteins, cytokine receptors, integrin proteins, lymphocyte activation signal molecules (SLAM proteins), activated NK cell receptors, BTLA, Toll ligand receptors, OX40, CD2, CD7, CD7, CD16, CD27, CD28, CD30, CD40, CD38, CD35, CD79A, CD79B, CDS, ICAM-1, LFA-1, (CD11a/CD18), 4-1BB(CD137), B7-H3, CDS, ICAM-1, ICOS(CD278), GITR, BAFFR, LIGHT, HVEM(LIGHTR), KIRDS2, SLAMF7, NKp80(KLRF1), NKp44, NKp30, NKp46, CD19, CD4, CD8α, CD8β, IL2Rβ, IL2Ry, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83-specific binding ligands, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, and PTCH2_{∘}

In some preferred embodiments, the full-length sequence of the human DAP10 includes an amino acid sequence having 80% or more identity to the amino acid sequence shown in SEQ ID NO: 25, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10 is shown as in SEQ ID NO: 25.

In some preferred embodiments, the full-length sequence of the human DAP10 includes an amino acid sequence having 80% or more identity to the amino acid sequence shown in SEQ ID NO: 26, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10 is shown as in SSEQ ID NO: 26.

In some preferred embodiments, the transmembrane domain of human DAP10 includes an amino acid sequence having 80% or more identity to the amino acid sequence shown in SEQ ID NO: 27, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domain of human DAP10 is shown as in SEQ ID NO: 27.

In some preferred embodiments, the full-length sequence of the human DAP12 includes an amino acid sequence having 80% or more identity to the amino acid sequence shown in SEQ ID NO: 28, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP12 is shown as in SEQ ID NO: 28.

In some preferred embodiments, the transmembrane domain of human DAP12 includes an amino acid sequence having 80% or more identity to the amino acid sequence shown in SEQ ID NO: 29, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domain of human DAP12 is shown as in SEQ ID NO: 29.

In some preferred embodiments, the fusion protein of the transmembrane domains of human DAP10 and human DAP12 includes an amino acid sequence having 80% or more identity to the amino acid sequence shown in SEQ ID NO: 30, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domains of human DAP10 and human DAP12 is shown as in SEQ ID NO: 30.

In some preferred embodiments, the sequence of fusion protein of human DAP10-DAP12 includes an amino acid sequence having 80% or more identity to the amino acid sequence shown in SEQ ID NO: 31, preferably an amino acid sequence having an identity of 85%, 90%, 95%, 96%, 97%, 98%, or 99% or more, and more preferably an amino acid sequence having an identity of 98%, or 99% or more; and amino acid sequence of the fusion protein of human DAP10-DAP12 is shown as in SEQ ID NO: 31.

In some preferred embodiments, the sequence of an intracellular signal transduction domain of human CD3zeta includes an amino acid sequence having 80% or more identity to the amino acid sequence shown in SEQ ID NO: 32, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human CD3zeta is shown as in SEQ ID NO: 32.

In some preferred embodiments, the human DAP10-CD3zeta sequence includes an amino acid sequence having 80% or more identity to the amino acid sequence shown in SEQ ID NO: 33, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP10-CD3zeta sequence is shown as in SEQ ID NO: 33.

In some preferred embodiments, the human DAP12-CD3zeta sequence includes an amino acid sequence having 80% or more identity to the amino acid sequence shown in SEQ ID NO: 34, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-CD3zeta sequence is shown as in SEQ ID NO: 34.

In some preferred embodiments, the human DAP10-DAP12-CD3zeta sequence includes an amino acid sequence having 80% or more identity to the amino acid sequence shown in SEQ ID NO: 35, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP10-DAP12-CD3zeta sequence is shown as in SEQ ID NO: 35.

In some preferred embodiments, the sequence of an intracellular signal transduction domain of human 41BB includes an amino acid sequence having 80% or more identity to the amino acid sequence shown in SEQ ID NO: 36, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human 41BB is shown as in SEQ ID NO: 36.

In some preferred embodiments, the the human DAP10-41BB sequence includes an amino acid sequence having 80% or more identity to the amino acid sequence shown in SEQ ID NO: 37, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10-41BB sequence is shown as in SEQ ID NO: 37.

In some preferred embodiments, the human DAP10-41BB-CD3zeta sequence includes an amino acid sequence having 80% or more identity to the amino acid sequence shown in SEQ ID NO: 38, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP10-41BB-CD3zeta sequence is shown as in SEQ ID NO: 38. In some preferred embodiments, the sequence of an intracellular signal transduction domain of human CD28 includes an amino acid sequence having 80% or more identity to the amino acid sequence shown in SEQ ID NO: 39, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human CD28 is shown as in SEQ ID NO: 39.

In some preferred embodiments, the human DAP10-CD28 sequence includes an amino acid sequence having 80% or more identity to the amino acid sequence shown in SEQ ID NO: 40, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP10-CD28 sequence is shown as in SEQ ID NO: 40.

In some preferred embodiments, the human DAP10-CD28-CD3zeta sequence includes an amino acid sequence having 80% or more identity to the amino acid sequence shown in SEQ ID NO: 41, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP10-CD28-CD3zeta sequence is shown as in SEQ ID NO: 41. In some preferred embodiments, the human DAP12-41BB sequence includes an amino acid sequence having 80% or more identity to the amino acid sequence shown in SEQ ID NO: 42, preferably an amino acid sequence having an identity of 85%, 90%, 95%, 96%, 97%, 98%, or 99% or more, and more preferably an amino cid sequence having an identity of 98%, or 99% or more; and the amino acid sequence of the human DAP12-41BB sequence is shown as in SEQ ID NO: 42.

In some preferred embodiments, the human DAP12-41BB-CD3zeta sequence includes an amino acid sequence having 80% or more identity to the amino acid sequence shown in SEQ ID NO: 43, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP12-41BB-CD3zeta sequence is shown as in SEQ ID NO: 43. In some preferred embodiments, the human DAP12-CD28 sequence includes an amino acid sequence having 80% or more identity to the amino acid sequence shown in SEQ ID NO: 44, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP12-CD28 sequence is shown as in SEQ ID NO: 44.

In some preferred embodiments, the human DAP12-CD28-CD3zeta sequence includes an amino acid sequence having 80% or more identity to the amino acid sequence shown in SEQ ID NO: 45, preferably an amino acid sequence having an identity of 85%, 90%, 95%, 96%, 97%, 98%, or 99% or more, and more preferably an amino acid sequence having an identity of 98%, or 99% or more; and the amino acid sequence of the DAP12-CD28-CD3zeta sequence is shown as in SEQ ID NO: 45. In some embodiments, in the recombinant protein molecule for targeted degradation of TCR of the UT module, the binding protein molecular domain targeting TCR or a functional variant thereof is derived from TCR antibody or a functional fragment thereof or the combination thereof.

In some preferred embodiments, the antibody is selected from the group consisting of a TCRα antibody, a TCRβ antibody, a TCRαβ antibody, a TCRγ antibody, a TCRδ antibody, a TCRγδ antibody, a TCR Vδ2 antibody, a TCR Cβ1 antibody; the functional fragment of the antibody is selected from the group consisting of Fd, Fv, Fab, Fab', F(ab')2, Fv(scFv), single chain antibody (scFv) or nanobody (nanobody), diabody, tribody and quadrubody; preferably, the TCR antibody is a single-chain TCR antibody; preferably, the amino acid sequence of the single-chain TCR antibody includes an amino acid sequence having 80% or more identity to the amino acid sequence shown in SEQ ID NO: 116, preferably an amino acid sequence having an identity of 85% or 90% , 95%, 96%, 97%, 98%, 99% or more, more preferably an amino acid sequence having an identity of 98% or 99% or more; the amino acid sequence of the full-length sequence of the single-chain TCR antibody is shown as in SEQ ID NO: 116.

In some preferred embodiments, the ERAD degradation domain in the UT module is derived from HCMV glycoprotein US2, US3, US11 or US10, adenovirus E3-19K or HHV-7US21.

In some preferred embodiments, the full-length sequence of the HCMV glycoprotein US2 includes an amino acid sequence having 80% or more identity to the amino acid sequence shown in SEQ ID NO: 46, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US2 is shown as in SEQ ID NO: 46.

In some preferred embodiments, the HLA binding domain of the HCMV glycoprotein US2 includes an amino acid sequence having 80% or more identity to the amino acid sequence shown in SEQ ID NO: 47, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US2 is shown as in SEQ ID NO: 47.

In some preferred embodiments, the ERAD degradation domain of the HCMV glycoprotein US2 includes an amino acid sequence having 80% or more identity to the amino acid sequence shown in SEQ ID NO: 48, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US2 is shown as in SEQ ID NO: 48.

In some preferred embodiments, the full-length sequence of the HCMV glycoprotein US3 includes an amino acid sequence having 80% or more identity to the amino acid sequence shown in SEQ ID NO: 49, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US3 is shown as in SEQ ID NO: 49.

In some preferred embodiments, the HLA binding domain of the HCMV glycoprotein US3 includes an amino acid sequence having 80% or more identity to the amino acid sequence shown in SEQ ID NO: 50, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US3 is shown as in SEQ ID NO: 50.

In some preferred embodiments, the ERAD degradation domain of the HCMV glycoprotein US3 includes an amino acid sequence having 80% or more identity to the amino acid sequence shown in SEQ ID NO: 51, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US3 is shown as in SEQ ID NO: 51.

In some preferred embodiments, the full-length sequence of the HCMV glycoprotein US11 includes an amino acid sequence having 80% or more identity to the amino acid sequence shown in SEQ ID NO: 52, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US11 is shown as in SEQ ID NO: 52.

In some preferred embodiments, the MHC binding domain of the HCMV glycoprotein US 11 includes an amino acid sequence having 80% or more identity to the amino acid sequence shown in SEQ ID NO: 53, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the HCMV glycoprotein US 11 is shown as in SEQ ID NO: 53.

In some preferred embodiments, the ERAD degradation domain of the HCMV glycoprotein US 11 includes an amino acid sequence having 80% or more identity to the amino acid sequence shown in SEQ ID NO: 54, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US11 is shown as in SEQ ID NO: 54.

In some preferred embodiments, the full-length sequence of the HCMV glycoprotein US10 includes an amino acid sequence having 80% or more identity to the amino acid sequence shown in SEQ ID NO: 55, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US10 is shown as in SEQ ID NO: 55.

In some preferred embodiments, the HLA binding domain of the HCMV glycoprotein US10 includes an amino acid sequence having 80% or more identity to the amino acid sequence shown in SEQ ID NO: 56, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US10 is shown as in SEQ ID NO: 56.

In some preferred embodiments, the ERAD degradation domain of the HCMV glycoprotein US10 includes an amino acid sequence having 80% or more identity to the amino acid sequence shown in SEQ ID NO: 57, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US10 is shown as in SEQ ID NO: 57.

In some preferred embodiments, the full-length sequence of the adenovirus E3-19K includes an amino acid sequence having 80% or more identity to the amino acid sequence shown in SEQ ID NO: 58, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the adenovirus E3-19K is shown as in SEQ ID NO: 58.

In some preferred embodiments, the MHC binding domain of the adenovirus E3-19K includes an amino acid sequence having 80% or more identity to the amino acid sequence shown in SEQ ID NO: 59, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the adenovirus E3-19K is shown as in SEQ ID NO: 59.

In some preferred embodiments, the ERAD degradation domain of the adenovirus E3-19K includes an amino acid sequence having 80% or more identity to the amino acid sequence shown in SEQ ID NO: 60, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the adenovirus E3-19K is shown as in SEQ ID NO: 60.

In some preferred embodiments, the full-length sequence of the HHV-7US21 includes an amino acid sequence having 80% or more identity to the amino acid sequence shown in SEQ ID NO: 61, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HHV-7US21 is shown as in SEQ ID NO:61.

In some preferred embodiments, the MHC binding domain of the HHV-7US21 includes an amino acid sequence having 80% or more identity to the amino acid sequence shown in SEQ ID NO: 62, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the HHV-7US21 is shown as in SEQ ID NO: 62.

In some preferred embodiments, the ERAD degradation domain of the HHV-7US21 includes an amino acid sequence having 80% or more identity to the amino acid sequence shown in SEQ ID NO: 63, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HHV-7US21 is shown as in SEQ ID NO: 63.

In some embodiments, the UT module further comprises (ii) a binding protein molecular domain targeting MHC I and/or MHC II or a functional variant thereof.

In some preferred embodiments, the binding protein molecular domain targeting MHC I and/or MHC II or a functional variant thereof, is a binding protein molecular domain, or a functional variant thereof, targeting HLA.

In some preferred embodiments, the binding protein molecular domain targeting MHC I and/or MHC II or a functional variant thereof further is derived from a viral endoplasmic reticulum protein that inhibits the expression of MHC molecule or promotes its degradation; preferably, the viral endoplasmic reticulum glycoprotein is selected from the group consisting of HCMV US6, HSV ICP47, CPXV012, HPV E6/E7, EBV BNFL2a or BHV UL49.5; preferably, the binding protein molecular domain targeting MHC I and/or MHC II, or a functional variant thereof, contains a TAP binding domain.

In some preferred embodiments, the full-length sequence of the HCMV US6 includes an amino acid sequence having 80% or more identity to the amino acid sequence shown inSEQ ID NO: 64, preferably an amino acid sequence having an identity of 85%, 90%, 95%, 96%, 97%, 98%, or 99% or more, and more preferably an amino acid sequence having an identity of 98%, or 99% or more; and amino acid sequence of full-length sequence of the HCMV US6 is shown as in SEQ ID NO: 64. In some preferred embodiments, the TAP binding domain of the HHV-7US6 includes an amino acid sequence having 80% or more identity to the amino acid sequence shown in SEQ ID NO: 65, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the HHV-7US6 is shown as in SEQ ID NO: 65.

In some preferred embodiments, the full-length sequence of the HSV ICP47 includes an amino acid sequence having 80% or more identity to the amino acid sequence shown in SEQ ID NO: 66, preferably an amino acid sequence having an identity of 85%, 90%, 95%, 96%, 97%, 98%, or 99% or more, and more preferably an amino acid sequence having an identity of 98%, or 99% or more; and amino acid sequence of full-length sequence of the HSV ICP47 is shown as in SEQ ID NO: 66. In some preferred embodiments, the TAP binding domain of the HSV ICP47 includes an amino acid sequence having 80% or more identity to the amino acid sequence shown in SEQ ID NO: 67, preferably an amino acid sequence having an identity of 85%, 90%, 95%, 96%, 97%, 98%, or 99% or more, and more preferably an amino acid sequence having an identity of 98%, or 99% or more; and amino acid sequence of the TAP binding domain of the HSV ICP47 is shown as in SEQ ID NO: 67.

In some preferred embodiments, the full-length sequence of the CPXV012 includes an amino acid sequence having 80% or more identity to the amino acid sequence shown in SEQ ID NO: 68, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the CPXV012 is shown as in SEQ ID NO:68.

In some preferred embodiments, the TAP binding domain of the CPXV012 includes an amino acid sequence having 80% or more identity to the amino acid sequence shown in SEQ ID NO: 69, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the CPXV012 is shown as in SEQ ID NO: 69.

In some preferred embodiments, the full-length sequence of the EBV BNFL2a includes an amino acid sequence having 80% or more identity to the amino acid sequence shown in SEQ ID NO: 70, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the EBV BNFL2a is shown as in SEQ ID NO: 70.

In some preferred embodiments, the TAP binding domain of the EBV BNFL2a includes an amino acid sequence having 80% or more identity to the amino acid sequence shown in SEQ ID NO: 71, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the EBV BNFL2a is shown as in SEQ ID NO: 71.

In some preferred embodiments, the full-length sequence of the BHV UL49.5 includes an amino acid sequence having 80% or more identity to the amino acid sequence shown in SEQ ID NO: 72, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the BHV UL49.5 is shown as in SEQ ID NO: 72.

In some preferred embodiments, the TAP binding domain of the BHV UL49.5 includes an amino acid sequence having 80% or more identity to the amino acid sequence shown in SEQ ID NO: 73, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the BHV UL49.5 is shown as in SEQ ID NO: 73.

In some preferred embodiments, the binding protein molecular domain targeting MHC I and/or MHC II or a functional variant thereof is derived from viral glycoproteins that degrade MHC and/or MHC II molecules; preferably, the viral glycoproteins are selected from the group consisting of HCMV glycoprotein US2, US3, US11 or US 10, adenovirus E3-19K, or HHV-7 US21.

In some preferred embodiments, the full-length sequence of the US2 includes an amino acid sequence having 80% or more identity to the amino acid sequence shown in SEQ ID NO: 74, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the US2 is shown as in SEQ ID NO:74.

In some preferred embodiments, the HLA binding domain of the US2 includes an amino acid sequence having 80% or more identity to the amino acid sequence shown in SEQ ID NO: 75, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US2 is shown as in SEQ ID NO: 75. In some preferred embodiments, the ERAD degradation domain of the US2 includes an amino acid sequence having 80% or more identity to the amino acid sequence shown in SEQ ID NO: 76, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the US2 is shown as in SEQ ID NO: 76.

In some preferred embodiments, the full-length sequence of the US3 includes an amino acid sequence having 80% or more identity to the amino acid sequence shown in SEQ ID NO: 77, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the US3 is shown as in SEQ ID NO:77.

In some preferred embodiments, the HLA binding domain of the US3 includes an amino acid sequence having 80% or more identity to the amino acid sequence shown in SEQ ID NO: 78, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US3 is shown as in SEQ ID NO: 78. In some preferred embodiments, the ERAD degradation domain of the US3 includes an amino acid sequence having 80% or more identity to the amino acid sequence shown in SEQ ID NO: 79, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the US3 is shown as in SEQ ID NO: 79.

In some preferred embodiments, the full-length sequence of the US11 includes an amino acid sequence having 80% or more identity to the amino acid sequence shown in SEQ ID NO: 80, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the US11 is shown as in SEQ ID NO:80. In some preferred embodiments, the HLA binding domain of the US11 includes an amino acid sequence having 80% or more identity to the amino acid sequence shown in SEQ ID NO: 81, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US11 is shown as in SEQ ID NO: 81. In some preferred embodiments, the ERAD degradation domain of the US11 includes an amino acid sequence having 80% or more identity to the amino acid sequence shown in SEQ ID NO: 82, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the US11 is shown as in SEQ ID NO: 82.

In some preferred embodiments, the binding protein molecular domain targeting MHC I and/or MHC II or a functional variant thereof further includes a viral protein that directively inhibits or degrades the NK target protein of MICA, MICB, ULBP1, ULBP2, ULBP3, ULBP4, ULBP5 or ULBP6; preferably, the viral protein is selected from HCMV UL16, UL141, UL142, or adenovirus E3-19K. In some preferred embodiments, the full-length sequence of the HCMV UL16 includes an amino acid sequence having 80% or more identity to the amino acid sequence shown in SEQ ID NO: 83, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL16 is shown as in SEQ ID NO: 83.

In some preferred embodiments, the NK target protein binding domain of the HCMV UL16 includes an amino acid sequence having 80% or more identity to the amino acid sequence shown in SEQ ID NO: 84, preferably an amino acid sequence having an identity of 85%, 90%, 95%, 96%, 97%, 98%, or 99% or more, and more preferably an amino acid sequence having an identity of 98%, or 99% or more; and amino acid sequence of the NK target protein binding domain of the HCMV LTL 16 is shown as in SEQ ID NO: 84.

In some preferred embodiments, the ERAD degradation domain of the HCMV UL16 includes an amino acid sequence having 80% or more identity to the amino acid sequence shown in SEQ ID NO: 85, preferably an amino acid sequence having an identity of 85%, 90%, 95%, 96%, 97%, 98%, or 99% or more, and more preferably an amino acid sequence having an identity of 98%, or 99% or more; and amino acid sequence of the ERAD degradation domain of the HCMV UL16 is shown as in SEQ ID NO: 85.

In some preferred embodiments, the full-length sequence of the HCMV UL141 includes an amino acid sequence having 80% or more identity to the amino acid sequence shown in SEQ ID NO: 86, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL141 is shown as in SEQ ID NO: 86.

In some preferred embodiments, the NK target protein binding domain of the HCMV UL141 includes an amino acid sequence having 80% or more identity to the amino acid sequence shown in SEQ ID NO: 87, preferably an amino acid sequence having an identity of 85%, 90%, 95%, 96%, 97%, 98%, or 99% or more, and more preferably an amino acid sequence having an identity of 98%, or 99% or more; and amino acid sequence of the NK target protein binding domain of the HCMV UL141 is shown as in SEQ ID NO: 87.

In some preferred embodiments, the ERAD degradation domain of the HCMV UL141 includes an amino acid sequence having 80% or more identity to the amino acid sequence shown in SEQ ID NO: 88, preferably an amino acid sequence having an identity of 85%, 90%, 95%, 96%, 97%, 98%, or 99% or more, and more preferably an amino acid sequence having an identity of 98%, or 99% or more; and amino acid sequence of the ERAD degradation domain of the HCMV UL141 is shown as in SEQ ID NO: 88.

In some preferred embodiments, the full-length sequence of the HCMV UL142 includes an amino acid sequence having 80% or more identity to the amino acid sequence shown in SEQ ID NO: 89, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL142 is shown as in SEQ ID NO: 89.

In some preferred embodiments, the MICA and ULBP3 binding domain of HCMV UL142 includes an amino acid sequence having 80% or more identity to the amino acid sequence shown in SEQ ID NO: 90, preferably an amino acid sequence having an identity of 85%, 90%, 95%, 96%, 97%, 98%, or 99% or more, and more preferably an amino acid sequence having an identity of 98%, or 99% or more; and the MICA and ULBP3 binding domains of HCMV UL142 is shown as in SEQ ID NO: 90. In some preferred embodiments, the Golgi residence domain of the HCMV LTL142 includes an amino acid sequence having 80% or more identity to the amino acid sequence shown in SEQ ID NO: 91, preferably an amino acid sequence having an identity of 85%, 90%, 95%, 96%, 97%, 98%, or 99% or more, and more preferably an amino acid sequence having an identity of 98%, or 99% or more; and amino acid sequence of the Golgi residence domain of the HCMV UL142 is shown as in SEQ ID NO: 91.

In some preferred embodiments, the binding protein molecular domain targeting MHC I and/or MHC II or a functional variant thereof further includes a viral protein that transports MHC I molecules from the Golgi apparatus to lysosomes for degradation; preferably, the viral protein is selected from the group consisting of HIV Nef, HIV Vpu, HHV-7 U21, HHV-8 KK3, HHV-8 KK5, MHV-68 MK3, and HTLV-1 p12.

In some preferred embodiments, the HIV Nef includes an amino acid sequence having 80% or more identity to the amino acid sequence shown in SEQ ID NO: 92, preferably an amino acid sequence having an identity of 85%, 90%, 95%, 96%, 97%, 98%, or 99% or more, and more preferably an amino acid sequence having an identity of 98%, or 99% or more; and the amino acid sequence of the HIV Nef is shown as in SEQ ID NO: 92.

In some preferred embodiments, the HIV Vpu includes an amino acid sequence having 80% or more identity to the amino acid sequence shown in SEQ ID NO: 114, preferably an amino acid sequence having an identity of 85%, 90%, 95%, 96%, 97%, 98%, or 99% or more, and more preferably an amino acid sequence having an identity of 98%, or 99% or more; and the amino acid sequence of the HIV Vpu is shown as in SEQ ID NO: 93.

In some preferred embodiments, the HHV-8 KK3 includes an amino acid sequence having 80% or more identity to the amino acid sequence shown in SEQ ID NO: 94, preferably an amino acid sequence having an identity of 85%, 90%, 95%, 96%, 97%, 98%, or 99% or more, and more preferably an amino acid sequence having an identity of 98%, or 99% or more; and the amino acid sequence of the HHV-8 KK3 is shown as in SEQ ID NO: 94.

In some preferred embodiments, the HHV-8 KK5 includes an amino acid sequence having 80% or more identity to the amino acid sequence shown in SEQ ID NO: 95, preferably an amino acid sequence having an identity of 85%, 90%, 95%, 96%, 97%, 98%, or 99% or more, and more preferably an amino acid sequence having an identity of 98%, or 99% or more; and the amino acid sequence of the HHV-8 KK5 is shown as in SEQ ID NO: 95.

In some preferred embodiments, the MHV-68 MK3 includes an amino acid sequence having 80% or more identity to the amino acid sequence shown in SEQ ID NO: 96, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHV-68 MK3 is shown as in SEQ ID NO: 96.

In some preferred embodiments, the HTLV-1 p12 includes an amino acid sequence having 80% or more identity to the amino acid sequence shown in SEQ ID NO: 97, preferably an amino acid sequence having an identity of 85%, 90%, 95%, 96%, 97%, 98%, or 99% or more, and more preferably an amino acid sequence having an identity of 98%, or 99% or more; and the amino acid sequence of the HTLV-1 p12 is shown as in SEQ ID NO: 97.

In some preferred embodiments, the binding protein molecular domain targeting MHC I and/or MHC II or a functional variant thereof further includes a viral protein that mediates the return of MHC-polypeptide molecules from the Golgi apparatus to the endoplasmic reticulum and promotes their degradation; preferably, the viral protein comprises an MHC binding structure and a KDEL receptor binding domain; preferably, the viral protein is Cowpox virus protein CPXV203.

In some preferred embodiments, the full-length sequence of the Cowpox virus protein CPXV203 includes an amino acid sequence having 80% or more identity to the amino acid sequence shown in SEQ ID NO: 98, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the Cowpox virus protein CPXV203 is shown as in SEQ ID NO: 98.

In some preferred embodiments, the MHC binding domain of the Cowpox virus protein CPXV203 includes an amino acid sequence having 80% or more identity to the amino acid sequence shown in SEQ ID NO: 99, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the Cowpox virus protein CPXV203 is shown as in SEQ ID NO: 99.

In some preferred embodiments, the KDEL receptor binding domain of the Cowpox virus protein CPXV203 includes an amino acid sequence having 80% or more identity to the amino acid sequence shown in SEQ ID NO: 100, preferably an amino acid sequence having an identity of 85%, 90%, 95%, 96%, 97%, 98%, or 99% or more, and more preferably an amino acid sequence having an identity of 98%, or 99% or more; and the amino acid sequence of the KDEL receptor binding domain of the Cowpox virus protein CPXV203 is shown as in SEQ ID NO: 100.

In some preferred embodiments, the full-length sequence of the Cowpox virus protein CPXV203 includes an amino acid sequence having 80% or more identity to the amino acid sequence shown in SEQ ID NO: 101, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the Cowpox virus protein CPXV203 is shown as in SEQ ID NO: 101.

In some preferred embodiments, the MHC binding domain of the Cowpox virus protein CPXV203 includes an amino acid sequence having 80% or more identity to the amino acid sequence shown in SEQ ID NO: 102, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the Cowpox virus protein CPXV203 is shown as in SEQ ID NO: 102.

In some preferred embodiments, the KDEL receptor binding domain of the Cowpox virus protein CPXV203 includes an amino acid sequence having 80% or more identity to the amino acid sequence shown in SEQ ID NO: 103, preferably an amino acid sequence having an identity of 85%, 90%, 95%, 96%, 97%, 98%, or 99% or more, and more preferably an amino acid sequence having an identity of 98%, or 99% or more; and the amino acid sequence of the KDEL receptor binding domain of the Cowpox virus protein CPXV203 is shown as in SEQ ID NO: 103.

In some embodiments, the multi-functional complex further comprises (4) a chimeric adaptor module and/or a receptor module with targeted killing activity against tumor cells;
The (4) chimeric adaptor module comprises: (i) an extracellular recognition domain targeting tumor, (ii) a transmembrane domain, and (iii) an intracellular signal transduction domain. Optionally, a hinge or linker is included between the extracellular recognition domain targeting tumor, the transmembrane domain and/or the intracellular signal transduction domain;
Preferably, the extracellular recognition domain targeting tumor of the chimeric adapter module is selected from the group consisting of a tumor antigen-specific binding domain, a tumor microenvironment target antigen binding domain, and/or a chemokine receptor targeting tumor microenvironment.

In some preferred embodiments, the extracellular recognition domain targeting tumor is selected from the group consisting of an antibody capable of targeting a tumor-associated antigen or a functional fragment thereof, TCR or the combination thereof. The functional fragment of the antibody is selected from the group consisting of Fd, Fv, Fab, Fab', F(ab') 2, Fv (scFv), single-chain antibody (scFv) or nanobody, double-chain antibody, three-chain antibody and four-chain antibody.

In some preferred embodiments, the transmembrane domain of the chimeric adapter module is selected from the group consisting of the NK-cell-activation receptor transmembrane domain, DAP 10 transmembrane domain, DAP12 transmembrane domain, CD8 transmembrane domain, CD28 transmembrane domain, CD4 transmembrane domain, 4-1BB transmembrane domain, OX40 transmembrane domain, ICOS transmembrane domain, CTLA-4 transmembrane domain, PD-1 transmembrane domain, LAG-3 transmembrane domain, 2B4 transmembrane domains, and BTLA transmembrane domain, as well as the combination thereof; preferably, the NK-cell-activation receptor is selected from the group consisting of NKG2D, NKG2C, NKG2E, NKG2F, NKG2H, CD94, KIR2DL4, KIR2DS1, KIR2DS2, KIR2DS4, KIR3DS1, natural cytotoxic receptor, TRAIL, DNAM-1, CD16a, 2B4, NTB-A, CRACC, and NKp80; preferably, the natural cytotoxic receptor is selected from the group consisting of Nkp46, Nkp44, and Nkp30.

In some preferred embodiments, the intracellular signal domain of the chimeric adapter module includes an intracellular signal transduction domain of the NK-cell-activation receptor and/or a co-stimulatory signal transduction domain.

In some preferred embodiments, the NK-cell-activation receptor is selected from the group consisting ofNKG2D, NKG2C, NKG2E, NKG2F, NKG2H, CD94, KIR2DL4, KIR2DS1, KIR2DS2, KIR2DS4, KIR3DS1, a natural cytotoxic receptor, TRAIL, DNAM-1, CD16a, 2B4, NTB-A, CRACC, and NKp80.

In some preferred embodiments, the intracellular signal domain further comprises a co-stimulatory signal transduction domain; preferably, the co-stimulatory signal transduction domain is selected from the group consisting of T cell co-stimulatory signal transduction domain, comprising, but not limited to, the intracellular signal domain derived from MHCI molecules, TNF receptor proteins, immunoglobulin-like proteins, cytokine receptors, integrin proteins, lymphocyte activation signal molecules (SLAM proteins), activated NK cell receptors, BTLA, Toll ligand receptors, OX40, CD2, CD7, CD16, CD27, CD28, CD30, CD40, CD38, CD35, CD79A, CD79B, CDS, ICAM-1, LFA-1, (CD11a/CD18), 4-1BB(CD137), B7-H3, CDS, ICAM-1, ICOS(CD278), GITR, BAFFR, LIGHT, HVEM(LIGHTR), KIRDS2, SLAMF7, NKp80(KLRF1), NKp44, NKp30, NKp46, CD19, CD4, CD8α, CD8β, IL2Rβ, IL2Ry, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83-specific binding ligands, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, and PTCH2; more preferably, the co-stimulatory signal transduction domain is selected from the group consisting of the NKG2D intracellular signal transduction domain, DAP10 intracellular signal transduction domain, DAP12 intracellular signal transduction domain, NCR intracellular signal transduction domain, CD28 intracellular signal transduction domain, 4-1BB intracellular signal transduction domain, OX40 intracellular signal transduction domain, and ICOS intracellular signal transduction domain;
the receptor module with targeted killing activity against tumor cells includes: (i) an extracellular recognition domain targeting tumor antigen; (ii) a transmembrane domain; and (iii) an intracellular co-stimulatory signal transduction domain; (iv) a T cell activation signal transduction domain (ITAM); optionally, a hinge or linker is included between the extracellular recognition domain targeting tumor antigen, the transmembrane domain, the intracellular co-stimulatory signal transduction domain, and/or the T cell activation signal transduction domain (ITAM);
the transmembrane domain of the receptor module with targeted killing activity against tumor cells is selected from the group consisting of CD8 transmembrane domain, α and/or β chain transmembrane domain of T cell receptor, CD28 transmembrane domain, CD3ε transmembrane domain, CD45 transmembrane domain, CD4 transmembrane domain, CD5 transmembrane domain, CD8 transmembrane domain, CD9 transmembrane domain, CD16 transmembrane domain, CD22 transmembrane domain, CD33 transmembrane domain, CD37 transmembrane domain, CD64 transmembrane domain, CD80 transmembrane domain, CD86 transmembrane domain, CD134 transmembrane domain, CD137 transmembrane domain, CD154 transmembrane domain, GITR transmembrane domain, and combinations thereof;
the T cell activation signal transduction domain is derived from CD3ζ, common FcRy (FCER1G), FcγRIIa, FcRβ, CD3γ, CD3δ, CD3ε, CD5, CD22, CD79a, CD79b, CD278 ("ICOS"), FcεRI CD66d, DAP10 and DAP12 and other intracellular signal transduction domains.

In some preferred embodiments, the linker is a flexible linker; preferably, the flexible linker includes the amino acid sequence indicated (Gly(x)Ser(y)n, wherein n is an integer from 1 to 10, and x and y are independently integers from 0 to 10, provided that x and y are not both 0; and more preferably, the linker includes an amino acid sequence indicated in SEQ ID NO: 105 or an amino acid sequence indicated in SEQ ID NO: 106.

In some preferred embodiments, the linker is a hinge; preferably, the hinge is an IgG1 hinge or an IgG4 hinge.

In some preferred embodiments, the IgG1 hinge includes an amino acid sequence having 80% or more identity to the amino acid sequence shown in SEQ ID NO: 106, preferably an amino acid sequence having an identity of 85%, 90%, 95%, 96%, 97%, 98%, or 99% or more, and more preferably an amino acid sequence having an identity of 98%, or 99% or more; and the amino acid sequence of the IgG1 hinge is shown as in SEQ ID NO: 106.

In some preferred embodiments, the IgG4 hinge includes an amino acid sequence having 80% or more identity to the amino acid sequence shown in SEQ ID NO: 107, preferably an amino acid sequence having an identity of 85%, 90%, 95%, 96%, 97%, 98%, or 99% or more, and more preferably an amino acid sequence having an identity of 98%, or 99% or more; and the amino acid sequence of the IgG4 hinge is shown as in SEQ ID NO: 107.

In some preferred embodiments, a cleavage peptide is included between the NK activating receptor module, CNK signal transduction module, and/or UT module, for example, T2A peptide, GSG-T2A peptide, E2A peptide, GSG-E2A peptide, F2A peptide, GSG-F2A peptide, P2A peptide, or GSG-P2A peptide.

In some preferred embodiments, the T2A includes an amino acid sequence having 80% or more identity to the amino acid sequence shown in SEQ ID NO: 108, preferably an amino acid sequence having an identity of 85%, 90%, 95%, 96%, 97%, 98%, or 99% or more, and more preferably an amino acid sequence having an identity of 98%, or 99% or more; and the amino acid sequence of the T2A is shown as in SEQ ID NO: 108.

In some preferred embodiments, the amino acid sequence of the GSG-T2A peptide is shown in SEQ ID NO: 109.

In some preferred embodiments, the P2A includes an amino acid sequence having 80% or more identity to the amino acid sequence shown in SEQ ID NO: 110, preferably an amino acid sequence having an identity of 85%, 90%, 95%, 96%, 97%, 98%, or 99% or more, and more preferably an amino acid sequence having an identity of 98%, or 99% or more; and the amino acid sequence of the P2Ais shown as in SEQ ID NO: 110.

In some preferred embodiments, the amino acid sequence of the GSG-P2A peptide is shown in SEQ ID NO: 111.

In some preferred embodiments, the E2A includes an amino acid sequence having 80% or more identity to the amino acid sequence shown in SEQ ID NO: 112, preferably an amino acid sequence having an identity of 85%, 90%, 95%, 96%, 97%, 98%, or 99% or more, and more preferably an amino acid sequence having an identity of 98%, or 99% or more; and the amino acid sequence of the E2A is shown as in SEQ ID NO: 112.

In some preferred embodiments, the amino acid sequence of the GSG-E2A peptide is shown in SEQ ID NO: 113.

In some preferred embodiments, the F2A includes an amino acid sequence having 80% or more identity to the amino acid sequence shown in SEQ ID NO: 114, preferably an amino acid sequence having an identity of 85%, 90%, 95%, 96%, 97%, 98%, or 99% or more, and more preferably an amino acid sequence having an identity of 98%, or 99% or more; and the amino acid sequence of the F2A is shown as in SEQ ID NO: 114.

In some preferred embodiments, the amino acid sequence of the GSG-F2A peptide is shown in SEQ ID NO: 115.

In some preferred embodiments, the multi-functional complex includes an amino acid sequence having 80% or more identity to the amino acid sequence shown in SEQ ID NO: 116, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex includes a single chain antibody of a TCR antibody as shown in SEQ ID NO: 116.

In some preferred embodiments, the multi-functional complex includes an amino acid sequence having 80% or more identity to the amino acid sequence shown in SEQ ID NO: 117, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 117.

In some preferred embodiments, the multi-functional complex includes an amino acid sequence having 80% or more identity to the amino acid sequence shown in SEQ ID NO: 121, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 121.

In some preferred embodiments, the multi-functional complex includes an amino acid sequence having 80% or more identity to the amino acid sequence shown in SEQ ID NO: 123, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 123.

In some preferred embodiments, the multi-functional complex includes an amino acid sequence having 80% or more identity to the amino acid sequence shown in SEQ ID NO: 125, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 125.

In some preferred embodiments, the multi-functional complex includes an amino acid sequence having 80% or more identity to the amino acid sequence shown in SEQ ID NO: 126, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 126.

In some preferred embodiments, the multi-functional complex includes an amino acid sequence having 80% or more identity to the amino acid sequence shown in SEQ ID NO: 127, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 127.

In some preferred embodiments, the multi-functional complex includes an amino acid sequence having 80% or more identity to the amino acid sequence shown in SEQ ID NO: 128, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 128.

In some preferred embodiments, the multi-functional complex includes an amino acid sequence having 80% or more identity to the amino acid sequence shown in SEQ ID NO: 129, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 129.

In some preferred embodiments, the multi-functional complex includes an amino acid sequence having 80% or more identity to the amino acid sequence shown in SEQ ID NO: 130, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 130.

In some preferred embodiments, the multi-functional complex includes an amino acid sequence having 80% or more identity to the amino acid sequence shown in SEQ ID NO: 131, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 131.

In one respect, the present disclosure provides a nucleic acid molecule encoding any of the multi-functional complexes above mentioned.

In some preferred embodiments, the nucleic acid molecule is DNA or RNA.

In some preferred embodiments, the RNA is mRNA.

In some preferred embodiments, the nucleic acid molecule includes a nucleotide sequence having 80% or more identity to the nucleotide sequence shown in SEQ ID NO: 118, preferably a nucleotide sequence having an identity of 85%, 90%, 95%, 96%, 97%, 98%, or 99% or more, and more preferably a nucleotide sequence having an identity of 98%, or 99% or more; and the nucleotide sequence of the nucleic acid molecule is shown as in SEQ ID NO: 118.

In some preferred embodiments, the nucleic acid molecule includes a nucleotide sequence having 80% or more identity to the nucleotide sequence shown in SEQ ID NO: 122, preferably a nucleotide sequence having an identity of 85%, 90%, 95%, 96%, 97%, 98%, or 99% or more, and more preferably a nucleotide sequence having an identity of 98%, or 99% or more; and the nucleotide sequence of the nucleic acid molecule is shown as in SEQ ID NO: 122.

In some preferred embodiments, the nucleic acid molecule includes a nucleotide sequence having 80% or more identity to the nucleotide sequence shown in SEQ ID NO: 124, preferably a nucleotide sequence having an identity of 85%, 90%, 95%, 96%, 97%, 98%, or 99% or more, and more preferably a nucleotide sequence having an identity of 98%, or 99% or more; and the nucleotide sequence of the nucleic acid molecule is shown as in SEQ ID NO: 124.

In one respect, the present disclosure provides an expressing vector containing the nucleic acid molecule above mentioned.

In some preferred embodiments, the vector is selected from the group consisting of: plasmid, cosmid, viral vector, RNA vector, or linear or circular DNA or RNA molecules;
In some preferred embodiments, the viral vector is selected from the group consisting of: retrovirus, adenovirus, parvoviruse (e.g., adeno-associated virus), coronavirus, negative-strand RNA virus such as orthomyxovirus (e.g., influenza virus), rhabdovirus (e.g., rabies and vesicular stomatitis viruses), paramyxovirus (e.g., Machi and Sendai), positive-strand RNA virus such as small RNA viruses, alphavirus, and double-stranded DNA virus, wherein the double-stranded DNA virus compirses adenovirus, herpesvirus (e.g., herpes simplex virus types 1 and 2, Epstein-Barr virus, cytomegalovirus) and pox Virus (e.g., vaccinia virus, fowlpox virus, and canarypox virus), Norwalk virus, togavirus, flavivirus, reovirus, papovavirus, hepadnavirus, baculovirus, and hepatitis virus.

In some preferred embodiments, the retroviral vector is selected from the group consisting of: avian leukoproliferative-sarcoma, mammalian C-type virus, B-type virus, D-type virus, HTLV-BLV collection, Lentivirus, bubble virus.

In some preferred embodiments, the lentiviral vector is selected from the group consisting of HIV-1, HIV-2, SIV, FIV, BIV, EIAV, CAEV or ovine demyelinating leukoencephalitis lentivirus.

In some preferred embodiments, the NK activating receptor module, CNK signal transduction module and/or UT module may regulate expression under the same promoter of the same vector, or under different promoters, or in a plurality of vectors.

In some preferred embodiments, the vector is lentiviral vector, a cleavage peptide-coding gene is included between the gene encoding NK activating receptor module, CNK signal transduction module and/or UT module ; preferably, the cleavable peptide is a 2A linker; the 2A linker is selected from T2A, P2A, E2A and F2A.

In some preferred embodiments, the vector further includes a promoter; and preferably, the promoter is an EF1α promoter or CMV promoter.

In one respect, the present disclosure provides an immune cell, comprising the nucleic acid or the expression vector above mentioned.

In some preferred embodiments, the immune cell is selected from the group consisting of T cell, NKT cell, NK cell, B cell, monocyte, macrophage, etc.

In one respect, the present disclosure provides a method for producing immune cells, comprising introducing the nucleic acid or the expression vector described herein into cells by methods selected from the following: electroporation, acoustic perforation, gene gun (e.g., gene gun with Au- particles), lipid transfection, polymer transfection, nanoparticles or polymer complexes.

In one aspect, the present disclosure provides a pharmaceutical composition comprising the multi-functional complex, the nucleic acid, the expression vector, the immune cell and/or the immune cell produced by the method described herein, and pharmaceutically acceptable carriers.

In one aspect, the present disclosure provides use of the multi-functional complex, the nucleic acid, the expression vector, the immune cell, immune cell produced by the method, and/or pharmaceutically acceptable carriers described herein, in the manufacture of a medicine for treating diseases.

In one aspect, the present disclosure provides a method of treating diseases, comprising administering the multi-functional complex, the nucleic acid, the expression vector, the immune cell and/or the pharmaceutical composition to a subject.

In some preferred embodiments, the diseases include various solid tumors and hematological tumors, viral infectious diseases, autoimmune diseases.

In some preferred embodiments, the solid tumors are selected from the group consisting of nervous system tumors, head and neck tumors, chest tumors, digestive system tumors, genitourinary system tumors, soft tissue and skin tumors, bone tumors, etc.

In some preferred embodiments, the nervous system tumors comprise diffuse glioma, diffuse astrocytoma and anaplastic astrocytoma, glioblastoma, oligodendroglioma, oligoastrocytoma, diffuse Gliomas, other astrocytomas, ependymomas, neuronal and mixed neuronal-glial tumors, medulloblastoma, other embryonal tumors, schwannomas, meningiomas, solitary fibrous tumors and hemangiopericytoma, etc.

In some preferred embodiments, the head and neck tumors comprise malignant tumors of the nasal cavity and sinuses, nasopharyngeal cancer, oral cancer, laryngeal cancer, salivary gland tumors, intracranial tumors, thyroid cancer, tongue cancer, etc.

In some preferred embodiments, the thoracic tumors comprise lung cancer, esophageal cancer, cardia cancer, breast cancer, mediastinal tumors, etc.

In some preferred embodiments, the digestive system tumors comprise gastric cancer, colorectal cancer, sigmoid colon and rectal cancer, liver cancer, pancreatic cancer and periampullary cancer, biliary tract cancer, malignant tumors of the small intestine, etc.

In some preferred embodiments, the genitourinary system tumors comprise kidney cancer, prostate cancer, bladder cancer, testicular cancer, penile cancer, cervical cancer, endometrial cancer, ovarian cancer, etc.

In some preferred embodiments, the soft tissue and skin tumors comprise malignant fibrous histiocytoma, rhabdomyosarcoma, synovial sarcoma, malignant melanoma of the skin, etc.

In some preferred embodiments, the bone tumors comprise osteosarcoma, Ewing's sarcoma, etc.

In some preferred embodiments, the colon cancer is a colon adenoma.

In some preferred embodiments, the breast cancer is a triple-negative breast cancer cell.

In some preferred embodiments, the liver cancer is hepatocellular carcinoma.

In some preferred embodiments, the disease is a hematological tumor selected from the group consisting of leukemia, lymphoma (HL), multiple myeloma (MM), myelodysplastic syndrome (MDS), etc.

In some preferred embodiments, the leukemia is B-cell acute lymphoblastic leukemia, T-cell acute lymphoblastic leukemia, acute myeloid leukemia, etc.

In some preferred embodiments, the viral infectious diseases comprise: respiratory viral diseases, gastrointestinal viral diseases, liver viral diseases, skin and mucous membrane viral diseases, ocular viral diseases, central nervous system viral diseases, lymphocytic viral diseases, insect-borne viral diseases, lentivirus infection diseases, etc.

In some preferred embodiments, the respiratory viral diseases comprise infections of rhinovirus, adenovirus, respiratory syncytial virus, parainfluenza virus, and coronavirus; influenza; mumps, etc. In some preferred embodiments, the gastrointestinal viral diseases comprise polio; cooksackie virus infection; ECHO virus infection; and viral gastroenteritis including rotavirus gastroenteritis, Norwalk virus gastroenteritis, adenovirus Viral gastroenteritis, astrovirus gastroenteritis, coronavirus gastroenteritis and calicivirus gastroenteritis, etc.

In some preferred embodiments, the liver viral diseases comprise viral hepatitis A, viral hepatitis B, viral hepatitis C, viral hepatitis D, viral hepatitis E, Epstein-Barr viral hepatitis, and cytomegalovirus hepatitis, etc.

In some preferred embodiments, the skin and mucous membrane viral diseases comprise measles, rubella, infantile acute rash, chickenpox and herpes zoster, smallpox, herpes simplex virus infection, rabies and foot-and-mouth disease, etc.

In some preferred embodiments, the ocular viral diseases comprise epidemic keratoconjunctivitis, follicular conjunctivitis and herpetic keratoconjunctivitis, etc.

In some preferred embodiments, the central nervous system viral diseases comprise Japanese encephalitis, western equine encephalitis, eastern equine encephalitis, St. Louis encephalitis, Venezuelan equine encephalitis, Murray Valley encephalitis, Californian encephalitis, forest encephalitis and lymphocytic choroid plexus meningitis, etc.

In some preferred embodiments, the lymphocytic viral diseases comprise infectious mononucleosis, cytomegalovirus infection and acquired immunodeficiency syndrome, etc.

In some preferred embodiments, the insect-borne viral diseases comprise: viral hemorrhagic fevers, including epidemic hemorrhagic fever, yellow fever, Crimean-Congo hemorrhagic fever, Rift Valley fever, Argentine hemorrhagic fever, Bolivian hemorrhagic fever, Lassa fever, Omu Scrubs hemorrhagic fever, Marburg disease and Ebola hemorrhagic fever, etc.; dengue fever and dengue hemorrhagic fever; West Nile fever; Colorado tick heat transfer; and sandfly fever, etc.

In some preferred embodiments, the lentiviral infection diseases comprise subacute sclerosing panencephalitis, Kuru disease, progressive multifocal leukoencephalopathy and subacute spongiform encephalopathy (corticostriatal spinal cord degeneration), etc.

In some preferred embodiments, the autoimmune diseases comprise organ-specific autoimmune diseases and systemic autoimmune diseases.

Preferably, the organ-specific autoimmune diseases comprise chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritic syndrome, vulgaris Pemphigus, pemphigoid, primary biliary cirrhosis, multiple sclerosis, acute idiopathic polyneuritis, etc.

In some preferred embodiments, the systemic autoimmune diseases comprise systemic lupus erythematosus, rheumatoid arthritis, systemic vasculitis, scleroderma, pemphigus, dermatomyositis, mixed connective tissue disease, autoimmune hemolytic anemia, thyroid autoimmune diseases, ulcerative colitis, etc.

In one aspect, the peresent disclosure provides a method to stimulate an immune response in subjects, comprising administering an effective amount of the multi-functional complex, the nucleic acid, the expression vector, the immune cell, the immune cell produced by the method, and/or the pharmaceutical composition described herein to a subject.

The present disclosure also provides cells, cell populations, and compositions (including pharmaceutical and therapeutic compositions) comprising the cells and populations, e.g., cells and cell populations produced by the provided methods, and methods, e.g., a therapeutic method for administering the cells and compositions to a subject, such as a patient.

Compositions including cells for administering are also provided, including pharmaceutical compositions and formulations, such as unit dosage form compositions including a number of cells for administering in a given dose or fraction thereof. Pharmaceutical compositions and formulations typically include one or more optional pharmaceutically acceptable carriers or excipients. In some embodiments, the composition comprises at least one additional therapeutic agent.

The term "pharmaceutical formulations" refers to a preparation in a form that allows the biological activity of the active ingredient contained therein to be effective, and does not contain additional components that would have unacceptable toxicity to the subject to whom the formulation is to be administered.

"Pharmaceutically acceptable carriers" refers to an ingredient of a pharmaceutical formulation, other than the active ingredient, that is not toxic to the subject. Pharmaceutically acceptable carriers include, but not limited to, buffers, excipients, stabilizers or preservatives.

In some aspects, the choice of carriers is determined in part by the specific cells and/or method of administration. Therefore, a variety of suitable formulations exist. For example, pharmaceutical compositions may contain preservatives. Suitable preservatives may include, for example, methylparaben, propylparaben, sodium benzoate and benzalkonium chloride. In some aspects, a mixture of two or more preservatives is used. The preservative or mixture thereof is typically present in an amount from about 0.0001% to about 2% by weight of the total composition. Pharmaceutically acceptable carriers are generally nontoxic to the receptor at doses and concentrations used and include, but not limited to: buffers such as phosphates, citrates, and other organic acids; antioxidants, including ascorbic acid and methionine; preservatives (such as octadecyldimethylbenzyl ammonium chloride; hexamethonium chloride; benzalkonium chloride; benzethonium chloride; phenol, butanol or benzyl alcohol; alkyl p-hydroxybenzoates Esters, such as methyl or propyl paraben; catechol; resorcin; cyclohexanol; 3-pentanol and m-cresol); low molecular weight (less than about 10 residues ) polypeptides; proteins, such as serum albumin, gelatin or immunoglobulins; hydrophilic polymers, such as polyvinylpyrrolidone; amino acids, such as glycine, glutamine, asparagine, histidine, arginine or lysine; monosaccharides, disaccharides, and other carbohydrates, including glucose, mannose, or dextrin; chelating agents, such as EDTA; sugars, such as sucrose, mannitol, trehalose, or sorbitol; salts that form counterions, such as sodium; metal complexes (e.g. zinc protein complexes); and/or non-ionic surfactants such as polyethylene glycol (PEG).

In some aspects, a buffer is included in the composition. Suitable buffers include, for example, citric acid, sodium citrate, phosphoric acid, potassium phosphate and various other acids and salts. In some aspects, a mixture of two or more buffers is used. The buffer or mixture thereof is typically present in an amount from about 0.001% to about 4% by weight of the total composition. Methods of preparing pharmaceutical compositions for administration are known.

Formulations may include aqueous solutions. The formulation or composition may also contain more than one active ingredient useful for the particular indication, disease or condition being treated with the cell, preferably those with activities that are complementary to the cell, where the respective activities do not adversely affect one another. Such active ingredients are suitably present in combination in amounts effective for the intended purpose. Thus, in some embodiments, the pharmaceutical compositions further comprise other pharmaceutically active agents or drugs, such as chemotherapeutic agents (e.g., asparaginase, busulfan, carboplatin, cisplatin, daunorubicin, doxorubicin, fluorouracil, gemcitabine, hydroxyurea, methotrexate, paclitaxel, rituximab, vinblastine and/or vincristine).

In some embodiments, a pharmaceutical composition contains an amount of cells effective to treat or prevent a disease or condition, such as a therapeutically effective amount or a prophylactically effective amount. In some embodiments, therapeutic or prophylactic efficacy is monitored by periodic assessment of treated subjects. The desired dose can be delivered by administering the cells as a single bolus, as multiple bolus, or as a continuous infusion.

Cells and composition(s) can be administered using standard administration techniques, formulations and/or devices. Administration of cells can be autologous or allogeneic. For example, immune response cells or progenitor cells can be obtained from one subject and administered to the same subject or to a different, compatible subject. Peripheral blood-derived immune response cells or progeny thereof (e.g., derived in vivo, ex vivo, or in vitro) can be administered by local injection, including catheter administration, systemic injection, local injection, intravenous injection, or parenteral administration. When therapeutic compositions (e.g., pharmaceutical compositions containing genetically modified immune response cells) are administered, they are typically formulated in unit dose injectable forms (solutions, suspensions, emulsions).

Formulations include those for oral, intravenous, intraperitoneal, subcutaneous, pulmonary, transdermal, intramuscular, intranasal, buccal, sublingual, and suppository administration. In some embodiments, the cell population is parenteral administration. The term "parenteral" as used herein includes intravenous, intramuscular, subcutaneous, rectal, vaginal and intraperitoneal administration. In some embodiments, cells are administered to a subject using peripheral systemic delivery by intravenous, intraperitoneal, or subcutaneous injection.

In some embodiments, the compositions are provided as sterile liquid preparations, such as isotonic aqueous solutions, suspensions, emulsions, dispersions, or viscous compositions, which in certain aspects can be buffered to a selected pH. Liquid preparations are generally easier to prepare than gels, other viscous compositions and solid compositions. Additionally, liquid compositions are somewhat more convenient to administer, especially by injection. Viscous compositions, on the other hand, can be formulated within an appropriate viscosity range to provide longer contact time with specific tissues. Liquid or viscous compositions may include a carrier, which may be a solvent or dispersion medium, including, for example, water, saline, phosphate buffered saline, polyols (e.g., glycerol, propylene glycol, liquid polyethylene glycol), and suitable mixtures thereof.

Sterile injectable solutions can be prepared by introducing the cells into a solvent, e.g., mixed with a suitable carrier, diluent, or excipient such as sterile water, physiological saline, glucose, dextrose, and the like. The compositions may contain auxiliary substances such as wetting, dispersing or emulsifying agents (e.g., methylcellulose), pH buffers, gelling or thickening additives, preservatives, flavorings and/or colorants, depending on routes of administration and desired preparations. In some respects, reference can be made to standard texts for the preparation of suitable preparations.

Various additives may be added that enhance the stability and sterility of the composition, including antimicrobial preservatives, antioxidants, chelators, and buffers. Prevention of microbial action can be ensured by various antibacterial and antifungal agents, such as parabens, chlorobutanol, phenol and sorbic acid. Prolonged absorption of the injectable pharmaceutical form may be brought about by the use of agents which delay absorption such as aluminum monostearate and gelatin.

Formulations for in vivo administration are generally sterile. Sterility can be easily achieved by, for example, filtration through a sterile membrane.

Also provided are methods of administering cells, populations and compositions to treat or prevent diseases, conditions and disorders (including cancer), and the use of such cells, populations and compositions to treat or prevent diseases, conditions and disorders (including cancer). In some embodiments, cells, populations and compositions are administered to a subject or patient suffering from a particular disease or condition to be treated, such as by adoptive cell therapy (e.g., adoptive T cell therapy). In some embodiments, cells and compositions (e.g., engineered compositions and End products produced after incubation and/or other processing steps) prepared by the provided methods are administered to a subject, e.g., suffering from diseases or conditions or subjects at risk of diseases or conditions. In some aspects, methods thereby treat, e.g., one or more symptoms of diseases or conditions, e.g., by reducing tumor burden in a cancer that expresses an antigen recognized by engineered T cells.

The method of cell administration for adoptive cell therapy is known and can be combined with the methods and compositions provided. For example, the methods of adoptive T cell therapy are described in U.S. Patent Application No. 2003/0170238 for example; U.S. Patent No. 4,690,915 of Rosenberg; and Rosenberg (2011) Nat Rev Clin Oncol.8(10):577-85). Details may be seen in, e.g., Themeli et al. (2013), Nat Biotechnol.31(10): 928-933; Tsukahara et al. (2013), Biochem Biophys Res Commun 438(1): 84-9; Davila et al. (2013) PLoS ONE 8(4): e61338.

As used herein, "subject" refers to a mammal, such as a human or other animal, and generally is human. In some embodiments, the subject, e.g., patient, to whom the cells, cell populations, or compositions are administered is a mammal, generally a primate, such as a human. In some embodiments, the primate is a monkey or an ape. The subject can be male or female and can be any suitable age, including infant, juvenile, adolescent, adult, and geriatric subjects. In some embodiments, the subject is a non-primate mammal, such as a rodent.

As used herein, "treatment" (and grammatical variations thereof such as "treat" or "treating") refers to complete or partial amelioration or reduction of a disease or condition or disorder, or a symptom, adverse effect or outcome, or phenotype associated therewith. Desirable effects of treatment include, but not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. These terms do not imply complete curing of a disease or complete elimination of any symptom or effect(s) on all symptoms or outcomes.

As used herein, "Delaying the development of a disease" refers delaying, hindering, slowing, retarding, stabilizing, inhibiting and/or delaying the development of a disease (e.g., cancer). This delay can be of varying lengths of time, depending on the history of the disease and/or the individual being treated. It will be apparent to those skilled in the art that a sufficient or significant delay may actually include prevention because the individual does not develop the disease. For example, the development of advanced cancer such as metastasis may be delayed.

As used herein, "Preventing" includes providing prophylaxis with respect to the occurrence or recurrence of a disease in a subject that may be predisposed to the disease but has not yet been diagnosed with the disease. In some embodiments, the provided cells and compositions are used to delay the development of a disease or to slow the progression of a disease.

As used herein, "inhibiting" a function or activity refers to reduce the function or activity when compared to the same condition (other than the condition or parameter of interest,) or compared to another condition. For example, the cells inhibiting the growth of a tumor reduce the growth rate of the tumor compared to the growth rate of the tumor in the absence of the cells.

In the context of administration, an "effective amount" of an agent, e.g., a pharmaceutical formulation, cells, or composition, refers to an amount effective, at dosages/amounts and for periods of time necessary, to achieve a desired result, such as a therapeutic or prophylactic result.

A "therapeutically effective amount" of an agent, e.g., a pharmaceutical formulation or cells, refers to an amount effective, at dosages and for periods of time necessary, to achieve a desired therapeutic result, such as for treatment of a disease, condition, or disorder, and/or pharmacokinetic or pharmacodynamic effect of the treatment. The therapeutically effective amount may vary according to factors such as the disease state, age, sex, and weight of the subject, and the populations of cells administered. In some embodiments, the provided methods involve administering the cells and/or compositions at effective amount, e.g., therapeutically effective amount.

A "prophylactically effective amount" refers to an amount effective, at dosages and for periods of time necessary, to achieve the desired prophylactic result. Typically but not necessarily, since a prophylactic dose is used in subjects prior to or at an earlier stage of disease, the prophylactically effective amount will be less than the therapeutically effective amount. In the context of lower tumor burden, the prophylactically effective amount in some aspects will be higher than the therapeutically effective amount.

In certain embodiments, the cells or individual populations or subtypes of cells are administered to the subject in the range of about one million to about one hundred billion cells, such as, 1 million to about 50 billion cells (e.g., about 5 million cells, about 25 million cells, about 500 million cells, about 1 billion cells, about 5 billion cells, about 20 billion cells, about 30 billion cells, about 40 billion cells, or any of the range defined by the above two values), such as about 10 million to about 100 billion cells(e.g., about 20 million cells, about 30 million cells, about 40 million cells, about 60 million cells, about 70 million cells, about 80 million cells, about 90 million cells, about 10 billion cells, about 25 billion cells, about 50 billion cells, about 75 billion cells, about 90 billion cells, or any of the range defined by the above two values), and in some cases, about 100 million cells to about 50 billion cells (e.g., about 120 million cells, about 250 million cells, about 350 million cells, about 450 million cells, about 650 million cells, about 800 million cells, about 900 million cells, about 3 billion cells, about 30 billion cells, about 45 billion cells) or any value between these ranges.

In some embodiments, the dose of total cells and/or individual subsets of cells is in the range between at or about 10⁴ cells/kg body weight to at or about 10⁹ cells/kg body weight, such as between 10⁵ and 10⁶ cells/kg body weight, for example, at least or at least about or at or about 1 × 10⁵ cells/kg, 1.5 × 10⁵ cells/kg, 2×10⁵ cells/kg or 1×10⁶ cells/kg body weight. For example, in some embodiments, the cells are administered between at or about 10⁴ and at or about 10⁹ T cells/kilogram (kg) body weight or within a certain margin of error, such as between 10⁵ and 10⁶ T cells/kg body weight, such as at least or at least about or at or about 1×10⁵ T cells/kg, 1.5×10⁵ T cells/kg, 2×10⁵ T cells/kg, or 1×10⁶ T cells/kg body weight.

Cells can be administered in any suitable way, e.g., by bolus, by injection, such as intravenous or subcutaneous injection, intraocular injection, periocular injection, subretinal injection, intravitreal injection, transseptal injection, subscleral injection, intrachoroidal injection, anterior intrachamber injection, subperineal injection, subconjunctival injection, sub-Tenon injection, retrobulbar injection, peribulbar injection, or posterior juxtascleral. In some embodiments, they are administered parenteral, intrapulmonary and intranasal, and if local treatment is desired, by intralesional administration. Parenteral infusions include intramuscular, intravenous, intraarterial, intraperitoneal, or subcutaneous administration. In some embodiments, a given dose is administered by administering cells in a single bolus. In some embodiments, it is administered by multiple boluses of administered cells, for example, in a period of not more than 3 days, or by continuous infusion of administered cells.

In some embodiments, a repeated-dose method is provided, wherein a first dose of cells is administered, followed by one or more second consecutive doses. When administered to subjects with adoptive therapy, the timing and size of multiple doses of cells are usually designed to increase the efficacy and/or activity and/or function of antigen-expressing T cells (e.g., CAR-expressing T cells). In some embodiments, repeated administration reduces the down-regulation or inhibitory activity that can occur when inhibitory immune molecules such as PD-1 and/or PD-L1 are upregulated on antigen-expressing T cells, e.g., CAR-expressing T cells. The method includes administering the first dose, usually followed by one or more consecutive doses, and having a specific time frame between different doses.

In the context of adoptive cell therapy, the administration of a given "dose" includes the administration of a given amount or number of cells as a single composition and/or as a single uninterrupted administration (e.g., as a single injection or continuous infusion), and also includes the administration of a given amount or number of cells provided in multiple individual compositions or infusions as divided doses in a specific period of time (not exceeding 3 days). Thus, in some cases, the first or consecutive dose is a single or consecutive administration of a specified number of cells administered or initiated at a single time point. However, in some cases, the first or consecutive doses are administered as multiple injections or infusions in a period of not more than three days, such as three or two days, once daily, or as multiple infusions over a single day.

In the following detailed description of the present disclosure embodiments, reference is made to the accompanying drawings. In the drawings, like reference numerals indicate similar elements, and there is shown by way of illustration specific embodiments in which the disclosure may be practiced. These embodiments are described in sufficient detail to enable those skilled in the art to practice the disclosure. In other instances, well-known processes, structures and techniques have not been shown in detail so as not to obscure the understanding of this specification. Therefore, the following detailed description should not be construed as limiting, and the technical solution of the present disclosure is limited only by the appended claims.

### Examples

Example 1: design of CNK-UT multi-functional complex.

In this example, four CNK-UT multi-functional complexes were designed. The first structure is the basic CNK-UT multi-functional complex, and the other three structures are CNK-UT multi-functional complexes with more functions formed by adding additional modules on the first structure.

### 1.1 a basic CNK-UT multi-functional complex

In some embodiments, a basic CNK-UT multi-functional complex is used, as shown schematically in Fig. 1A, including 3 modules: (1) NK activating receptor module, (2) CNK signal transduction module, and (3) UT module. Optionally, a hinge or linker is included among the NK activating receptor module, the CNK signal transduction module, and/or the UT module.

Wherein,
(1) a NK activating receptor module, comprising at least a NK-cell-activating receptor or a functional variant thereof, wherein the NK-cell-activating receptor comprises: (a) an extracellular domain (ED) of the NK-cell-activating receptor or a functional variant thereof, (b) a transmembrane domain (TMD) of the NK-cell-activating receptor or a functional variant thereof, and (c) an intracellular domain (ICD) of the NK-cell-activating receptor or a functional variant thereof; and optionally, a hinge or linker is included among the extracellular domain of the NK-cell-activating receptor or a functional variant thereof, the transmembrane domain of NK-cell-activating receptor or a functional variant thereof, and/or the intracellular domain of the NK-cell-activating receptor or a functional variant thereof;

The NK-cell-activating receptor is selected from NKG2D, NKG2C, NKG2E, NKG2F, NKG2H, CD94, KIR2DL4, KIR2DS1, KIR2DS2, KIR2DS4, KIR3DS1, natural cytotoxicity receptor (NCR), TRAIL, signal lymphocytic activation molecule (SLAM) family molecules 2B4 (also known as CD244), DNAX attachment molecule 1 (DNAM-1, also known as CD226), CD16a, 2B4, NTB-A, CRACC (CS1) and NKp80, wherein the natural cytotoxic receptor includes NKp46 (also known as NCR1 or CD335), NKp44 (also known as NCR2 or CD336) and NKp30 (also known as NCR3 or CD337).

In some preferred embodiments, the CNK multi-functional complex includes an amino acid sequence selected from SEQ ID NOs: 1~24.

(2) a CNK signal transduction module, comprising at least (i) a NK cell signal adaptor or a functional variant thereof, wherein the NK cell signal adaptor comprises: (a) an extracellular domain (ED) of the NK cell signal adaptor or a functional variant thereof, (b) a transmembrane domain (TMD) of the NK cell signal adaptor or a functional variant thereof, and (c) an intracellular domain (ICD) of the NK cell signal adaptor or a functional variant thereof; and wherein, optionally, a hinge or linker is included among the extracellular domain of the NK cell signal adaptor or a functional variant thereof, the transmembrane domain of NK cell signal adoptor or a functional variant thereof, and/or the intracellular domain of the NK cell signal adaptor or a functional variant thereof.

The NK cell signal adaptor in the CNK signal transduction module is DAP10 or DAP12.

In some preferred embodiments, the functional variant of CNK cell signal adaptor is selected from a DAP10 mutant or a DAP 12 mutant, or a fusion protein of DAP10 and DAP12, or a fusion protein of wild-type DAP10 or DAP12 with a mutant type DAP10 or DAP12.

The functional variant of CNK cell signal adaptor is selected from a DAP10 mutant or a DAP12 mutant, or a fusion protein of DAP10 and DAP12, or a fusion protein of wild-type DAP10 or DAP12 with a mutant type DAP10 or DAP12.

In some preferred embodiments, the CNK signal transduction module further includes (ii) immunoreceptor activation signal transduction domain (ITAM) and/or (iii) T cell co-stimulatory signal transduction domain.

In some preferred embodiments, a hinge or linker is included among the NK cell signal adaptor or a functional variant thereof, the immunoreceptor activation signal transduction domain (ITAM), and/or the T cell co-stimulatory signal transduction domain; preferably, the NK cell signal adaptor or a functional variant thereof is fused with the immunoreceptor activation signal transduction domain (ITAM).

In some preferred embodiments, the immunoreceptor activation signal transduction domain (ITAM) derives from an intracellular activation signal transduction domain of an immunoreceptor; preferably, the immunoreceptor is selected from TCRζ, CD2, CD3γ, CD3δ, CD3ε, CD3ζ, CD5, CD22, FcRy, CD66d, FcαRI, FcyRI, FcγRII, FcyRIII, Dectin-1, CLEC-1, CD72, CD79A, CD79B; preferably, the immunoreceptor activation signal transdution domain (ITAM) is fused with the NK cell signal adaptor or a functional variant thereof; and preferably, the immunoreceptor is CD3ζ.

In some preferred embodiments, the T cell co-stimulatory signal transduction domain is derived from an intracellular signal transduction domain of a co-simulation molecule.

In some preferred embodiments, the CNK signal transduction module includes an amino acid sequence selected from SEQ ID NOs: 25-45.

(3) a UT module, comprising at least (i) a recombinant protein molecule for targeted degradation of TCR, MHC, and/or a NK cell target target, or a functional variant thereof, wherein the recombinant protein molecule for targeted degradation of TCR, MHC, and/or a NK cell target target comprises: (a) a binding protein molecular domain targeting TCR, MHC, and/or a NK cell target, or a functional variant thereof, (b) a transmembrane domain of a viral endoplasmic reticulum (ER) resident glycoprotein, or a functional variant, and (c) a cytoplasmic domain of a viral endoplasmic reticulum resident glycoprotein, or a functional variant; the transmembrane domain of the viral endoplasmic reticulum resident glycoprotein or a functional variant thereof and the cytoplasmic domain of the viral endoplasmic reticulum resident glycoprotein or a functional variant thereof form an ERAD degradation domain; optionally, the binding protein molecular domain targeting TCR or a functional variant thereof, the transmembrane domain of the viral endoplasmic reticulum resident glycoprotein or a functional variant thereof and/or the cytoplasmic domain of the viral endoplasmic reticulum resident glycoprotein or a functional variant thereof comprise a hinge or linker.

In the recombinant protein molecule for targeted degradation of TCR of the UT module, the binding protein molecular domain targeting TCR or a functional variant thereof is derived from a TCR antibody or a functional fragment thereof or the combination thereof.

In some preferred embodiments, the antibody is selected from a TCRα antibody, a TCRβ antibody, a TCRαβ antibody, a TCRγ antibody, a TCRδ antibody, a TCRγδ antibody, a TCR Vδ2 antibody, a TCR Cβ1 antibody; the functional fragment of the antibody is selected from Fd, Fv, Fab, Fab', F(ab')2, Fv(scFv), single chain antibody (scFv) or nanobody, double-stranded antibody, triple-standed antibody and tetra-stranded antibody; preferably, the TCR antibody is a single-chain TCR antibody. In some preferred embodiments, the TCR-targeted binding protein molecule is a TCRαβ antibody. In some preferred embodiments, the amino acid sequence of the single chain TCR antibody includes an amino acid sequence having 80% or more identity, preferably 85%, 90%, 95%, 96%, 97%, 98%, 99% or more identity, and more preferably 98%, 99% or more identity to the amino acid sequence shown in SEQ ID NO: 116; and the amino acid sequence of the full-length sequence of the single chain TCR antibody is shown as in SEQ ID NO: 116.

In some preferred embodiments, the ERAD degradation domain in the UT module is from HCMV glycoprotein US2, US3, US11 or US 10, adenovirus E3-19K or HHV-7US21.

In some preferred embodiments, the ERAD degradation domain in the UT module comprises the amino acid sequence selected from SEQ ID NOs: 46-63.

Gene element fragments expressing various modules of the basic CNK-UT multi-functional complex can be synthesized by gene synthesis techniques. With a polycistronic expression scheme, self-cleaving 2A peptide (2A: T2A, p2A, E2A, F2A) and other elements are used to link gene element fragments of different modules, to obtain the gene fragment of the basic CNK-UT multi-functional complex; the synthesized gene fragment of the basic CNK-UT multi-functional complex is cloned into a lentiviral vector through molecular cloning technology, and transfected into T cells to achieve simultaneous expression of multiple different elements, thereby not only allowing the T cells specifically recognize and kill the NK targetsns, but also effectively inhibiting and degrading TCR via UT elements. Alternatively, simultaneous expression of multiple CNK-UT elements can be achieved by placing different functional elements under different promoters inthe same lentiviral vector and transfecting T cells by the vector; or, simultaneous expression of multiple CNK-UT elements can also be achieved by placing different functional elements into two lentivirus and transfecting T cells by both vectors at the same time.

### 1.2 a CNK-UT multi-functional complex further comprising an MHC-targeted binding protein molecular domain

In some embodiments, a CNK-UT multi-functional complex that simultaneously degrades or inhibits TCR and MHC I and/or MHC II is used, a schematic diagram of which is shown in Fig. 1B, which also includes three modules: (1) a NK activating receptor module; (2) a CNK signal transduction module; and (3) a UT module comprising a recombinant protein molecular domain for targeted degradation of TCR or a functional variant thereof,a binding protein molecular domain for targeted degradation of MHC I and/or MHC II or a functional variant thereof. Compared with the basic CNK-UT multi-functional complex, it is different in that (3) the UT module further comprises abinding protein molecular domain for targeting MHC I, or a functional variant thereof. Optionally, the NK activating receptor module, the CNK signal transduction module and the UT module are linked with a hinge or a linker.

In some embodiments, the MHC I- and/or MHC II-targeted binding protein molecular domain targeting or a functional variant thereof is a binding protein molecular domain targeting HLA or a functional variant thereof.

In some embodiments, the MHC I-targeted binding protein molecular domain may be derived from a viral endoplasmic reticulum protein that inhibits MHC I molecules. In some embodiments, the viral endoplasmic reticulum glycoprotein that inhibits MHC I molecules is selected from human cytomegalovirus (HCMV) US6, herpes simplex virus (HSV) ICP47, cowpox virus (CPXV) CPXV12, bovine herpesvirus (BHV) UL49.5, or Epstein Barr virus (EBV) BNFL2a, *etc.* The above viral endoplasmic reticulum protein binds to the transporter associated with antigen processing (TAP), thereby preventing TAP-mediated peptide transport to the endoplasmic reticulum, inhibiting the assembly of MHC molecules, and achieving the inhibition of MHC I expression. In some preferred embodiments, the MHC I- and/or MHC II-targeted binding protein molecular domain or a functional variant thereof includes a TAP binding domain. In some preferred embodiments, the TAP binding domain contains amino acid sequences selected from SEQ ID NOs: 64-73.

In some embodiments, the MHC I-targeted binding protein molecular domain may also be derived from a viral glycoprotein that degrades MHC I molecules. In some embodiments, the viral glycoprotein is selected from the group consisting of HCMV glycoprotein US2, US3, US11, US10, adenovirus E19, and the like. In some preferred embodiments, the MHC I- and/or MHC II-targeted binding protein molecular domain or a functional variant thereof includes a MHC binding domain. In some preferred embodiments, the MHC binding domain contains amino acid sequences selected from SEQ ID NOs: 74-82.

In some embodiments, the MHC I-targeted binding protein molecular domain can also be derived from viral proteins that targetedly inhibit or degrade NK target proteins such as MICA, MICB, ULBP1-6, *etc.* In some embodiments, the viral protein is selected from HCMVUL16, UL141, UL142, adenovirus E3-19K9, and the like. In some preferred embodiments, the amino acid sequence of the viral protein is shown in SEQ ID NOs: 83-91.

In some embodiments, the MHC I-targeted binding protein molecular domain may also be derived from a viral protein that transports MHC I molecules from the Golgi apparatus to lysosomes for degradation. In some preferred embodiments, the viral protein is selected from HIV Nef, HIV Vpu, HHV-7 U21, HHV-8 KK3, HHV-8 KK5, MHV-68 MK3, HTLV-1 p12, *etc.* In some preferred embodiments, the amino acid sequence of the viral protein contains the amino acid sequence shown in SEQ ID NOs: 92-97.

In some embodiments, the MHC I- and/or MHC II-targeted binding protein molecular domain or a functional variant thereof further includes a viral protein that mediates the return of MHC-polypeptide molecules from the Golgi apparatus to the endoplasmic reticulum and promotes the degradation of MHC- polypeptide molecules; preferably, the viral protein comprises an MHC binding structure and a KDEL receptor binding domain. In some embodiments, the viral protein is Cowpox virus protein CPXV203. In some preferred embodiments, the viral protein contains the amino acid sequence shown in SEQ ID NOs: 98-103.

The gene fragment of the CNK-UT multi-functional complex further comprising a MHC I-targeted binding protein molecular domain was obtained in the same manner as obtaining the gene fragment of the basic CNK-UT multi-functional complex. The synthesized gene fragment of the CNK-UT multi-functional complex was cloned into a lentiviral vector by molecular cloning, transfected into T cells, andused to simultaneously express the multiple different elements, thereby enabling the T cells not only to specifically recognize and kill the NK target, but also to effectively inhibit and degrade TCR and MHC I expression by way of the UT elements.

### 1.3 a CNK-UT multi-functional complex further comprising botha MHC I-targeted binding protein molecular domain and a chimeric adaptor

In some embodiments, a multi-functional complex that simultaneously degrades TCR and MHC I and enables CNK-T cell recognition and activation against tumor antigen specificity is used, a schematic diagram of which is shown in Fig. 1C, which includes four modules (1) a NK activating receptor module; (2) a CNK signal transduction module; (3) a UT module; and (4) a chimeric adaptor module. Compared with the CNK-UT multi-functional complex having an additional MHC I-targeted binding protein molecules domain, it is different in the addition of (4) a chimeric adaptor module. Optionally, the NK activating receptor module, the CNK signal transduction module, the UT module, and/or the chimeric adaptor module are linked with a hinge or a linker.

The (4) chimeric adaptor module comprises: (i) a tumor-targeted extracellular recognition domain, (ii) a transmembrane domain, and (iii) an intracellular signal transduction domain. Optionally, a hinge or a linker is included among the tumor-targeted extracellular recognition domain, the transmembrane domain and/or the intracellular signal transduction domain;

In some embodiments, the tumor-targeted extracellular recognition domain of the chimeric adaptor is selected from a tumor antigen-specific binding domain, a tumor microenvironment target antigen binding domain, and/or a chemokine receptor targeting tumor microenvironment.

In some embodiments, the tumor-targeted extracellular recognition domain is selected from an antibody capable of targeting a tumor-associated antigen or a functional fragment thereof, a TCR or the combination thereof. The functional fragment of the antibody is selected from Fd, Fv, Fab, Fab', F(ab') 2, Fv (scFv), single-chain antibody (scFv) or nanobody, double-stranded antibody, triple-stranded antibody and tetra-stranded antibody.

In some embodiments, the transmembrane domain of the chimeric adaptor is selected from the NK cell activating receptor transmembrane domain, DAP10 transmembrane domain, DAP12 transmembrane domain, CD8 transmembrane domain, CD28 transmembrane domain, CD4 transmembrane domain, 4-1BB transmembrane domain, OX40 transmembrane domain, ICOS transmembrane domain, CTLA-4 transmembrane domain, PD-1 transmembrane domain, LAG-3 transmembrane domain, 2B4 transmembrane domains, and BTLA transmembrane domain, as well as the combination thereof; preferably, the NK cell activating receptor is selected from KG2D, NKG2C, NKG2E, NKG2F, NKG2H, CD94, KIR2DL4, KIR2DS1, KIR2DS2, KIR2DS4, KIR3DS1, natural cytotoxic receptor, TRAIL, DNAM-1, CD16a, 2B4, NTB-A, CRACC, and NKp80; preferably, the natural cytotoxic receptor is selected from Nkp46, Nkp44, and Nkp30.

In some embodiments, the intracellular signal domain of the chimeric adaptor includes an intracellular signal transduction domain of the NK cell activating receptor and/or a co-stimulatory signal transduction domain.

In some embodiments, the NK cell activating receptor is selected from NKG2D, NKG2C, NKG2E, NKG2F, NKG2H, CD94, KIR2DL4, KIR2DS1, KIR2DS2, KIR2DS4, KIR3DS1, a natural cytotoxic receptor, TRAIL, DNAM-1, CD16a, 2B4, NTB-A, CRACC, orNKp80.

In some embodiments, the intracellular signal domain further comprises a co-stimulatory signal transduction domain; preferably, the co-stimulatory signal transduction domain is selected from T cell co-stimulatory signal transduction domains, comprising but not limited to, the intracellular signal domain derived from MHC class I molecules, TNF receptor proteins, immunoglobulin-like proteins, cytokine receptors, integrin proteins, lymphocyte activation signal molecules (SLAM proteins), activated NK cell receptors, BTLA, Toll ligand receptors, OX40, CD2, CD7, CD16, CD27, CD28, CD30, CD40, CD38, CD35, CD79A, CD79B, CDS, ICAM-1, LFA-1, (CD11a/CD18), 4-1BB(CD137), B7-H3, CDS, ICAM-1, ICOS(CD278), GITR, BAFFR, LIGHT, HVEM(LIGHTR), KIRDS2, SLAMF7, NKp80(KLRF1), NKp44, NKp30, NKp46, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83-specific binding ligands, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, PTCH2 and the like; more preferably, the co-stimulatory signal transduction domain is selected from the NKG2D intracellular signal domain, DAP10 intracellular signal domain, DAP12 intracellular signal domain, NCR intracellular signal domain, CD28 intracellular signal domain, 4-1BB intracellular signal domain, OX40 intracellular signal domain, and ICOS intracellular signal domain.

The gene fragment of the CNK-UT multi-functional complex comprising an additional MHC I-targeted binding protein molecular domain and a chimeric adaptor was obtained in the same manner as obtaining the gene fragment of the basic CNK-UT multi-functional complex. The synthesized gene fragment of the CNK-UT multi-functional complex was cloned into a lentiviral vector by molecular cloning, transfected into T cells, and used to simultaneously express the four different elements, thereby enabling T cells not only to specifically recognize and kill the NK target, but also to effectively inhibit and degrade TCR and MHC I expression with the help of the UT elements, and also achieving specific recognition on tumor antigens and activation of CNK-T cells by way of the chimeric adaptor.

1.4 a CNK-UT multi-functional complex further comprising both a MHC I-targeted binding protein molecular domain and a receptor with targeted killing activity against tumor cells ( *e.g.*, CAR or TCR) In some embodiments, a multi-functional complex that is able to achieve the simultaneous degradation of TCR and MHC I as well as the tumor antigen-specific recognition and killing effect is used, a schematic diagram of which is shown in Fig. 1D, which includes four modules: (1) a NK activating receptor module, (2) a CNK signal transduction module, (3) a UT module, and (4) a receptor module with targeted killing activity against tumor cells. It differs from the CNK-UT multi-functional complex comprising MHC I-targeted binding protein molecular domain in that it further comprises (4) a receptor module with targeted killing activity against tumor cells. Optionally, the NK activating receptor module, the CNK signal transduction module, the UT module, and/or the receptor module with targeted killing activity against tumor cells are linked with a hinge or a linker.

The (4) receptor module with targeted killing activity against tumor cells includes: (i) a tumor antigen-targented extracellular recognition domain; (ii) a transmembrane domain; (iii) an intracellular costimulatory signal transdution domain; and (iv) a T cell activation signal transdution domain (ITAM); optionally, a hinge or a linker is contained among the tumor antigen-targented extracellular recognition domain, the transmembrane domain, the intracellular costimulatory signal transdution domain, and the T cell activation signal transdution domain (ITAM);
the receptor module with targeted killing activity against tumor cells is selected from CD8 transmembrane domain, α and/or β chain transmembrane domain of T cell receptor, CD28 transmembrane domain, CD3ε transmembrane domain, CD45 transmembrane domain, CD4 transmembrane domain, CD5 transmembrane domain, CD8 transmembrane domain, CD9 transmembrane domain, CD16 transmembrane domain, CD22 transmembrane domain, CD33 transmembrane domain, CD37 transmembrane domain, CD64 transmembrane domain, CD80 transmembrane domain, CD86 transmembrane domain, CD134 transmembrane domain, CD137 transmembrane domain, CD154 transmembrane domain, GITR transmembrane domain, and the combinations thereof.

The T cell activation signal transduction domain is derived from CD3ζ, common FcRy (FCER1G), FcyRIIa, FcRβ, CD3γ, CD3δ, CD3ε, CD5, CD22, CD79a, CD79b, CD278 ("ICOS"), FcεRI CD66d, DAP10 and DAP12 and other intracellular signal transduction domains.

In some preferred embodiments, the (4) receptor module with targeted killing activity against tumor cells is a CAR or TCR receptor with targeted killing activity against tumor cells.

The gene fragment of CNK-UT multi-functional complex further comprising both a MHC I-targeted binding protein molecular domain and a receptor with targeted killing activity against tumor cells (e.g., CAR or TCR receptor) was obtained in the same manner as obtaining the basic CNK-UT multi-functional complex. The synthesized gene fragment of the CNK-UT multi-functional complex was cloned into a lentiviral vector by molecular cloning, transfecting T cells, and used for simultaneously express the four different elements, thereby enabling T cells not only to specifically recognize and kill the NK target, but also to effectively inhibit and degrade TCR and MHC I with the help of the UT elements, and also to achiev the specific recognition and killing effect against tumor cells via the CAR or TCR structure as well as the increased clearanceefficiency for tumor cells by cooperating with the CNK elements.

Example 2: Design of CNK-UT element expressing the CNK-UT multi-functional complex.

In this example, four CNK-UT elements for expressing CNK-UT multi-functional complex were designed.

### 2.1 CNK-UT element structure design 1

In some embodiments, as shown in Fig. 2A, the expression of multiple functional elements by one single vector is achieved by using a single lentiviral expression vector with EF1α promoter and by linking the following combination of CNK-UT elements via elements such as the self-cleaving 2A peptide: DAP10-DAP12 ICD-T2A-NKG2D-p2A-anti-TCR-AdE3 ERAD.

### 2.2 CNK-UT element structure design 2

In some embodiments, as shown in Fig. 2B, the expression of multiple functional elements by one single vector is achieved by using a single lentiviral expression vector with EF1α promoter and by linking the following combination of CNK-UT elements via elements such as the self-cleaving 2A peptide: DAP10-DAP12 ICD-T2A-NKG2D-p2A-anti-TCR-US2 ERAD.

### 2.3 CNK-UT element structure design 3

In some embodiments, as shown in Fig. 2C, the expression of multiple functional elements by one single vector is achieved as follows: using one single lentiviral vector having the polycistronic expression system with double promoters, respectively regulating the expression of multi-genes DAP10-CD3ζ-T2A-NKG2D-p2A-anti-TAA scFv-DAP10 and anti-TCR-AdE3 ERAD-E2A-AdE3 by EF1α promoter and CMV promoter.

### 2.4 CNK-UT element structure design 4

In some embodiments, as shown in Fig. 2D, the expression of multiple functional elements in the same T cell is achieved as follows: using two different lentiviral expression vectors that respectively regulate the expression of multi-genes anti-TAA scFv-CD28/4-1BB-CD3ζ-T2A-DAP10-CD3ζ-T2A-NKG2D and anti-TCR-AdE3 ERAD-T2A-AdE3, preparing the different lentivirus and co-transfecting the T cells.

### Example 3: Preparation of expression plasmid of specific chimeric antigen receptor targeting NKG2DL

The nucleotide sequence of CNK-UT (the nucleotide sequence is shown in SEQ ID NO: 118; the encoded protein thereof is shown in SEQ ID NO: 117, wherein the functional fragment for the chimeric antigen antibody is linked by the order of DAP10-CD3ζ-T2A-NKG2D-p2A-anti-TCR scFv-AdE3) was obtained by whole gene synthesis, and inserted into the lentiviral vector pCDH-CMV-MCS-EF1α-Puro (purchased from Youbao Biotech) or pLVX-EF1α-AcGFP1-C1Vector (Takara) by molecular cloning, so as to construct the desired CNK-UT lentiviral vector. The plasmids (which were confirmed by sequencing) were used to transform DH5α competent cells (Thermo Fisher). Single colonies were picked and cultured in large-scale. Then, the plasmids were purified and produced by using the PureLinkTM HiPure Plasmid Maxiprep Kit (Thermo Fisher), to obtain the CNK-UT lentiviral plasmid.

### Example 4: Preparation of virus for CNK-UT

293T cells (provided by ATCC, Cat# CRL3216TM) were co-transfected by the CNK-UT lentiviral plasmid obtained in Example 3 as well as the packaging plasmids psPAX2 and pMD2.G (purchased from Addgene, Cat# 12259&12260) at the ratio of 1.64pmol: 1.3pmol: 0.72pmol. Polyethyleneimine (408727, Sigma) was used as the transfection reagent, at the ratio of DNA: µg PEI=1:3. The details for the preparation of the packaging plasmids can be found in the instruction of PureLinkTM HiPure Plasmid Maxiprep Kit (K210006, Thermo), other details of the transfection process can be found in the instruction of Sigma Transfections. 16 hours after transfection, culture medium was changed to the complete medium (purchased from Life Technologies, Cat# 11995-065). After culturing for 24 hours, 48 hours and 72 hours, the supernatants with lentivirus were collected, combined, and centrifuged at -80°C, 3000rpm for 10-15 minutes, then filtered through a 0.45µm filter membrane, and finally ultra-centrifuged at 25000rpm, 4°C for 2-3 hours, so as to obtain the concentrate of lentivirus. The lentivirus concentrate was collected and stored at -80°C.

### Example 5: Preparation of CNK-UT cells

Collect fresh peripheral blood from healthy donors, add PBS+EDTA 1: 1, and separate fresh peripheral blood mononuclear cells by Ficoll density gradient centrifugation. Then, CD3+ T cells were positively sorted and eluted by utilizing CD3 sorting magnetic beads, MS separation column and MiniMACS^{™} separation device (Miltenyi Biotec). The isolated CD3+ T cells were stimulated by culturing with anti-CD3/anti-CD28 antibody-conjugated magnetic beads (Human T-Activator CD3/CD28, Invitrogen, Cat# 11161D), according to the steps as follows: Dilute the peripheral blood mononuclear cells to a concentration of 1 × 10 ⁶ single cells/ml, spread them in a 24-well plate, add the magnetic beads to the cells at a ratio of 1:1 and mix well. The mixture was resuspended in culture medium (OpTmizerTM T-Cell Expansion SFM, A1048503, Life Technologies) containing IL2 50U/ml and IL15 5ng/ml, and cultured in a 37°C, 5% CO₂ incubator for 1 day. Then, the cells were transfected with the lentivirus prepared in Example 4 that loaded with the CNK-UT elements, according to the steps as follows: adding the lentivirus concentrate prepared in Example 4 to the cells (MOI=3-5), adding Polybrene 10µg/ml as well, centrifuging at a low speed (500g-1000g/min) for 30-60 minutes in a flat-angle centrifuge, then culturing the cells in an incubator at 37°C. Cells expressing CNK-UT were obtained 48 hours after infection, which may be used for cell phenotyping by flow cytometry. Cells cultured for another 8 days may be used for phenotype detection and cellular function experiments.

### Example 6: Phenotype detection of CNK-UT cells

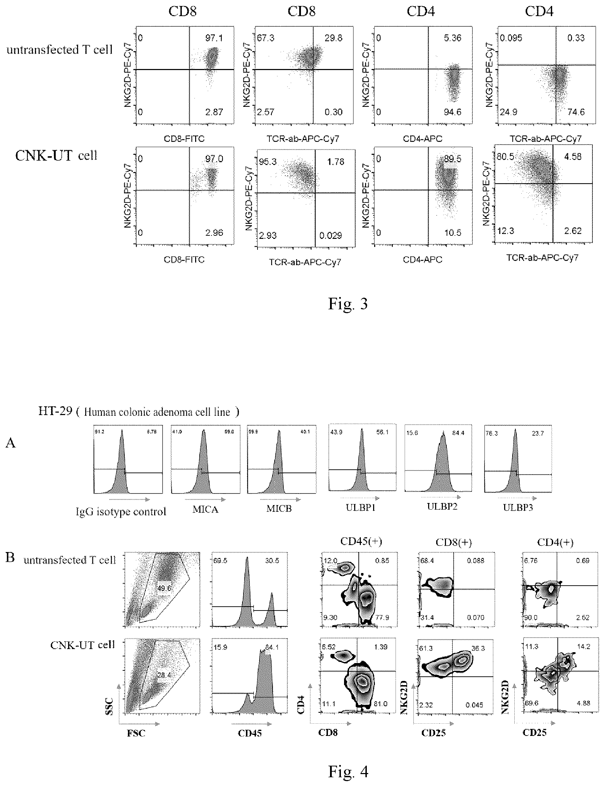

Fig. 3 illustrates the flow cytometry detection results of the basic phenotype of CNK-UT (DAP10-CD3ζ-T2A-NKG2D-p2A-anti-TCR scFv-AdE3) cells. Cells were cultured according to Example 2 for 10 days, and 5 × 10⁵ untransfected T cells and the cells transfected with CNK-UT virus vectorwere collected respectively. The cells were treated with antibodies CD8-Pacific Blue, CD4-APC, NKG2D-PE-Cy7, TCR-αβ-APC-Cy7 (all antibodies were purchased from Biolegend) for staining. Then, the stained cells underwent phenotyping by a flow cytometer (BD FACSCanto II). The results showed that for the untransfected T cells, the CD8 T cells express NKG2D and TCR-αβ, the CD4 T cells do not express NKG2D, but highly express TCR-αβ; while for the cells modified with transfected CNK-UT, both the CD8 and the CD4 T cells highly express NKG2D, but not express TCR-αβ.

### Example 7: Detection of broad-spectrum tumor-killing function of CNK-UT cells

In this example, the broad-spectrum tumor-killing function of the CNK-UT cells prepared according to Example 5 were studied. Human colonic adenoma cell line HT29 was purchased from Procell (CL-0118), triple negative breast cancer cell MDA-MB453 was purchased from Procell (CL-0152), acute myeloid leukemia cell line THP1 was purchased from Procell (CL-0233), hepatocellular carcinoma (HCC) HepG2 was purchased from Procell (CL-0103), hepatocellular carcinoma (HCC) PLC was purchased from Procell (CL-0415).

### 7.1 CNK-UT cells recognize and specifically kills human colon adenoma cell line HT29

The CNK-UT cells used in this example are the cells prepared in Example 5.

Fig. 4 shows the CNK-UT cells recognize and specifically kill human colonic adenoma cell line HT29. Wherein:
A: Expression of different NK target proteins in human colonic adenoma cell line HT29

For Human colonic adenoma cell line HT29, 0.5×10⁶ cells were digested with trypsin, and respectively treated with antibodies MICA-APC, MICB-APC, ULBP1-APC, ULBP2-APC, ULBP3-APC for staining, while the IgG isotype (purchased from R&D Systems) was used as the control. Then, the cell phenotypes were determined by a flow cytometer (BD FACSCanto II). The results showed that HT29 cells highly express ULBP2, but weakly express MICA, MICB, ULBP1 and ULBP3.

### B: Effective killing against HT29 and activation ability of CNK-UT cells

In the co-culture experiment of T cells and tumor cells, the human colonic adenoma cell line HT29 was counted and spread at 0.5×10⁶ cells/well in a 24-well plate. The cells were cultured until 80% confluent. Then, the untransfected T cells or the CNK-UT cells (DAP10-CD3ζ-T2A-NKG2D-p2A-ant-TCR scFv-AdE3) were added respectively at the effector-to-target ratio of 1:5. After 24 hours, all cells were digested, collected, and treated with antibodies CD45-PerCP-Cy5.5, CD8-Pacific Blue, CD4-APC, NKG2D-PE-Cy7, CD25-APC-Cy7 (purchased from Biolegend) for staining. The stained cells were detected by flow cytometry. To analyze the results, CD45(-) tumor cells and CD45(+) T cells were distinguished based on CD45, then the CD8 and CD4 T cells were analyzed by the surface expression of NKG2D and the activation marker CD25. The experimental data showed that 59.0% of HT29 cells have upregulated expression of MICA, 40.1% have upregulated expression of MICB, 56.1% have upregulated expression of ULBP1, 84.4% have upregulated expression of ULBP2, 23.7% have upregulated expression of ULBP3 (Fig. 4A), CNK-UT cells display effective killing activity on HT29 cells. In the co-culture system of the control group, 69.5% of the cells are tumor cells, T cells do not express CD25. However, in the co-culture system of CNK-UT cells, according to the FSC/SSC results, most tumor cells have been eliminated, the proportion of T cell has significantly increased upto 84.1%, 36.345% of CD8+T cells have upregulated expression of CD25, and 19.08% of CD4+ T cells have upregulated expression of CD25 (Fig. 4B).

### 7.2 CNK-UT cells recognize and specifically kill MDA-MB453

The CNK-UT cells used in this example are the cells prepared in Example 5.

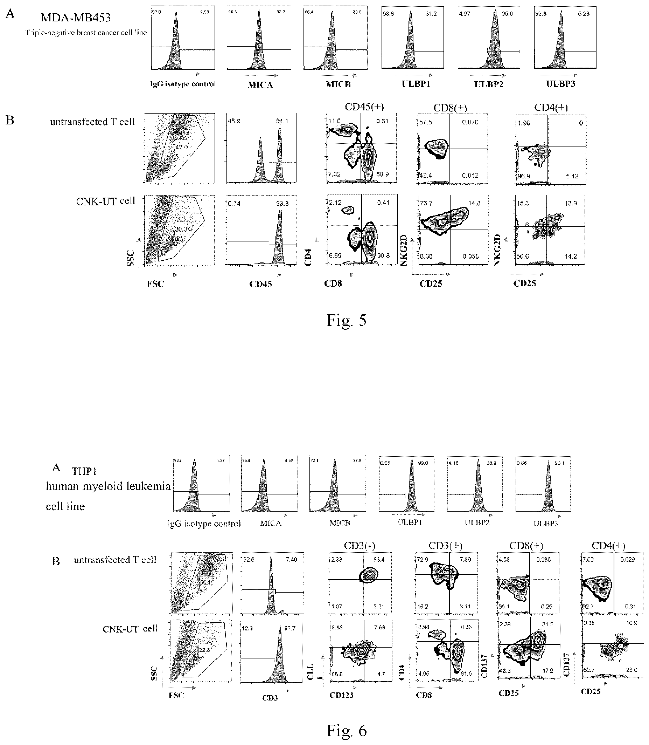

Fig. 5 shows the CNK-UT cells recognize and specifically kill MDA-MB453.
A: Expression of different NK target proteins in triple-negative breast cancer cell line MDA-MB453 For MDA-MB453, 0.5×10⁶ cells were digested with trypsin, and respectively treated with antibodies MICA-APC, MICB-APC, ULBP1-APC, ULBP2-APC, ULBP3-APC for staining, while the IgG isotype (purchased from R&D Systems) was used as the control. Then, the cell phenotypes were determined by a flow cytometer (BD FACSCanto II). The results showed that MDA-MB453 cells highly express MICA, ULBP2, but weakly express MICB, ULBP1 and ULBP3.
B: Effective killing against MDA-MB453 and activation ability of CNK-UT cells

In the co-culture experiment of T cells and tumor cells, the breast cancer cell line MDA-MB453 was counted and spread at 0.5×10⁶ cells/well in a 24-well plate. The cells were cultured until 80% confluent. Then, the untransfected T cells or the CNK-UT cells (DAP10-CD3ζ-T2A-NKG2D-p2A-ant-TCR scFv-AdE3) were added respectively at the effector-to-target ratio of 1:5. After 24 hours, all cells were digested, collected, and treated with antibodies CD45-PerCP-Cy5.5, CD8-Pacific Blue, CD4-APC, NKG2D-PE-Cy7, CD25-APC-Cy7 (purchased from Biolegend) for staining. The stained cells were detected by flow cytometry. The experimental data showed that 83.7% of MDA-MB453 cells have upregulated expression of MICA, 33.6% have upregulated expression of MICB, 31.2% have upregulated expression of ULBP1, 95.0% have upregulated expression of ULBP2, 6.23% have upregulated expression of ULBP3 (Fig. 5A), CNK-UT cells display effective killing activity on MDA-MB453 cells. In the co-culture system of the control group, 48.9% of the cells are tumor cells, T cells do not express CD25. However, in the co-culture system of CNK-UT cells, tumor cells only account for 6.74%, 14.8% of CD8+T cells have upregulated expression of CD25, and 28.1% of CD4+ T cells have upregulated expression of CD25 (Fig. 5B). The experimental data indicated that CNK-UT cells have good killing effect on MDA-MB453 cells, and achieving specific activation.

### 7.3 CNK-UT cells recognize and specifically kill THP1

The CNK-UT cells used in this example are the cells prepared in Example 5.

Fig. 6 illustrates that CNK-UT cells recognize and specifically kill THP1.
A: Expression of different NK targets in acute myeloid leukemia cells THP1
   0.5×10⁶ THP1 cells from centrifuge were respectively treated with antibodies MICA-APC, MICB-APC, ULBP1-APC, ULBP2-APC, ULBP3-APC for staining, while the IgG isotype (purchased from R&D Systems) was used as the control. The results showed that THP1 cells highly express ULBP1, ULBP2, and ULBP3, but weakly express MICA and MICB.
B: Effective killing against THP1 and activation ability of CNK-UT cells

In the co-culture experiment of T cells and tumor cells, the AML cell line THP1 was counted and spread at 0.5×10⁶ cells/well in a 24-well plate. The untransfected T cells or the CNK-UT cells (DAP10-CD3ζ-T2A-NKG2D-p2A-ant-TCR scFv-AdE3) were added respectively at the effector-to-target ratio of 1:5 into the tumor cell. After 24 hours, all cells were centrifuged, collected, and treated with antibodies CD45-PerCP-Cy5.5, CD8-Pacific Blue, CD4-APC, CD137-PE-Cy7, CD25-APC-Cy7 (purchased from Biolegend) for staining. The stained cells were detected by flow cytometry. The experimental data showed that 27.9% of THP1 cells have upregulated expression of MICB, 99.0% have upregulated expression of ULBP1, 95.8% have upregulated expression of ULBP2, 99.1% have upregulated expression of ULBP3 (Fig. 6A), CNK-UT cells display effective killing activity on THP 1 cells. In the co-culture system of the control group, 92.6% of the cells are tumor cells, T cells do not have upregulated expression of CD25. However, in the co-culture system of CNK-UT cells, tumor cells only account for 12.3%, 49.1% of CD8+T cells have upregulated expression of CD25, 33.59% of CD8+T cells have upregulated expression of CD137, 33.9% of CD4+ T cells have upregulated expression of CD25, and 11.28% of CD4+ T cells have upregulated expression of CD137. Experimental results confirmed that CNK-UT cells can effectively kill THP1 cells, eliminate THP1 from the co-culture system, and achieve specific activation, and upregulate the expression of T cell activating markers such as CD25 and CD137.

### 7.4 CNK-UT may cooperate with conventional CAR-T technology to enhance the recognition and specific killing of tumor cells

The CNK-UT cells used in this example are the cells prepared in Example 5.

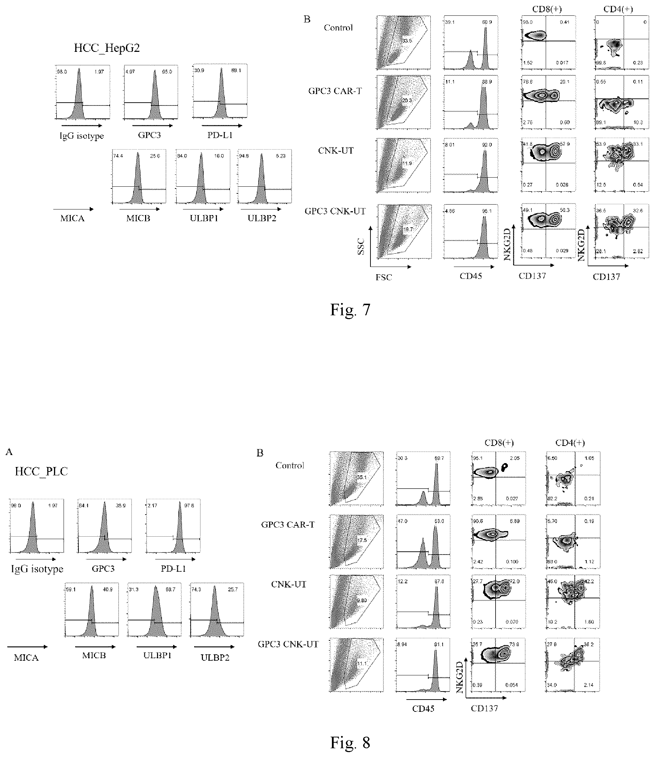

Fig. 7 illustrates that CNK-UT cells can upgrade conventional CAR-T technology to achieve more powerful targeted killing and activation capabilities.
A: Expression of GPC3, PD-L1 and different NK targets in hepatocellular carcinoma (HCC) HepG2 0.5×10⁶ HepG2 cells from trypsin digestion, and respectively treated with antibodies GPC3-APC, PD-L1-APC, MICA-APC, MICB-APC, ULBP1-APC, ULBP2-APC, ULBP3-APC for staining, while the IgG isotype (purchased from R&D Systems) was used as the control. Then, the cell phenotypes were determined by a flow cytometer (BD FACSCanto II). The results showed that HepG2 cells highly express GPC3, but weakly express PD-L1, MICA, MICB, ULBP1 and ULBP2. B: CAR/CNK-UT cells show more efficient killing against HepG2 cells and activation ability than conventional CAR-T cells

Preparation of GPC3 CAR-T cells: by reference to Examples 3 to 5, the anti-GPC3 41BB-CD3ζ nucleotide sequence (SEQ ID NO: 120, the amino acid sequence encoded thereby is shown in SEQ ID NO: 119) was obtained by whole gene synthesis, and linked into the lentiviral vector pCDH-CMV-MCS-EF1α-Puro through molecular cloning. Then, the plasmid and lentivirus were prepared and used for transfecting T cells. The conventional GPC3 CAR-T cells were obtained.

Preparation of GPC3 CAR/CNK-UT cells: by reference to Examples 3 to 5, the anti-GPC3 41BB-CD3ζ-T2A-DAP10-CD3ζ-T2A-NKG2D-p2A-anti-TCR scFv-AdE3 nucleotide sequence (SEQ ID NO: 122, the amino acid sequence encoded thereby is shown in SEQ ID NO: 121) was obtained by whole gene synthesis, and linked into the lentiviral vector pCDH-CMV-MCS-EF1α-Puro through molecular cloning. Then, the plasmid and lentivirus were prepared and used for transfecting T cells. After expansion, the GPC3 CAR/CNK-UT cells were obtained.

In the co-culture experiment of T cells and tumor cells, cells were counted and spread at 0.5×10⁶ cells/well in a 24-well plate. The cells were cultured until 80% confluent. Then, the conventional GPC3 CAR-T (anti-GPC3-41BB-CD3ζ) cells, the CNK-UT cells (DAP10-CD3ζ-T2A-NKG2D-p2A-anti-TCR scFv-AdE3), and the GPC3 CAR/CNK-UT cells (anti-GPC3 41BB-CD3ζ-T2A-DAP10-CD3ζ-T2A-NKG2D-p2A-anti-TCR scFv-AdE3) were respectively added at the effector-to-target ratio of 1:5 into the tumor cell. After 24 hours, all cells were digested, collected, and treated with antibodies CD45-PerCP-Cy5.5, CD8-Pacific Blue, CD4-APC, NKG2D-PE, and CD137-PE-Cy7 (purchased from Biolegend) for staining. The stained cells were detected by flow cytometry. All cells were digested and detected by flow cytometry technology. Tumor cells and T cells were distinguished based on CD45, and then the expression of activation markers CD137 on surface of CD8 and CD4 cells were also analyzed. Experimental data showed that 95.0% of HepG2 cells highly expressed GPC3, 69.1% of cells have upregulated expression of PD-L1, 18.2% of cells express MICA, 25.6% of cells upregulated MICB, 16.0% of cells upregulated ULBP1, and 5.23% of cells have upregulated expression of ULBP2. After 24 hours of co-culture with E:T=1:5, in the co-culture system with the control T cell, 39.1% of the cells were tumor cells, neitherCD4+ nor CD8+ T cells upregulated CD25; in the co-culture system with GPC3 CAR-T, tumor cells only accounted for 11.1%, 20.1% CD8+ T cells had upregulated expression of CD137; 10.3% CD4+ T cells had upregulated expression of CD137; in the co-culture system with CNK-UT, tumor cells only accounted for 8.01%, and 57.9% CD8+ T cells had upregulated expression of CD137; 33.1% CD4+T cells had upregulated expression of CD137; in the co-culture system with GPC3 CAR/CNK-UT, tumor cells only accounted for 4.86%, and 50.3% CD8+T cells had upregulated expression of CD137; 35.42% CD4+T cells had upregulated expression of CD137. Therefore, CNK-UT cellsare superior to conventional CAR-T cells, in killing HepG2 cells as well as specific activation and upregulation of CD137. GPC3 CAR/CNK-UT cells that integrate GPC3 41BB-Z CAR and CNK-UT modules have even higher ability of effective tumor killing and activation.

### 7.5 Targeted killing against PD-L1 high-expressed tumor cells and activation ability of CNK-UT cells The CNK-UT cells used in this example are the cells prepared in Example 5.

Fig. 8 illustrates that CNK-UT cells can upgrade conventional CAR-T technology to achieve targeted killing and activation ability in case of tumor cells with high PD-L1 expression.
A: Expression of GPC3, PD-L1 and different NK targets of hepatocellular carcinoma (HCC) PLC 0.5×10⁶ PLC cells from trypsin digestion were respectively treated with antibodies GPC3-APC, PD-L1-APC, MICA-APC, MICB-APC, ULBP1-APC, ULBP2-APC, ULBP3-APC for staining, while the IgG isotype (purchased from R&D Systems) was used as the control. Then, the cell phenotypes were determined by a flow cytometer (BD FACSCanto II). The results show that PLC cells highly expressed PD-L1 and ULBP1, and weakly expressed GPC3, MICA, MICB and ULBP2.
B: CNK-UT cells show more effective killing against HepG2 cells and activation ability than conventional CAR-T cells

In co-culture experiment of T cells and tumor cells, the untransfected T cells, conventional GPC3 CAR-T cells, CNK-UT cells and GPC3 CAR/CNK-UT cells were respectively added at effect-to-target ratio of 1:5 into PLC cells, and co-cultured for 24 hours. Then, all cells were digested and detected by flow cytometry. Tumor cells and T cells were distinguished based on CD45, and then the expression of activation markers CD25 and CD137 on surface of CD8/CD4 cells was also analyzed. Experimental data showed that 35.9% of PLC cells highly expressed GPC3, 97.8% of cells had upregulated expression of PD-L1, 56.9% of cells expressed MICA, 40.9% of cells upregulated MICB, 68.7% of cells upregulated ULBP1, and 25.7% of cells had upregulated expression of ULBP2. After co-cultured at E:T=1:5 for 24 hours, 30.3% of the cells in the control T cell co-culture system were tumor cells, neither untransfected CD4+ nor CD8+ T cells upregulated CD137; in the GPC3 CAR-T co-culture system, up to 47.0% of cells were tumor cells, only 6.89% CD8+ T cells upregulated CD137 expression; CD4+ T cells barely upregulated CD137 expression; in the CNK-UT co-culture system, tumor cells only accounted for 12.2%, and 72.0% CD8+ T cells upregulated CD137 expression; 42.2% CD4+T cells upregulated CD137 expression; in the GPC3 CAR/CNK-UT co-culture system, tumor cells only accounted for 8.94%, and 73.9% CD8+T cells upregulated CD137 expression; 36.2% CD4+T cells upregulated the expression of CD137. Expermental results show that since PLC highly expresses PD-L1 and weakly expresses GPC3, the conventional GPC3 CAR-T had very poor performance in killing PLC, but CNK-UT cells can effectively kill PLC cells, achieve specific activation and upregulation ofCD137; GPC3 CAR/CNK-UT cells that integrate GPC3 CAR and CNK-UT modules are even more effective in tumor killing and activation.

### Example 8: Functional detection of CNK-UT cells in tumor models

In this example, immunodeficiency mice (NSG mice, purchased from Jiangsu Jicui Yaokang Biotechnology Co., Ltd., Cat# T001475) were used as the tumor-bearing model to perform in vivo pharmacodynamic studies.

### 8.1 Therapeutic effect of CNK-UT cells on the animal model of liver cancer cell line HepG2 Experimental animals were divided into three groups as follow:

Negative control group: Administer control T cells, T cells obtained from the same donor, untransfected T cells proliferated after the stimulation of magnetic beads;
Isotype control group: Administer conventional GPC3 CAR-T cells. The GPC3 CAR-T cells were prepared by reference with Examples 3 to 5, the anti-GPC3 41BB-CD3ζ nucleotide sequence was synthesized by full gene synthesis, and inserted into the lentiviral vector CDH-CMV-MCS-EF1α-Puro by molecular cloning method. The plasmid and lentivirus were prepared and used for transfecting T cells to obtain the conventional GPC3 CAR-T cells.

CNK-UT group: Administer GPC3 CAR/CNK-UT cells.

Preparation of GPC3 CAR/CNK-UT cells: by reference to Examples 3 to 5, the nucleotide sequence of anti-GPC3 41BB-CD3ζ-T2A-DAP10-CD3ζ-T2A-NKG2D-p2A-anti-TCR scFv-AdE3 was synthesized by whole gene synthesis, and linked into the lentiviral vector pCDH-CMV-MCS-EF1α-Puro through molecular cloning to preparehe plasmid and lentivirus for transfecting T cells. After expansion of the transfected T cells,GPC3 CAR/CNK-UT cells were obtained.

The experimental animals were divided into groups, and respectively administered the control T cells, conventional GPC3 CAR-T cells and GPC3 CAR/CNK-UT cells. The animals were weighed. 3×10⁶ HepG2 cells were inoculated into 15 NSG mice in right armpit, the inoculation day was recorded as day 0. After 7 days, successful modeling was observed in all mice. The mice were divided into 3 groups, and respectively received the administration of the primary T cells from normal donor (control T cells), conventional GPC3-CAR-T cells, orGPC3 CAR/CNK-UT cells. The clearance of in vivo tumor fluorescence was observed. The administration of cells were all in form of tail vein injection at the dosage of 2 × 10⁶ per mouse for three times (i.e., on Day 7, 9 and 11, respectively). The first administration date was recorded as Day 7, and the fluorescence was observed every 7 days after Day 7.

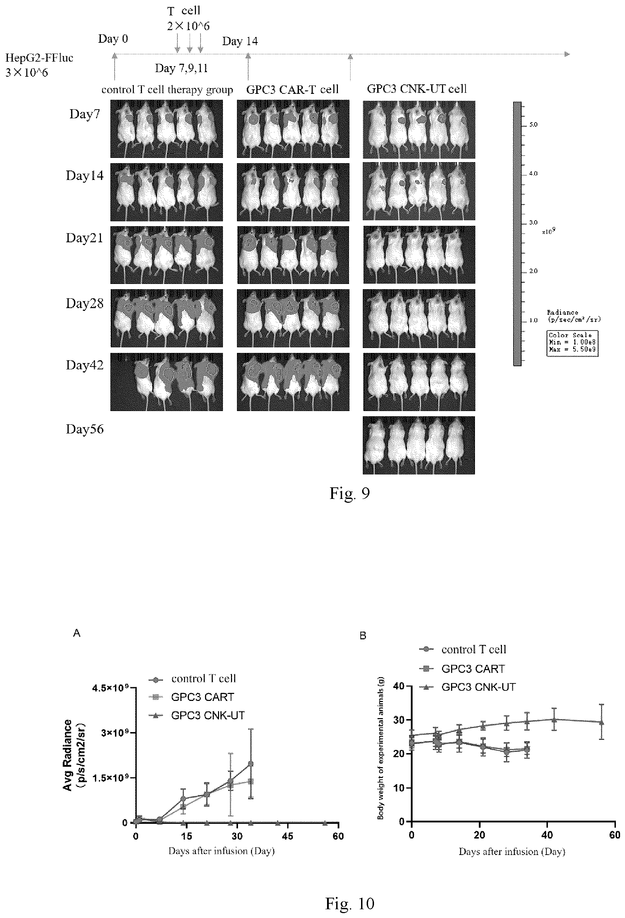

Fig. 9 shows that GPC3 CAR/CNK-UT cells have higher tumor clearance ability in mice than GPC3 CAR-T cells.

After establishing the model by hepatocarcinoma cell line HepG2-Ffluc, the untransfected T cells (left), GPC3 CAR-T (middle) or GPC3 CAR/CNK-UT (right) were administered at 2 × 10⁶, and the tumor growth was monitored weekly by the IVIS animal fluorescence imaging system. The experimental results showed that the fluorescence in the control T cell group continuely increased, the fluorescence in GPC3 CAR-T cell group increased slowly, while the tumor fluorescence in CNK-T001 group kept decreasing until disappeared. The results confirmed that GPC3 CAR/CNK-UT cells have more significant antitumor effect than conventional CART cells in the mouse model.

Fig. 10 shows that GPC3 CAR/CNK-UT cells have higher tumor clearance ability than GPC3 CAR-T cells.

In view of the tumor fluorescence curve plotted on the basis of quantification of the fluorescence image of Fig. 10, it can be seen that neither control T cells (blue) nor GPC3 CAR-T (red) are able to completely inhibit tumor growth, however (green) can significantly inhibit tumor growth. The tumor fluorescence gradually decreased until invisible, confirming the anti-tumor effect of GPC3 CAR/CNK-UT cells in mouse models.

The results of the above-mentioned experiments were as follows. In the negative control mice, the tumor size gradually increased, the first death case appeared between Day 28 and Day 42, and none could survive after Day 42. In the isotype control group with administration of GPC3 CAR/CAR-T cells, tumor growth was observed to be inhibited on Day 14, but no tumor was completely eliminated; an increase of tumor size was observed on Day 21; and almost no difference compared to the negative control group could be observed after Day 28; all mice were died between Day 42 and Day 56; indicating that single-target GPC3 CAR-T could only partially inhibit the tumor, but could not completely clear it, let alone prevent recurrence. In the CNK-UT group with administration of GPC3 CAR/CNK-UT cells, the in vivo tumor fluorescent signal was significantly decreased on Day 14, and completely disappeared on Day 21; all mice survived until the cut-off day (Day 56) with no tumor recurrence. The results confirmed that GPC3CAR/CNK-UT cells have good anti-tumor activity in vivo, which is significantly superior to that of GPC3 CAR-T, and such anti-tumor effect can be maintained for at least 56 days even after the tumors were removed.

In addition, the body weight changes of mice after infusion of different cells were also studied. Compared with the body weight on Day 0, the experimental animals in the CNK-UT group showed a slight increase in body weight after the infusion of CNK-UT cells, while animals in both the isotype control group (infused with GPC3-CAR-T) and the negative control group (infused with control T cells) showed a gradual decrease in body weight as the experiment progressed.

Based on the above-mentioned experimental results, it is suggested that after different treatments, tumors in mice injected the control T cells or GPC3 CAR-T cells kept growing, while injection of GPC3 CAR/CNK-UT cells could eliminate tumor cells in 21 days without reoccurrence, since GPC3 CAR/CNK-UT cells have superior killing activity compared to GPC3 CAR-T cells. In addition, infusion of GPC3 CAR/CNK-UT causes no cytotoxicity, the animals have prolonged survival period, improved living quality and increased body weight.

### 8.2 Target-killing and activation ability of CNK-UT cells in case of triple-negative breast cancer cell MDA-MB453

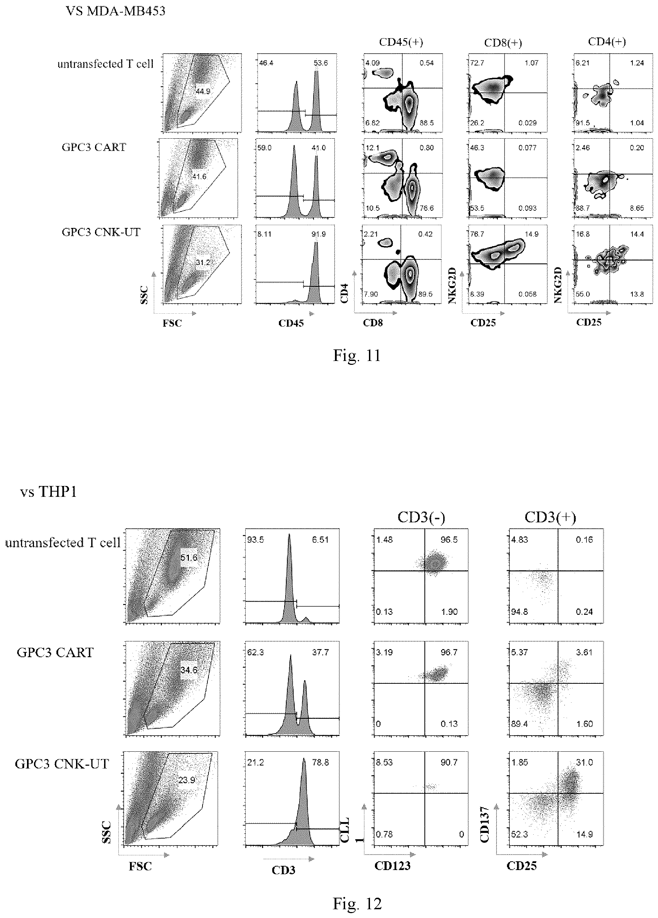

Fig. 11 illustrates that GPC3 CAR/CNK-UT can specifically recognize and kill the triple negative breast cancer cell MDA-MB453.

In the co-culture experiment of T cells and tumor cells, the untransfected T cells, conventional GPC3 CAR-T cells, CNK-UT cells or GPC3 CAR/CNK-UT cells were added at effect-to-target ratio of 1:5 to MDA-MB453 cells respectively. After co-culture for 24 hours, all cells were digested and detected by flow cytometry technology. Tumor cells and T cells were distinguished by the presence of CD45, then the expression of NK modules NKG2D as well as surface activation markers CD25, was analyzed for CD8 and CD4 cells.

MDA-MB453 is a triple-negative breast cancer cell line. The cells do not express GPC3, such that GPC3 CAR-T cannot eliminate MDA-MB453 (after co-culture for 24 hours, a large number of CD45-negative tumor cells still exist in the system), while CNK-UT products can effectively kill and eliminate tumor cells. Among CD45(+) T cells, CD8(+) and CD4(+) T cells both highly express the CNK element NKG2D and upregulate the T cell activation marker CD25.

### 8.3 Target-killing and activation ability of CNK-UT cells in case of acute myeloid leukemia cell line THP1

Fig. 12 illustrates that GPC3 CAR/CNK-UT can specifically recognize and kill acute myeloid leukemia cell line THP1.

In the co-culture experiment of T cells and tumor cells, the untransfected T cells, GPC3 CAR-T cells, CNK-UT cells and GPC3 CAR/CNK-UT cells were added at the effect-to-target ratio of 1:5 to THP1 cells respectively. After 24 hours, all cells were digested and detected by flow cytometry technology.

Tumor cells and T cells were distinguished by the presence of CD3. Then, it is analyzed for the expression of surface markers CD123 and CLL1 on CD3(-) tumor cells, and the expression of surface activation markers CD25 and CD137 of CD3(+) cells.

The results show that THP1 is a leukemia cell line without expressing GPC3, so GPC3 CAR-T cannot eliminate THP1. After 24 hours of co-culture, CD3(-), CD123(+), CLL1(+) tumor cells still exist in a large amount in the system. However, the GPC3 CAR/CNK-UT product can effectively kill and eliminate tumor cells. Detection of up-regulated T cell activation markers CD25 and CD137 in CD3(+) T cells indicates that GPC3 CAR/CNK-UT can specifically recognize THP1 cells, be activated, kill and eliminate tumor cells.

Example 9: The killing and eliminating ability of CNK-UT cells with upgraded NCR elements to different tumors

This example studies the killing and eliminating abilities of CNK-UT cells to different tumors which were prepared based on Example 5 and had upgraded NCR elements. The renal cell carcinoma (RCC) tumor cell line 786-O was purchased from Pronoce (CL-0010); the renal cell carcinoma (RCC) tumor cell line ACHN was purchased from Pronoce (CL-0021); epithelial adenoma (Lung epithelial carcinoma) tumor cell line A549 was purchased from Pronoce (CL-0016); acute myeloid leukemia tumor cell line UL60 was purchased from Pronoce (CL-0110) .

### 9.1 CNK-UT cells recognize and specifically kill RCC tumor cell line 786-0

Preparation of NCR2 CAR/CNK-UT cells: by reference to Examples 3 to 5, the nucleotide sequence of NCR2-CD28-CD3ζ (SEQ ID NO: 124, the amino acid sequence encoded thereby is shown in SEQ ID NO: 123) was synthesized by whole gene synthesis; the nucleotide sequence ofDAP10-CD3ζ-T2A-NKG2D-p2A-anti-TCR scFv-AdE3 was synthesized by whole gene synthesis. The sequences were linked into the lentiviral vector pCDH-CMV-MCS-EF1α-Puro through molecular cloning, to prepare the plasmid and lentivirus for transfecting T cells. After expanding the transfected T cells, the NCR2 CAR/CNK-UT cells were obtained.

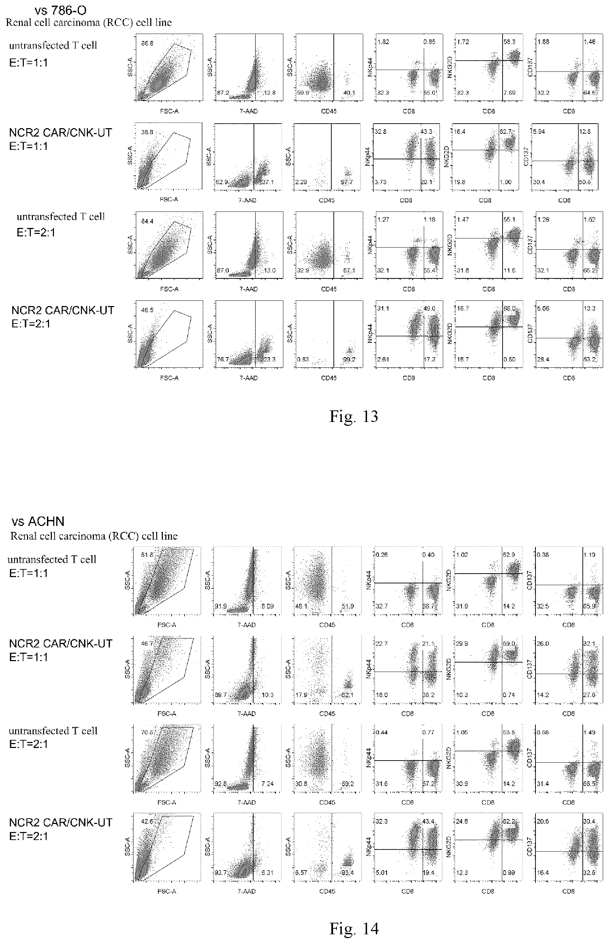

Fig. 13 illustrates the recognition, specific killing and activation ability of CNK-UT cells in case ofrenal cell carcinoma 786-O cells.

In the co-culture experiment of T cells and tumor cells, the RCC cancer cell line 786-O was counted and spread at 0.5 ×10⁶ cells/well in a 24-well plate. The untransfected T cells or the NCR2 CAR/CNK-UT cells (NCR2-CD28-CD3ζ; DAP10-CD3ζ-T2A-NKG2D-p2A-ant-TCR scFv-AdE3) were added respectively at the effector-to-target ratio of 1:1 and 2:1 into the tumor cell. After 24 hours, all cells were centrifuged, collected, and treated with antibodies 7-AAD, CD45-APC-Cy5.5, CD8-APC, NKG2D-PE-Cy7, NKp44-PE, and CD137-Pacific Blue (purchased from Biolegend) for staining. The stained cells were detected by flow cytometry. Experimental data showed that NCR2 CAR/CNK-UT cells can effectively kill 786-O cells. In the culture system with E: T=1: 1, after 48 hours of co-culture, tumor cells in the control group accounted for 59.9%, and the control T cells neither expressed Nkp44 nor upregulated the activation marker CD137. However, in the NCR2CAR/CNK-UT cell co-culture system, tumor cells only accounted for 2.29%, and 12.8% of CD8+ T cells had upregulated expression of CD137. In the culture system with E: T=2:1, after 48 hours of co-culture, tumor cells in the control group accounted for 32.9%, and the control T cells neither expressed Nkp44 nor upregulated the activation marker CD137. In contrast, in the NCR2CAR/CNK-UT cell co-culture system, tumor cells only accounted for 0.83%, and 13.3% of CD8+ T cells has upregulated expression of CD137. It can be seen that upgradation of NCR modules can further increase the killing and elimination ability of CNK-UT cells against different tumors.

### 9.2 CNK-UT cells recognize and specifically kill renal cell carcinoma (RCC) tumor cell line ACHN

Fig. 14 illustrates the recognition, specific killing and activation ability of CNK-UT cells in case of renal cell carcinoma ACHN cells.

In the co-culture experiment of T cells and tumor cells, the RCC cell line ACHN was counted and spread at 0.5 ×10⁶ cells/well in a 24-well plate. The untransfected T cells or the NCR2 CAR/CNK-UT cells (NCR2-CD28-CD3ζ; DAP10-CD3ζ-T2A-NKG2D-p2A-ant-TCR scFv-AdE3) were added respectively at the effector-to-target ratio of 1:1 and 2:1 into the tumor cell. After 24 hours, all cells were centrifuged, collected, and treated with antibodies 7-AAD, CD45-APC-Cy5.5, CD8-APC, NKG2D-Cy7, NKp44-PE, and CD137-Pacific Blue (purchased from Biolegend) for staining. The stained cells were detected by flow cytometry. Experimental data showed that NCR2 CAR/CNK-UT cells can effectively kill ACHN tumor cells. In the culture system with E: T=1: 1, after 48 hours of co-culture, tumor cells in the control group accounted for 48.1%, and the control T cells neither expressed Nkp44 nor upregulated the activation marker CD137. However, in the NCR2 CAR/CNK-UT cell co-culture system, tumor cells only accounted for 17.9%, and 32.1% of CD8+ T cells had upregulated expression of CD137. In the culture system with E:T=2: 1, after 48 hours of co-culture, tumor cells in the control group accounted for 30.8%, and the control T cells neither expressed Nkp44 nor upregulated the activation marker CD137. However, in the NCR2 CAR/CNK-UT cell co-culture system, tumor cells only accounted for 6.57%, and 30.4% of CD8+ T cells had upregulated expression ofCD137.

### 9.3 CNK-UT cells recognize and specifically kill Lung epithelial carcinoma cell line A549

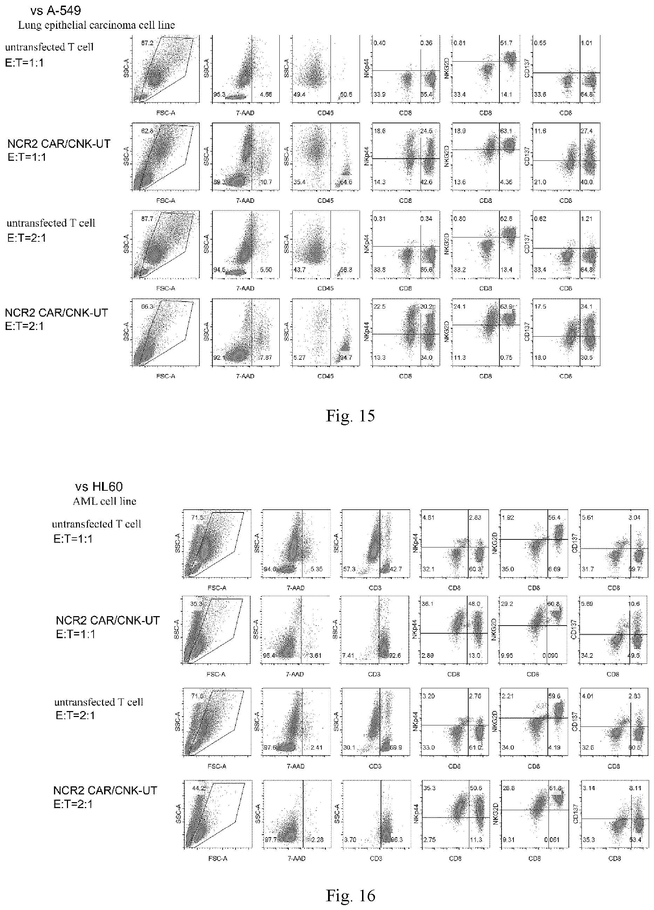

Fig. 15 illustrates the recognition, specific killing and activation ability of CNK-UT cells in case of pulmonary epithelial adenoma A549 cells.

In the co-culture experiment of T cells and tumor cells, the lung cancer cell line A549 was counted and spread at 0.5×10⁶ cells/well in a 24-well plate. The untransfected T cells or the NCR2 CAR/CNK-UT cells (NCR2-CD28-CD3ζ; DAP10-CD3ζ-T2A-NKG2D-p2A-ant-TCR scFv-AdE3) were added respectively at the effector-to-target ratio of 1:1 and 2:1 into the tumor cell. After 24 hours, all cells were centrifuged, collected, and treated with antibodies 7-AAD, CD45-APC-Cy5.5, CD8-APC, NKG2D-PE-Cy7, NKp44-PE, and CD137-Pacific Blue (purchased from Biolegend) for staining. The stained cells were detected by flow cytometry. Experimental data showed that NCR2 CAR/CNK-UT cells can effectively kill A549 tumor cells. In the culture system with E: T=1:1, after 48 hours of co-culture, tumor cells in the control group accounted for 49.4%, and the control T cells neither expressed Nkp44 nor upregulated the activation marker CD137. However, in the NCR2 CAR/CNK-UT cell co-culture system, tumor cells only accounted for 35.4%, and 27.4% of CD8+ T cells had upregulated expression of CD137. In the culture system with E:T=2: 1, after 48 hours of co-culture, tumor cells in the control group accounted for 43.7%, and the control T cells neither expressed Nkp44nor upregulated the activation marker CD137. In the NCR2 CAR/CNK-UT cell co-culture system, tumor cells only accounted for 5.27%, and 34.1% of CD8+ T cells had upregulated expression of CD137.

### 9.4 CNK-UT cells recognize and specifically kill Acute myeloid leukemia cell line UL60

Fig. 16 illustrates the recognition, specific killing and activation ability of CNK-UT cells in case of AML cell line UL60.

In the co-culture experiment of T cells and tumor cells, the lung cancer cell line UL60 was counted and spread at 0.5×10⁶ cells/well in a 24-well plate. The untransfected T cells or the NCR2 CAR/CNK-UT cells (NCR2-CD28-CD3ζ; DAP10-CD3ζ-T2A-NKG2D-p2A-ant-TCR scFv-AdE3) were added respectively at the effector-to-target ratio of 1:1 and 2:1 into the tumor cell. After 24 hours, all cells were centrifuged, collected, and treated with antibodies 7-AAD, CD45-APC-Cy5.5, CD8-APC, NKG2D-PE-Cy7, NKp44-PE, and CD137-Pacific Blue (purchased from Biolegend) for staining. The stained cells were detected by flow cytometry. Experimental data showed that the NCR2 CAR/CNK-UT cells can effectively kill UL60 tumor cells. In the culture system with E: T=1: 1, after 48 hours of co-culture, CD3(-) tumor cells accounted for 57.3% in the control group, and the control T cells neither expressed Nkp44 nor upregulated the activation marker CD137. However, in the NCR2 CAR/CNK-UT cell co-culture system, tumor cells only accounted for 7.41%, and 10.6% of CD8+ T cells had upregulated expression of CD137. In the culture system with E: T=2:1, after 48 hours of co-culture, tumor cells accounted for 30.1% in the control group, and the control T cells neither expressed Nkp44 nor upregulated the activation marker CD137. However, in the NCR2 CAR/CNK-UT cell co-culture system, tumor cells only accounted for 3.7%, and 8.11% of CD8+ T cells had upregulated expression of CD137.

### Example 10: Optimizing the design of UT elements to improve CNK-UT cells in terms of the recognition and specific killing against tumor cell line U937

Preparation of CNK-UT(UL16) and CNK-UT(UL16/E3-19K) cells: by reference to Examples 3 to 5, the nucleotide sequence of DAP10-CD3ζ-T2A-NKG2D (the amino acid sequence encoded thereby is shown in SEQ ID NO: 125) was synthesized by whole gene synthesis; the nucleotide sequence of anti-TCR scFv-AdE3-p2A-UL16 (the amino acid sequence encoded thereby is shown in SEQ ID NO: 126) was synthesized by whole gene synthesis; the nucleotide sequence of anti-TCR scFv-AdE3-p2A-UL16-p2A-E3-19K (the amino acid sequence encoded thereby is shown in SEQ ID NO: 127) was synthesized by whole gene synthesis. The seuqences were linked into the lentiviral vector pCDH-CMV-MCS-EF1α-Puro through molecular cloning to prepare the plasmid and lentivirus for transfecting T cells. After expanding the transfected T cellsexpanded, CNK-UT(UL16) and CNK-UT(UL16/E3-19K) cells were obtained.

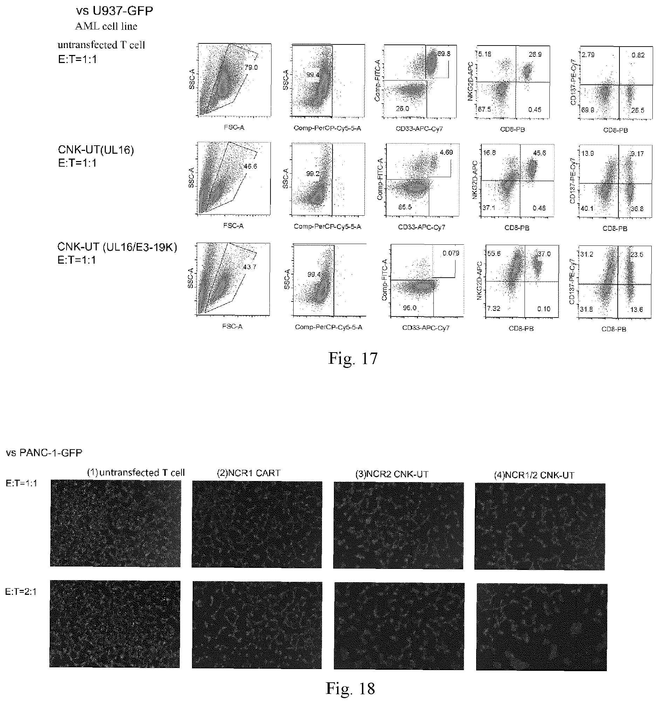

Fig. 17 illustrates the recognition, specific killing and activation ability of CNK-UT cells in case of AML cell line U937 (purchased from U937, item number CL-0239).

In the co-culture experiment of T cells and tumor cells, the lung cancer cell line U937-GFP were counted and spread at 0.5×10⁶ cells/well in a 24-well plate. The untransfected T cells, the CNK-UT(UL16) cells (DAP10-CD3ζ-T2A-NKG2D; anti-TCR scFv-AdE3-p2A-UL16), or the CNK-UT(UL16/E3-19K) cells (DAP10-CD3ζ-T2A-NKG2D; anti-TCR scFv-AdE3-p2A-UL16-p2A-E3-19K) were added respectively at the effector-to-target ratio of 1:1 into the tumor cell. After 24 hours, all cells were centrifuged, collected, and treated with antibodies 7-AAD, CD33-APC-Cy5.5, CD8-Pacific Blue, NKG2D-APC, CD137-PE-Cy7 (purchased from Biolegend) for staining. The stained cells were detected by flow cytometry. Experimental data showed that the CNK-UT (UL16/E3-19K) design had higher efficiency in killing U937 tumor cells than CNK-UT (UL16) cells. In the culture system with E: T=1:1, after 48 hours of co-culture, CD33(+) tumor cells accounted for 69.8% in the control group, and the control T cells did not upregulate the activation marker CD137. In the co-culture system of CNK-UT (UL16) cells, CD33+ GFP+ tumor cells only accounted for 4.69%, while 23.07% of T cells had upregulated expression of CD137. In the co-culture system of CNK-UT (UL16/E3-19K) cells, tumor cells only accounted for 0.079%, and 54.7% of T cells had upregulated expression of CD137.

### Example 11: Optimizing the design of NK elements to improve CNK-UT cells against tumor cell lines

### 11.1 Optimizing the design of NK elements to improve CNK-UT cells in terms of the recognition and specific killing against tumor cell line U937

Preparation of NCR1 CAR-T, NCR2 CNK-UT and NCR1/2 CNK-UT cells: by reference to Examples 3 to 5, the nucleotide sequence of NCR1-CD28-CD3ζ (the amino acid sequence encoded thereby is shown in SEQ ID NO: 128) was synthesized by whole gene synthesis; the nucleotide sequence of DAP10-CD3ζ-T2A-NKG2D-p2A-anti-TCR scFv-AdE3 (the amino acid sequence encoded thereby is shown in SEQ ID NO: 117) was synthesized by whole gene synthesis; the nucleotide sequence of NCR2-CD28-CD3ζ(the amino acid sequence encoded thereby is shown in SEQ ID NO: 123) was synthesized by whole gene synthesis; the nucleotide sequence of NCR2-CD28-CD3ζ-p2A-NCR1-CD28-CD3ζ(the amino acid sequence encoded thereby is shown in SEQ ID NO: 129) was synthesized by whole gene synthesis. The sequences were linked into the lentiviral vector pCDH-CMV-MCS-EF1α-Puro through molecular cloning to prepare the plasmid and lentivirus for transfecting T cells. Afterexpansion of the transfected T cells, the NCR1 CAR-T, NCR2 CNK-UT and NCR1/2 CNK-UT cells were obtained.

Fig. 18 illustrates the recognition, specific killing and activation ability of CNK-UT cells in case of AML cell line U937.

In the co-culture experiment of T cells and tumor cells, the lung cancer cell line U937-GFP were counted and spread at 0.5 ×10⁶ cells/well in a 24-well plate. The untransfected T cells, NCR1 CAR-T cells (NCR1-CD28-CD3ζ), NCR2 CNK-UT cells (DAP10-CD3ζ-T2A-NKG2D-p2A-anti-TCR scFv-AdE3; NCR2-CD28-CD3ζ) and NCR1/2 CNK-UT cells (DAP10-CD3ζ-T2A-NKG2D-p2A-anti-TCR scFv-AdE3; NCR2-CD28-CD3ζ-p2A-NCR1-CD28-CD3ζ) were added respectively at the effect-to-target ratio of 1: 1 and 2: 1. After 48 hours, the cells were observed and photographed under a fluorescence microscope. Experimental data showed that the addition of NK recognition elements can significantly improve the killing effect of CNK-UT cells against U937 tumor cells.

### 11.2 Optimizing the design of NK elements to improve CNK-UT cells in terms of the recognition and specific killing against tumor cell line U937

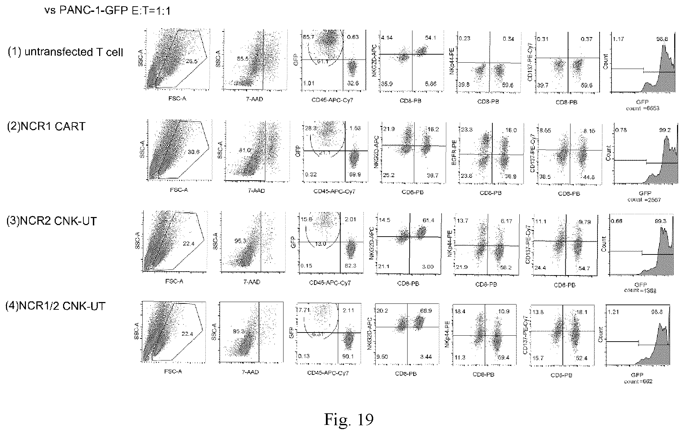

Fig. 19 illustrates the recognition, specific killing and activation ability of CNK-UT cells in case of pancreatic cancer cell line PANC-1 (purchased from Pnoxil, item number CL-0184).

Preparation of NCR1 CAR-T, NCR2 CNK-UT and NCR1/2 CNK-UT cells: by reference to Examples 3 to 5, the nucleootide sequence ofNCR1-CD28-CD3ζ-p2A-tEGFR (the amino acid sequence encoded therby is shown in SEQ ID NO: 130) was synthesized by whole gene synthesis; the nucleootide sequence of DAP10-CD3ζ-T2A-NKG2D-p2A-anti-TCR scFv-AdE3 (the amino acid sequence encoded therby is shown in SEQ ID NO: 117) was synthesized by whole gene synthesis; the nucleootide sequence of NCR2-CD28-CD3ζ-p2A-tEGFR (the amino acid sequence encoded therby is shown in SEQ ID NO: 131) was synthesized by whole gene synthesis; the nucleootide sequence of NCR2-CD28-CD3ζ-p2A-NCR1-CD28-CD3ζ(the amino acid sequence encoded therby is shown in SEQ ID NO: 129) was synthesized by whole gene synthesis. The sequences were linked into the lentiviral vector pCDH-CMV-MCS-EF1α-Puro through molecular cloningto prepare the plasmid and lentivirus for transfecting T cells. After the expansion of the transfected T cells, the NCR1 CAR-T, NCR2 CNK-UT and NCR1/2 CNK-UT cells were obtained.

In the co-culture experiment of T cells and tumor cells, the pancreatic cancer cell line PANC-1-GFP was counted and spread at 0.5×10⁶ cells/well in a 24-well plate. The untransfected T cells, the NCR1 CAR-T cells (NCR1-CD28-CD3ζ-p2A-tEGFR), NCR2 CNK-UT cells (DAP10-CD3ζ-T2A-NKG2D-p2A-anti-TCR scFv-AdE3; NCR2-CD28-CD3ζ-p2A-tEGFR), or the NCR1/2 CNK-UT (DAP10-CD3ζ-T2A-NKG2D-p2A-anti-TCR scFv-AdE3; NCR2-CD28-CD3ζ-p2A-NCR1-CD28-CD3ζ) were added respectively at the effector-to-target ratio of 1:1 into the tumor cell. After 48 hours, all cells were centrifuged, collected, and treated with antibodies 7-AAD, CD33-APC-Cy5.5, CD8-Pacific Blue, NKG2D-APC, NKp44-PE or EGFR-PE, CD137-PE-Cy7 (purchased from Biolegend) for staining. The stained cells were detected by flow cytometry. Experimental data showed that compared with NCR2 CAR/CNK-UT, the CNK-UT designed by combining NCR1 and NCR2 CAR had more ability of effective killing and activation regarding pancreatic cancer PANC-1 tumor cells. In the culture system with E: T=1:1, after 48 hours of co-culture, GFP(+) CD45(-) tumor cells accounted for 65.7% in the control group, with the flow cytometry count at 6653. The control T cells did not upregulate the activation marker CD137. In the co-culture system of NCR1 CAR-T cells, GFP (+) CD45(-) tumor cells accounted for only 28.3%, the flow cytometry count was 2567, and 16.65% of T cells had upregulated expression of CD137. In the co-culture system of NCR2 CNK-UT cells, tumor cells only accounted for 15.6%, the flow cytometry count was 1368, while 20.89% of T cells had upregulated expression of CD137. In the co-culture system of NCR1/2 CNK-UT cells, tumor cells only accounted for 7.71%, the flow cytometry count was 662, and 21.9% of T cells had upregulated expression of CD137.

## Claims

1. A multi-functional complex comprising the following modules:
(1) a NK activating receptor module, comprising at least a NK-cell-activating receptor or a functional variant thereof, wherein the NK-cell-activating receptor comprises: (a) an extracellular domain (ED) of the NK-cell-activating receptor or a functional variant thereof, (b) a transmembrane domain (TMD) of the NK-cell-activating receptor or a functional variant thereof, and (c) an intracellular domain (ICD) of the NK-cell-activating receptor or a functional variant thereof; and wherein, optionally, a hinge or linker is included among the extracellular domain of the NK-cell-activating receptor or a functional variant thereof, the transmembrane domain of NK-cell-activating receptor or a functional variant thereof, and/or the intracellular domain of the NK-cell-activating receptor or a functional variant thereof;
(2) a CNK signal transduction module, comprising at least (i) a NK cell signal adaptor or a functional variant thereof, wherein the NK cell signal adaptor comprises: (a) an extracellular domain (ED) of the NK cell signal adaptor or a functional variant thereof, (b) a transmembrane domain (TMD) of the NK cell signal adaptor or a functional variant thereof, and (c) an intracellular domain (ICD) of the NK cell signal adaptor or a functional variant thereof; and wherein, optionally, a hinge or linker is included among the extracellular domain of the NK cell signal adaptor or a functional variant thereof, the transmembrane domain of NK cell signal adoptor or a functional variant thereof, and/or the intracellular domain of the NK cell signal adaptor or a functional variant thereof; and
(3) a UT module, comprising at least (i) a recombinant protein molecule for targeted degradation of TCR, MHC, and/or a NK cell target target, or a functional variant thereof, wherein the recombinant protein molecule for targeted degradation of TCR, MHC, and/or a NK cell target comprises: (a) a binding protein molecular domain targeting TCR, MHC, and/or a NK cell target, or a functional variant thereof, (b) a transmembrane domain of a viral endoplasmic reticulum (ER) resident glycoprotein, or a functional variant, and (c) a cytoplasmic domain of a viral endoplasmic reticulum resident glycoprotein, or a functional variant thereof; the transmembrane domain of the viral endoplasmic reticulum resident glycoprotein or a functional variant thereof and the cytoplasmic domain of the viral endoplasmic reticulum resident glycoprotein or a functional variant thereof form an ERAD degradation domain; optionally, the molecular domain of the binding protein targeting TCR or a functional variant thereof, the transmembrane domain of the viral endoplasmic reticulum resident glycoprotein or a functional variant thereof and/or the cytoplasmic domain of the viral endoplasmic reticulum resident glycoprotein or a functional variant thereof comprise a hinge or linker; optionally, a hinge or linker is included among the NK activating receptor module, the CNK signal transduction module, and/or the UT module.

2. The multi-functional complex according to claim 1, wherein the NK-cell-activating receptor in the NK activating receptor module is selected from the group consisting of NKG2D, NKG2C, NKG2E, NKG2F, NKG2H, CD94, KIR2DL4, KIR2DS1, KIR2DS2, KIR2DS4, KIR3DS1, natural cytotoxic receptors, TRAIL, DNAM-1, CD16a, 2B4, NTB-A, CRACC and NKp80; preferably, the natural cytotoxic receptors are selected from NKp46, NKp44 and NKp30;
preferably, the NK-cell-activating receptor is a mammalian derived NK-cell-activating receptor; preferably, the mammal is selected from the group consisting of human, primate, rat, horse, cattle, sheep, goat, cat, pig, dog, llama, alpacas, elephant, squirrel, guinea pig;
preferably, the NK-cell-activating receptor is a recombinant NK-cell-activating receptor comprising different original NK-cell-activating receptor domains;
preferably, the NK-cell-activating receptor is a human derived NK-cell-activating receptor; and
preferably, the NK-cell-activating receptor is a recombinant NK-cell-activating receptor comprising different original and human derived NK-cell-activating receptor domains;
preferably, the NK-cell-activating receptor is a mouse derived NK-cell-activating receptor; and
preferably, the NK-cell-activating receptor is a recombinant NK-cell-activating receptor comprising different original and mouse derived NK-cell-activating receptor domains;
preferably, the NK-cell-activating receptor is a recombinant NK-cell-activating receptor comprising human derived NK-cell-activating receptor domain and mouse derived NK-cell-activating receptor domain;
preferably, the extracellular domain of the NK-cell-activating receptor is the extracellular domain of the human or murine NK-cell-activating receptor;
preferably, the transmembrane domain of the NK-cell-activating receptor is the transmembrane domain of the human or murine NK-cell-activating receptor;
preferably, the intracellular domain of the NK-cell-activating receptor is the intracellular domain of the human or murine NK-cell-activating receptor;
preferably, the functional variant of the NK-cell-activating receptor is selected from the NK-cell-activating receptor mutant, wild-type fusion protein, or wild-type and mutant fusion protein;
preferably, the extracellular domain of human NKG2D includes an amino acid sequence having 80% or more identity to the amino acid sequence shown in SEQ ID NO: 1, preferably an amino acid sequence having an identify of 85%, 90%, 95%, 96%, 97%, 98%, or 99% or more, and more preferably an amino acid sequence having an identity of 98%, or 99% or more; and the amino acid sequence of the extracellular domain of human NKG2D is shown as in SEQ ID NO: 1;
preferably, the full-length sequence of the human NKG2D includes an amino acid sequence having 80% or more identity to the amino acid sequence shown in SEQ ID NO: 2, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKG2D is shown as in SEQ ID NO: 2;
preferably, the extracellular domain of mouse NKG2D includes an amino acid sequence having 80% or more identity to the amino acid sequence shown in SEQ ID NO: 3, preferably an amino acid sequence having an identify of 85%, 90%, 95%, 96%, 97%, 98%, or 99% or more, and more preferably an amino acid sequence having an identity of 98%, or 99% or more; and the amino acid sequence of the extracellular domain of mouse NKG2D is shown as in SEQ ID NO: 3;
preferably, the full-length sequence of the mouse NKG2D includes an amino acid sequence having 80% or more identity to the amino acid sequence shown in SEQ ID NO: 4, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the mouse NKG2D is shown as in SEQ ID NO: 4;
preferably, the full length sequence of the human-mouse recombinant NKG2D includes an amino acid sequence having 80% or more identity to the amino acid sequence shown in SEQ ID NO: 5, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human-mouse recombinant NKG2D is shown as in SEQ ID NO: 5;
preferably, the full-length sequence of the human NKG2C includes an amino acid sequence having 80% or more identity to the amino acid sequence shown in SEQ ID NO: 6, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKG2C is shown as in SEQ ID NO: 6;
preferably, the full length sequence of the human NKG2E includes an amino acid sequence having 80% or more identity to the amino acid sequence shown in SEQ ID NO: 7, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKG2E is shown as in SEQ ID NO: 7;
preferably, the full length sequence of the human NKG2F includes an amino acid sequence having 80% or more identity to the amino acid sequence shown in SEQ ID NO: 8, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKG2F is shown as in SEQ ID NO: 8;
preferably, the full-length sequence of the human CD94 includes an amino acid sequence having 80% or more identity to the amino acid sequence shown in SEQ ID NO: 9, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human CD94 is shown as in SEQ ID NO: 9;
preferably, the full length sequence of the human KIR2DL4 includes an amino acid sequence having 80% or more identity to the amino acid sequence shown in SEQ ID NO: 10, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human KIR2DL4 is shown as in SEQ ID NO: 10;
preferably, the full length sequence of the human KIR2DS1 includes an amino acid sequence having 80% or more identity to the amino acid sequence shown in SEQ ID NO: 11, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human KIR2DS1 is shown as in SEQ ID NO: 11;
preferably, the full length sequence of the human KIR2DS2 includes an amino acid sequence having 80% or more identity to the amino acid sequence shown in SEQ ID NO: 12, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human KIR2DS2 is shown as in SEQ ID NO: 12;
preferably, the full length sequence of the human KIR2DS4 includes an amino acid sequence having 80% or more identity to the amino acid sequence shown in SEQ ID NO: 13, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human KIR2DS4 is shown as in SEQ ID NO: 13;
preferably, the full length sequence of the human KIR3DS 1 includes an amino acid sequence having 80% or more identity to the amino acid sequence shown in SEQ ID NO: 14, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human KIR3DS 1 is shown as in SEQ ID NO: 14;
preferably, the full-length sequence of the human NKp46 includes an amino acid sequence having 80% or more identity to the amino acid sequence shown in SEQ ID NO: 15, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKp46 is shown as in SEQ ID NO: 15;
preferably, the full-length sequence of the human NKp44 includes an amino acid sequence having 80% or more identity to the amino acid sequence shown in SEQ ID NO: 16, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKp44 is shown as in SEQ ID NO: 16;
preferably, the full-length sequence of the human NKp30 includes an amino acid sequence having 80% or more identity to the amino acid sequence shown in SEQ ID NO: 17, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human NKp30 is shown as in SEQ ID NO: 17;
preferably, the full length sequence of the human DNAM1 includes an amino acid sequence having 80% or more identity to the amino acid sequence shown in SEQ ID NO: 18, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DNAM1 is shown as in SEQ ID NO: 18;
preferably, the full length sequence of the human TRAIL includes an amino acid sequence having 80% or more identity to the amino acid sequence shown in SEQ ID NO: 19, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human TRAIL is shown as in SEQ ID NO: 19;
preferably, the full length sequence of the human CD 16a includes an amino acid sequence having 80% or more identity to the amino acid sequence shown in SEQ ID NO: 20, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CD16a is shown as in SEQ ID NO: 20;
preferably, the full length sequence of the human 2B4 includes an amino acid sequence having 80% or more identity to the amino acid sequence shown in SEQ ID NO: 21, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human 2B4 is shown as in SEQ ID NO: 21;
preferably, the full length sequence of the human NTB-A includes an amino acid sequence having 80% or more identity to the amino acid sequence shown in SEQ ID NO: 22, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NTB-A is shown as in SEQ ID NO: 22;
preferably, the full length sequence of the human CRACC includes an amino acid sequence having 80% or more identity to the amino acid sequence shown in SEQ ID NO: 23, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human CRACC is shown as in SEQ ID NO: 23; and
preferably, the full length sequence of the human NKp80 includes an amino acid sequence having 80% or more identity to the amino acid sequence shown in SEQ ID NO: 24, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human NKp80 is shown as in SEQ ID NO: 24.

3. The multi-functional complex according to claim 1 or 2, wherein the NK cell signal adaptor in the CNK signal transduction module is DAP10 or DAP12;
preferably, the NK cell signal adaptor is a mammalian derived NK cell signal adaptor; preferably, the mammal is selected from the group consisting of human, primate, rat, horse, cattle, sheep, goat, cat, pig, dog, llama, alpacas, elephant, squirrel, guinea pig;
preferably, the NK cell signal adaptor is a recombinant NK cell signal adaptor comprising different original NK cell signal adaptor domains;
preferably, the NK cell signal adaptor is a human derived NK cell signal adaptor; preferably, the NK cell signal adaptor is a recombinant NK cell signal adaptor comprising different human derived NK cell signal adaptor domains;
preferably, the NK cell signal adaptor is a mouse derived NK cell signal adaptor; preferably, the NK cell signal adaptor is a recombinant NK cell signal adaptor comprising different mouse derived NK cell signal adaptor domains;
preferably, the NK cell signal adaptor is a recombinant NK cell signal adaptor comprising human derived NK cell signal adaptor domain and mouse derived NK cell signal adaptor domain;
preferably, the extracellular domain of the NK cell signal adaptor is the extracellular domain of the human or murine NK cell signal adaptor;
preferably, the transmembrane domain of the NK cell signal adaptor is the transmembrane domain of the human or murine NK cell signal adaptor;
preferably, the intracellular domain of the NK cell signal adaptor is the intracellular domain of the human or murine NK cell signal adaptor;
the functional variant of the CNK cell signal adaptor is selected from a mutant of DAP10 or DAP12, or a fusion protein of DAP10 and DAP12, or a wild-type DAP10 or DAP12 fusion protein with a mutant type DAP10 or DAP12;
preferably, the CNK signal transduction module further includes (ii) immunoreceptor activation signal transduction domain (ITAM) and/or (iii) T cell co-stimulatory signal transduction domain;
preferably, a hinge or linker is included among the NK cell signal adaptor or a functional variant thereof, the immunoreceptor activation signal transduction domain (ITAM), and/or the T cell co-stimulatory signal transduction domain; preferably, the NK cell signal adaptor or a functional variant thereof is fused with the immunoreceptor activation signal transduction domain (ITAM);
preferably, the immunoreceptor activation signal transduction domain (ITAM) derives from an intracellular activation signal transduction domain of an immunoreceptor; preferably, the immunoreceptor is selected from TCRζ, CD2, CD3γ, CD3δ, CD3ε, CD3ζ, CD5, CD22, FcRy, CD66d, FcαRI, FcyRI, FcyRII, FcyRIII, Dectin-1, CLEC-1, CD72, CD79A, CD79B; preferably, the immunoreceptor activation signal transdution domain (ITAM) is fused with the NK cell signal adaptor or a functional variant thereof; and preferably, the immunoreceptor is CD3ζ;
preferably, the T cell co-stimulatory signal transduction domain is derived from an intracellular signal transduction domain of an co-stimulatory molecule; preferably, the co-stimulatory molecules are selected from the group consisting of MHC class I molecules, TNF receptor proteins, immunoglobulin-like proteins, cytokine receptors, integrin proteins, lymphocyte activation signal molecules (SLAM proteins), activated NK cell receptors, BTLA, Toll ligand receptors, OX40, CD2, CD7, CD16, CD27, CD28, CD30, CD40, CD38, CD35, CD79A, CD79B, CDS, ICAM-1, LFA-1, (CD11a/CD18), 4-1BB(CD137), B7-H3, CDS, ICAM-1, ICOS(CD278), GITR, BAFFR, LIGHT, HVEM(LIGHTR), KIRDS2, SLAMF7, NKp80(KLRF1), NKp44, NKp30, NKp46, CD19, CD4, CD8α, CD8β, IL2Rβ, IE2Kγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100 SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83-specific binding ligands, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, and PTCH2;
preferably, the full-length sequence of the human DAP10 includes an amino acid sequence having 80% or more identity to the amino acid sequence shown in SEQ ID NO: 25, preferably an amino acid sequence having an identify of 85%, 90%, 95%, 96%, 97%, 98%, or 99% or more, and more preferably an amino acid sequence having an identity of 98%, or 99% or more; and the amino acid sequence of the full-length sequence of the human DAP10 is shown as in SEQ ID NO: 25;
preferably, the full-length sequence of the human DAP10 includes an amino acid sequence having 80% or more identity to the amino acid sequence shown in SEQ ID NO: 26, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP10 is shown as in SEQ ID NO: 26;
preferably, the transmembrane domain of human DAP10 includes an amino acid sequence having 80% or more identity to the amino acid sequence shown in SEQ ID NO: 27, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domain of human DAP10 is shown as in SEQ ID NO: 27;
preferably, the full-length sequence of the human DAP12 includes an amino acid sequence having 80% or more identity to the amino acid sequence shown in SEQ ID NO: 28, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the human DAP12 is shown as in SEQ ID NO: 28;
preferably, the transmembrane domain of human DAP12 includes an amino acid sequence having 80% or more identity to the amino acid sequence shown in SEQ ID NO: 29, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domain of human DAP12 is shown as in SEQ ID NO: 29;
preferably, the fusion protein of the transmembrane domains of human DAP10 and human DAP12 includes an amino acid sequence having 80% or more identity to the amino acid sequence shown in SEQ ID NO: 30, preferably an amino acid sequence having an identity of 85%, 90%, 95%, 96%, 97%, 98%, or 99% or more, and more preferably an amino acid sequence having an identity of 98%, or 99% or more; and the amino acid sequence of the transmembrane domains of human DAP10 and human DAP12 is shown as in SEQ ID NO: 30;
preferably, the sequence of fusion protein of human DAP10-DAP12 includes an amino acid sequence having 80% or more identity to the amino acid sequence shown in SEQ ID NO: 31, preferably an amino acid sequence having an identity of 85%, 90%, 95%, 96%, 97%, 98%, or 99% or more, and more preferably an amino acid sequence having an identity of 98%, or 99% or more; and amino acid sequence of the fusion protein of human DAP10-DAP12 is shown as in SEQ ID NO: 31;
preferably, the sequence of an intracellular signal transduction domain of human CD3zeta includes an amino acid sequence having 80% or more identity to the amino acid sequence shown in SEQ ID NO: 32, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human CD3zeta is shown as in SEQ ID NO: 32;
preferably, the human DAP10-CD3zeta sequence includes an amino acid sequence having 80% or more identity to the amino acid sequence shown in SEQ ID NO: 33, preferably an amino acid sequence having an identity of 85%, 90%, 95%, 96%, 97%, 98%, or 99% or more, and more preferably an amino acid sequence having an identity of 98%, or 99% or more; and amino acid sequence of the human DAP10-CD3zeta sequence is shown as in SEQ ID NO: 33;
preferably, the human DAP12-CD3zeta sequence includes an amino acid sequence having 80% or more identity to the amino acid sequence shown in SEQ ID NO: 34, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-CD3zeta sequence is shown as in SEQ ID NO: 34;
preferably, the human DAP10-DAP12-CD3zeta sequence includes an amino acid sequence having 80% or more identity to the amino acid sequence shown in SEQ ID NO: 35, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP10-DAP12-CD3zeta sequence is shown as in SEQ ID NO: 35;
preferably, the sequence of an intracellular signal transduction domain of human 41BB includes an amino acid sequence having 80% or more identity to the amino acid sequence shown in SEQ ID NO: 36, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human 41BB is shown as in SEQ ID NO: 36;
preferably, the human DAP10-41BB sequence includes an amino acid sequence having 80% or more identity to the amino acid sequence shown in SEQ ID NO: 37, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP10-41BB sequence is shown as in SEQ ID NO: 37;
preferably, the human DAP10-41BB-CD3zeta sequence includes an amino acid sequence having 80% or more identity to the amino acid sequence shown in SEQ ID NO: 38, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP10-41BB-CD3zeta sequence is shown as in SEQ ID NO: 38;
preferably, the sequence of an intracellular signal transduction domain of human CD28 includes an amino acid sequence having 80% or more identity to the amino acid sequence shown in SEQ ID NO: 39, preferably an amino acid sequence having an identity of 85%, 90%, 95%, 96%, 97%, 98%, or 99% or more, and more preferably an amino acid sequence having an identity of 98%, or 99% or more; and the amino acid sequence of the intracellular signal transduction domain of human CD28 is shown as in SEQ ID NO: 39;
preferably, the human DAP10-CD28 sequence includes an amino acid sequence having 80% or more identity to the amino acid sequence shown in SEQ ID NO: 40, preferably an amino acid sequence having an identity of 85%, 90%, 95%, 96%, 97%, 98%, or 99% or more, and more preferably an amino acid sequence having an identity of 98%, or 99% or more; and the amino acid sequence of human DAP10-CD28 is shown as in SEQ ID NO: 40;
preferably, the human DAP10-CD28-CD3zeta sequence includes an amino acid sequence having 80% or more identity to the amino acid sequence shown in SEQ ID NO: 41, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP10-CD28-CD3zeta sequence is shown as in SEQ ID NO: 41;
preferably, the human DAP12-41BB sequence includes an amino acid sequence having 80% or more identity to the amino acid sequence shown in SEQ ID NO: 42, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-41BB sequence is shown as in SEQ ID NO: 42;
preferably, the human DAP12-41BB-CD3zeta sequence includes an amino acid sequence having 80% or more identity to the amino acid sequence shown in SEQ ID NO: 43, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-41BB-CD3zeta sequence is shown as in SEQ ID NO: 43;
preferably, the human DAP12-CD28 sequence includes an amino acid sequence having 80% or more identity to the amino acid sequence shown in SEQ ID NO: 44, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-CD28 sequence is shown as in SEQ ID NO: 44;
preferably, the human DAP12-CD28-CD3zeta sequence includes an amino acid sequence having 80% or more identity to the amino acid sequence shown in SEQ ID NO: 45, preferably an amino acid sequence having an identity of 85%, 90%, 95%, 96%, 97%, 98%, or 99% or more, and more preferably an amino acid sequence having an identity of 98%, or 99% or more; and the amino acid sequence of the human DAP12-CD28-CD3zeta sequence is shown as in SEQ ID NO: 45;

4. The multi-functional complex according to any one of claims 1-3, wherein in the recombinant protein molecule for targeted degradation of TCR of the UT module, the binding protein molecular domain targeting TCR or a functional variant thereof is derived from a TCR antibody or a functional fragment thereof or the combination thereof;
preferably, the antibody is selected from a TCRα antibody, a TCRβ antibody, a TCRαβ antibody, a TCRγ antibody, a TCRδ antibody, a TCRγδ antibody, a TCR Vδ2 antibody, a TCR Cβ1 antibody; and the functional fragment of the antibody is selected from Fd, Fv, Fab, Fab', F(ab')2, Fv (scFv), single-chain antibody (scFv) or nanobody (nanobody), double-stranded antibody, triple-stranded antibody and tetra-stranded antibody; preferably, the TCR antibody is a single-chain TCR antibody; preferably, the amino acid sequence of the single chain TCR antibody includes an amino acid sequence having 80% or more identity, preferably 85%, 90%, 95%, 96%, 97%, 98%, 99% or more identity, and more preferably 98%, 99% or more identity to the amino acid sequence shown in SEQ ID NO: 116; and the amino acid sequence of the full-length sequence of the single chain TCR antibody is shown as in SEQ ID NO: 116;
preferably, the ERAD degradation domain in the UT module is derived from HCMV glycoprotein US2, US3, US11 or US 10, adenovirus E3-19K, or HHV-7 US21;
preferably, the full-length sequence of the HCMV glycoprotein US2 includes an amino acid sequence having 80% or more identity to the amino acid sequence shown in SEQ ID NO: 46, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US2 is shown as in SEQ ID NO: 46;
preferably, the HLA binding domain of the HCMV glycoprotein US2 includes an amino acid sequence having 80% or more identity to the amino acid sequence shown in SEQ ID NO: 47, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US2 is shown as in SEQ ID NO: 47;
preferably, the ERAD degradation domain of the HCMV glycoprotein US2 includes an amino acid sequence having 80% or more identity to the amino acid sequence shown in SEQ ID NO: 48, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US2 is shown as in SEQ ID NO: 48;
preferably, the full-length sequence of the HCMV glycoprotein US3 includes an amino acid sequence having 80% or more identity to the amino acid sequence shown in SEQ ID NO: 49, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US3 is shown as in SEQ ID NO: 49;
preferably, the HLA binding domain of the HCMV glycoprotein US3 includes an amino acid sequence having 80% or more identity to the amino acid sequence shown in SEQ ID NO: 50, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US3 is shown as in SEQ ID NO: 50;
preferably, the ERAD degradation domain of the HCMV glycoprotein US3 includes an amino acid sequence having 80% or more identity to the amino acid sequence shown in SEQ ID NO: 51, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US3 is shown as in SEQ ID NO: 51;
preferably, the full-length sequence of the HCMV glycoprotein US11 includes an amino acid sequence having 80% or more identity to the amino acid sequence shown in SEQ ID NO: 52, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US11 is shown as in SEQ ID NO: 52;
preferably, the MHC binding domain of the HCMV glycoprotein US11 includes an amino acid sequence having 80% or more identity to the amino acid sequence shown in SEQ ID NO: 53, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the HCMV glycoprotein US 11 is shown as in SEQ ID NO: 53;
preferably, the ERAD degradation domain of the HCMV glycoprotein US 11 includes an amino acid sequence having 80% or more identity to the amino acid sequence shown in SEQ ID NO: 54, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US 11 is shown as in SEQ ID NO: 54;
preferably, the full-length sequence of the HCMV glycoprotein US 10 includes an amino acid sequence having 80% or more identity to the amino acid sequence shown in SEQ ID NO: 55, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV glycoprotein US 10 is shown as in SEQ ID NO: 55;
preferably, the HLA binding domain of the HCMV glycoprotein US 10 includes an amino acid sequence having 80% or more identity to the amino acid sequence shown in SEQ ID NO: 56, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the HCMV glycoprotein US 10 is shown as in SEQ ID NO: 56;
preferably, the ERAD degradation domain of the HCMV glycoprotein US 10 includes an amino acid sequence having 80% or more identity to the amino acid sequence shown in SEQ ID NO: 57, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV glycoprotein US 10 is shown as in SEQ ID NO: 57;
preferably, the full-length sequence of the adenovirus E3-19K includes an amino acid sequence having 80% or more identity to the amino acid sequence shown in SEQ ID NO: 58, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the adenovirus E3-19K is shown as in SEQ ID NO: 58;
preferably, the MHC binding domain of the adenovirus E3-19K includes an amino acid sequence having 80% or more identity to the amino acid sequence shown in SEQ ID NO: 59, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the adenovirus E3-19K is shown as in SEQ ID NO: 59;
preferably, the ERAD degradation domain of the adenovirus E3-19K includes an amino acid sequence having 80% or more identity to the amino acid sequence shown in SEQ ID NO: 60, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the adenovirus E3-19K is shown as in SEQ ID NO: 60;
preferably, the full-length sequence of the HHV-7 US21 includes an amino acid sequence having 80% or more identity to the amino acid sequence shown in SEQ ID NO: 61, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HHV-7 US21 is shown as in SEQ ID NO: 61;
preferably, the MHC binding domain of the HHV-7 US21 includes an amino acid sequence having 80% or more identity to the amino acid sequence shown in SEQ ID NO: 62, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the HHV-7 US21 is shown as in SEQ ID NO: 62;
preferably, the ERAD degradation domain of the HHV-7 US21 includes an amino acid sequence having 80% or more identity to the amino acid sequence shown in SEQ ID NO: 63, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HHV-7 US21 is shown as in SEQ ID NO: 63;

5. The multi-functional complex according to any one of claims 1-4, wherein the UT module further comprises (ii) a binding protein molecular domain targeting MHC I and/or MHC II or a functional variant thereof;
preferably, the MHC I- and/or MHC II-targeted binding protein molecular domain or a functional variant thereof is a binding protein molecular domain targeting HLA or a functional variant thereof;
preferably, the binding protein molecular domain targeting MHC I and/or MHC II or a functional variant thereof further is derived from a viral endoplasmic reticulum protein that inhibits the expression of MHC molecule or promotes its degradation; preferably, the viral endoplasmic reticulum glycoprotein is selected from HCMV US6, HSV ICP47, CPXV012, HPV E6/E7, EBV BNFL2a or BHV UL49.5; preferably, the binding protein molecular domain targeting MHC I and/or MHC II, or its functional variant, contains a TAP binding domain;
preferably, the full-length sequence of the HCMV US6 includes an amino acid sequence having 80% or more identity to the amino acid sequence shown in SEQ ID NO: 64, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV US6 is shown as in SEQ ID NO: 64;
preferably, the TAP binding domain of the HHV-7 US6 includes an amino acid sequence having 80% or more identity to the amino acid sequence shown in SEQ ID NO: 65, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the HHV-7 US6 is shown as in SEQ ID NO: 65;
preferably, the full-length sequence of the HSV ICP47 includes an amino acid sequence having 80% or more identity to the amino acid sequence shown in SEQ ID NO: 66, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HSV ICP47 is shown as in SEQ ID NO: 66;
preferably, the TAP binding domain of the HSV ICP47 includes an amino acid sequence having 80% or more identity to the amino acid sequence shown in SEQ ID NO: 67, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the HSV ICP47 is shown as in SEQ ID NO: 67;
preferably, the full-length sequence of the CPXV012 includes an amino acid sequence having 80% or more identity to the amino acid sequence shown in SEQ ID NO: 68, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the CPXV012 is shown as in SEQ ID NO:68;
preferably, the TAP binding domain of the CPXV012 includes an amino acid sequence having 80% or more identity to the amino acid sequence shown in SEQ ID NO: 69, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the CPXV012 is shown as in SEQ ID NO: 69;
preferably, the full-length sequence of the EBV BNFL2a includes an amino acid sequence having 80% or more identity to the amino acid sequence shown in SEQ ID NO: 70, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the EBV BNFL2a is shown as in SEQ ID NO: 70;
preferably, the TAP binding domain of the EBV BNFL2a includes an amino acid sequence having 80% or more identity to the amino acid sequence shown in SEQ ID NO: 71, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the EBV BNFL2a is shown as in SEQ ID NO: 71;
preferably, the full-length sequence of the BHV UL49.5 includes an amino acid sequence having 80% or more identity to the amino acid sequence shown in SEQ ID NO: 72, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the BHV UL49.5 is shown as in SEQ ID NO: 72;
preferably, the TAP binding domain of the BHV UL49.5 includes an amino acid sequence having 80% or more identity to the amino acid sequence shown in SEQ ID NO: 73, preferably an amino acid sequence having an identity of 85%, 90%, 95%, 96%, 97%, 98%, or 99% or more, and more preferably an amino acid sequence having an identity of 98%, or 99% or more; and the amino acid sequence of the TAP binding domain of the BHV UL49.5 is shown as in SEQ ID NO: 73;
preferably, the binding protein molecular domain targeting MHC I and/or MHC II or a functional variant thereof is derived from viral glycoproteins that degrade MHC and/or MHC II molecules; preferably, the viral glycoproteins are selected from HCMV glycoprotein US2, US3, US11 or US10, adenovirus E3-19K, or HHV-7 US21;
preferably, the full-length sequence of the US2 includes an amino acid sequence having 80% or more identity to the amino acid sequence shown in SEQ ID NO: 74, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the US2 is shown as in SEQ ID NO:74;
preferably, the HLA binding domain of the US2 includes an amino acid sequence having 80% or more identity to the amino acid sequence shown in SEQ ID NO: 75, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US2 is shown as in SEQ ID NO: 75;
preferably, the ERAD degradation domain of the US2 includes an amino acid sequence having 80% or more identity to the amino acid sequence shown in SEQ ID NO: 76, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the US2 is shown as in SEQ ID NO: 76;
preferably, the full-length sequence of the US3 includes an amino acid sequence having 80% or more identity to the amino acid sequence shown in SEQ ID NO: 77, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the US3 is shown as in SEQ ID NO:77;
preferably, the HLA binding domain of the US3 includes an amino acid sequence having 80% or more identity to the amino acid sequence shown in SEQ ID NO: 78, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US3 is shown as in SEQ ID NO: 78;
preferably, the ERAD degradation domain of the US3 includes an amino acid sequence having 80% or more identity to the amino acid sequence shown in SEQ ID NO: 79, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the US3 is shown as in SEQ ID NO: 79;
preferably, the full-length sequence of the US11 includes an amino acid sequence having 80% or more identity to the amino acid sequence shown in SEQ ID NO: 80, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the US11 is shown as in SEQ ID NO:80;
preferably, the HLA binding domain of the US11 includes an amino acid sequence having 80% or more identity to the amino acid sequence shown in SEQ ID NO: 81, preferably an amino acid sequence having an identity of 85%, 90%, 95%, 96%, 97%, 98%, or 99% or more, and more preferably an amino acid sequence having an identity of 98%, or 99% or more; and the amino acid sequence of the HLA binding domain of the US 11 is shown as in SEQ ID NO: 81;
preferably, the ERAD degradation domain of the US 11 includes an amino acid sequence having 80% or more identity to the amino acid sequence shown in SEQ ID NO: 82, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the US11 is shown as in SEQ ID NO: 82;
preferably, the binding protein molecular domain targeting MHC I and/or MHC II or a functional variant thereof further includes a viral protein that directively inhibits or degrades the NK target protein of MICA, MICB, ULBP1, ULBP2, ULBP3, ULBP4, ULBP5 or ULBP6; preferably, the viral protein is selected from HCMV UL16, UL141, UL142, or adenovirus E3-19K;
preferably, the full-length sequence of the HCMV UL16 includes an amino acid sequence having 80% or more identity to the amino acid sequence shown in SEQ ID NO: 83, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL16 is shown as in SEQ ID NO: 83;
preferably, the NK target protein binding domain of the HCMV UL16 includes an amino acid sequence having 80% or more identity to the amino acid sequence shown in SEQ ID NO: 84, preferably an amino acid sequence having an identity of 85%, 90%, 95%, 96%, 97%, 98%, or 99% or more, and more preferably an amino acid sequence having an identity of 98%, or 99% or more; and the amino acid sequence of the NK target protein binding domain of the HCMV UL16 is shown as in SEQ ID NO: 84;
preferably, the ERAD degradation domain of the HCMV UL16 includes an amino acid sequence having 80% or more identity to the amino acid sequence shown in SEQ ID NO: 85, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV UL16 is shown as in SEQ ID NO: 85;
preferably, the full-length sequence of the HCMV UL141 includes an amino acid sequence having 80% or more identity to the amino acid sequence shown in SEQ ID NO: 86, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the HCMV UL141 is shown as in SEQ ID NO: 86;
preferably, the NK target protein binding domain of the HCMV UL141 includes an amino acid sequence having 80% or more identity to the amino acid sequence shown in SEQ ID NO: 87, preferably an amino acid sequence having an identity of 85%, 90%, 95%, 96%, 97%, 98%, or 99% or more, and more preferably an amino acid sequence having an identity of 98%, or 99% or more; and the amino acid sequence of the NK target protein binding domain of the HCMV UL141 is shown as in SEQ ID NO: 87;
preferably, the ERAD degradation domain of the HCMV UL141 includes an amino acid sequence having 80% or more identity to the amino acid sequence shown in SEQ ID NO: 88, preferably an amino acid sequence having an identity of 85%, 90%, 95%, 96%, 97%, 98%, or 99% or more, and more preferably an amino acid sequence having an identity of 98%, or 99% or more; and the amino acid sequence of the ERAD degradation domain of the HCMV UL141 is shown as in SEQ ID NO: 88;
preferably, the full-length sequence of the HCMV UL142 includes an amino acid sequence having 80% or more identity to the amino acid sequence shown in SEQ ID NO: 89, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length of the HCMV UL142 is shown as in SEQ ID NO: 89;
preferably, the MICA and ULBP3 binding domain of the HCMV UL142 includes an amino acid sequence having 80% or more identity to the amino acid sequence shown in SEQ ID NO: 90, preferably an amino acid sequence having an identity of 85%, 90%, 95%, 96%, 97%, 98%, or 99% or more, and more preferably an amino acid sequence having an identity of 98%, or 99% or more; and the amino acid sequence of the MICA and ULBP3 binding domain of the HCMV UL142 is shown as in SEQ ID NO: 90;
preferably, the Golgi residence domain of the HCMV UL142 includes an amino acid sequence having 80% or more identity to the amino acid sequence shown in SEQ ID NO: 91, preferably an amino acid sequence having an identity of 85%, 90%, 95%, 96%, 97%, 98%, or 99% or more, and more preferably an amino acid sequence having an identity of 98%, or 99% or more; and the amino acid sequence of the Golgi residence domain of the HCMV UL142 is shown as in SEQ ID NO: 91;
preferably, the binding protein molecular domain targeting MHC I and/or MHC II or a functional variant thereof further includes a viral protein that transports MHC I molecules from the Golgi apparatus to lysosomes for degradation; preferably, the viral protein is selected from HIV Nef, HIV Vpu, HHV-7 U21, HHV-8 KK3, HHV-8 KK5, MHV-68 MK3, and HTLV-1 p12;
preferably, the HIV Nef includes an amino acid sequence having 80% or more identity to the amino acid sequence shown in SEQ ID NO: 92, preferably an amino acid sequence having an identity of 85%, 90%, 95%, 96%, 97%, 98%, or 99% or more, and more preferably an amino acid sequence having an identity of 98%, or 99% or more; and the amino acid sequence of the HIV Nef is shown as in SEQ ID NO:92;
preferably, the HIV Vpu includes an amino acid sequence having 80% or more identity to the amino acid sequence shown in SEQ ID NO: 93, preferably an amino acid sequence having an identity of 85%, 90%, 95%, 96%, 97%, 98%, or 99% or more, and more preferably an amino acid sequence having an identity of 98%, or 99% or more; and the amino acid sequence of the HIV Vpu is shown as in SEQ ID NO:93;
preferably, the HHV-8 KK3 includes an amino acid sequence having 80% or more identity to the amino acid sequence shown in SEQ ID NO: 94, preferably an amino acid sequence having an identity of 85%, 90%, 95%, 96%, 97%, 98%, or 99% or more, and more preferably an amino acid sequence having an identity of 98%, or 99% or more; and the amino acid sequence of HHV-8 KK3 is shown as in SEQ ID NO: 94;
preferably, the HHV-8 KK5 includes an amino acid sequence having 80% or more identity to the amino acid sequence shown in SEQ ID NO: 95, preferably an amino acid sequence having an identity of 85%, 90%, 95%, 96%, 97%, 98%, or 99% or more, and more preferably an amino acid sequence having an identity of 98%, or 99% or more; and the amino acid sequence of the HHV-8 KK5 is shown as in SEQ ID NO: 95;
preferably, the MHV-68 MK3 includes an amino acid sequence having 80% or more identity to the amino acid sequence shown in SEQ ID NO: 96, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHV-68 MK3 is shown as in SEQ ID NO: 96;
preferably, the HTLV-1 p12 includes an amino acid sequence having 80% or more identity to the amino acid sequence shown in SEQ ID NO: 97, preferably an amino acid sequence having an identity of 85%, 90%, 95%, 96%, 97%, 98%, or 99% or more, and more preferably an amino acid sequence having an identity of 98%, or 99% or more; and the amino acid sequence of the HTLV-1 p12 is shown as in SEQ ID NO: 97;
preferably, the binding protein molecular domain targeting MHC I and/or MHC II or a functional variant thereof further includes a viral protein that mediates the return of MHC- polypeptide molecules from the Golgi apparatus to the endoplasmic reticulum and promotes their degradation; preferably, the viral protein comprises an MHC binding structure and a KDEL receptor binding domain; preferably, the viral protein is Cowpox virus protein CPXV203;
the UT module further includes an MHC-binding structure and a KDEL receptor-binding domain of a viral functional protein, which mediate the return of MHC-polypeptide molecules from the Golgi apparatus to the endoplasmic reticulum and promotes their degradation, such as the CPXV203 of vaccinia virus proteins;
preferably, the full-length sequence of the vaccinia virus protein CPXV203 includes an amino acid sequence having 80% or more identity to the amino acid sequence shown in SEQ ID NO: 98, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the vaccinia virus protein CPXV203 is shown as in SEQ ID NO:98;
preferably, the MHC binding domain of the vaccinia virus protein CPXV203 includes an amino acid sequence having 80% or more identity to the amino acid sequence shown in SEQ ID NO: 99, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the vaccinia virus protein CPXV203 is shown as in SEQ ID NO: 99;
preferably, the KDEL receptor binding domain of the vaccinia virus protein CPXV203 includes an amino acid sequence having 80% or more identity to the amino acid sequence shown in SEQ ID NO: 100, preferably an amino acid sequence having an identity of 85%, 90%, 95%, 96%, 97%, 98%, or 99% or more, and more preferably an amino acid sequence having an identity of 98%, or 99% or more; and the amino acid sequence of the KDEL receptor binding domain of the vaccinia virus protein CPXV203 is shown as in SEQ ID NO: 100;
preferably, the full-length sequence of the vaccinia virus protein CPXV203 includes an amino acid sequence having 80% or more identity to the amino acid sequence shown in SEQ ID NO: 101, preferably an amino acid sequence having an identity of 85%, 90%, 95%, 96%, 97%, 98%, or 99% or more, and more preferably an amino acid sequence having an identity of 98%, or 99% or more; and the amino acid sequence of the full-length sequence of the vaccinia virus protein CPXV203 is shown as in SEQ ID NO: 101;
preferably, the MHC binding domain of the vaccinia virus protein CPXV203 includes an amino acid sequence having 80% or more identity to the amino acid sequence shown in SEQ ID NO: 102, preferably an amino acid sequence having an identity of 85%, 90%, 95%, 96%, 97%, 98%, or 99% or more, and more preferably an amino acid sequence having an identity of 98%, or 99% or more; and the amino acid sequence of the MHC binding domain of the vaccinia virus protein CPXV203 is shown as in SEQ ID NO: 102;
preferably, the KDEL receptor binding domain of the vaccinia virus protein CPXV203 includes an amino acid sequence having 80% or more identity to the amino acid sequence shown in SEQ ID NO: 103, preferably an amino acid sequence having an identity of 85%, 90%, 95%, 96%, 97%, 98%, or 99% or more, and more preferably an amino acid sequence having an identity of 98%, or 99% or more; and the amino acid sequence of the KDEL receptor binding domain of the vaccinia virus protein CPXV203 is shown as in SEQ ID NO: 103.

6. The multi-functional complex according to any one of claims 1-5, wherein the multi-functional complex further comprises (4) a cadaptorhimeric adaptor module and/or a receptor module with targeted killing activity against tumor cells;
(4) the chimeric adaptor module comprises: (i) a tumor-targeted extracellular recognition domain, (ii) a transmembrane domain, and (iii) an intracellular signal transduction domain; optionally, a hinge or linker is included among the tumor-targeted extracellular recognition domain, the transmembrane domain and/or the intracellular signal transduction domain;
preferably, the tumor-targeted extracellular recognition domain of the chimeric adaptor is selected from a tumor antigen-specific binding domain, a tumor microenvironment target antigen binding domain, and/or a chemokine receptor targeting tumor microenvironment;
preferably, the tumor-targeted extracellular recognition domain is selected from an antibody capable of targeting a tumor-associated antigen or a functional fragment thereof, a TCR or the combination thereof. The functional fragment of the antibody is selected from Fd, Fv, Fab, Fab', F(ab') 2, Fv (scFv), single-chain antibody (scFv) or nanobody, double-stranded antibody, triple-stranded antibody and tetra-stranded antibody;
preferably, the transmembrane domain of the chimeric adaptor module adaptoris selected from the NK cell activating receptor transmembrane domain, DAP10 transmembrane domain, DAP12 transmembrane domain, CD8 transmembrane domain, CD28 transmembrane domain, CD4 transmembrane domain, 4-1BB transmembrane domain, OX40 transmembrane domain, ICOS transmembrane domain, CTLA-4 transmembrane domain, PD-1 transmembrane domain, LAG-3 transmembrane domain, 2B4 transmembrane domains, and BTLA transmembrane domain, as well as the combination thereof; preferably, the NK cell activating receptor is selected from NKG2D, NKG2C, NKG2E, NKG2F, NKG2H, CD94, KIR2DL4, KIR2DS1, KIR2DS2, KIR2DS4, KIR3DS1, natural cytotoxic receptor, TRAIL, DNAM-1, CD16a, 2B4, NTB-A, CRACC, and NKp80; preferably, the natural cytotoxic receptor is selected from Nkp46, Nkp44, and Nkp30;
preferably, the intracellular signal transduction domain of the chimeric adaptor includes an intracellular signal transduction domain of the NK cell activating receptor and/or a co-stimulatory signal transduction domain;
preferably, the NK cell-activating receptor is selected from NKG2D, NKG2C, NKG2E, NKG2F, NKG2H, CD94, KIR2DL4, KIR2DS1, KIR2DS2, KIR2DS4, KIR3DS1, a natural cytotoxic receptor, TRAIL, DNAM-1, CD16a, 2B4, NTB-A, CRACC, orNKp80;
preferably, the intracellular signal domain further comprises a co-stimulatory signal transduction domain; preferably, the co-stimulatory signal transduction domain is selected from the group consisting of T cell co-stimulatory signal transduction domain, comprising, but not limited to, the intracellular signal domain derived from MHC class I molecules, TNF receptor proteins, immunoglobulin-like proteins, cytokine receptors, integrin proteins, lymphocyte activation signal molecules (SLAM proteins), activated NK cell receptors, BTLA, Toll ligand receptors, OX40, CD2, CD7, CD16, CD27, CD28, CD30, CD40, CD38, CD35, CD79A, CD79B, CDS, ICAM-1, LFA-1, (CD11a/CD18), 4-1BB(CD137), B7-H3, CDS, ICAM-1, ICOS(CD278), GITR, BAFFR, LIGHT, HVEM(LIGHTR), KIRDS2, SLAMF7, NKp80(KLRF1), NKp44, NKp30, NKp46, CD19, CD4, CD8α, CD8β, IL2Rβ, IL2Rγ, IL7Rα, ITGA4, VLA1, CD49a, ITGA4, IA4, CD49D, ITGA6, VLA-6, CD49f, ITGAD, CD11d, ITGAE, CD103, ITGAL, CD11a, LFA-1, ITGAM, CD11b, ITGAX, CD11c, ITGB1, CD29, ITGB2, CD18, LFA-1, ITGB7, NKG2D, NKG2C, NCR, DAP10, DAP12, TNFR2, TRANCE/RANKL, DNAM1(CD226), SLAMF4(CD244, 2B4), CD84, CD96(Tactile), CEACAM1, CRTAM, Ly9(CD229), CD160(BY55), PSGL1, CD100SEMA4D), CD69, SLAMF6(NTB-A, Ly108), SLAM (SLAMF1, CD150, IPO-3), BLAME (SLAMF8), SELPLG (CD162), LTBR, LAT, GADS, SLP-76, PAG/Cbp, CD19a, CD83-specific binding ligands, CARD11, FcRa, FcRp, FcRy, Fyn, HVEM, ICOS, Lck, LAG3, LAT, LRP, NOTCH1, Wnt, OX40, ROR2, Ryk, SLAMF1, Slp76, pTa, TCRa, TCRp, TRIM, ZAP70, and PTCH2; more preferably, the co-stimulatory signal transduction domain is selected from the NKG2D intracellular signal domain, DAP10 intracellular signal domain, DAP12 intracellular signal domain, NCR intracellular signal domain, CD28 intracellular signal domain, 4-1BB intracellular signal domain, OX40 intracellular signal domain, and ICOS intracellular signal domain;
the receptor module with targeted killing activity against tumor cells includes: (i) an extracellular recognition domain targeting tumor antigen; (ii) a transmembrane domain; and (iii) an intracellular co-stimulatory signal transduction domain; (iv) a T cell activation signal transduction domain (ITAM); optionally, a hinge or a linker is contained between the extracellular recognition domain targeting tumor antigen, the transmembrane domain, the intracellular co-stimulatory signal transduction domain, and/or the T cell activation signal transduction domain (ITAM);
the transmembrane domain of the receptor module with targeted killing activity against tumor cells is selected from CD8 transmembrane domain, α and/or β chain transmembrane domain of T cell receptor, CD28 transmembrane domain, CD3ε transmembrane domain, CD45 transmembrane domain, CD4 transmembrane domain, CD5 transmembrane domain, CD8 transmembrane domain, CD9 transmembrane domain, CD16 transmembrane domain, CD22 transmembrane domain, CD33 transmembrane domain, CD37 transmembrane domain, CD64 transmembrane domain, CD80 transmembrane domain, CD86 transmembrane domain, CD134 transmembrane domain, CD137 transmembrane domain, CD154 transmembrane domain, GITR transmembrane domain, and combinations thereof;
the T cell activation signal transduction domain is derived from CD3ξ, common FcRy (FCER1G), FcyRIIa, FcRβ, CD3γ, CD3δ, CD3ε, CD5, CD22, CD79a, CD79b, CD278 ("ICOS"), FcεRI CD66d, DAP10 and DAP12 and other intracellular signal transduction domains;
preferably, the linker is a flexible linker; preferably, the flexible linker includes the amino acid sequence indicated (Gly(x)Ser(y)n, wherein n is an integer from 1 to 10, and x and y are independently integers from 0 to 10, provided that x and y are not both 0; and more preferably, the linker includes an amino acid sequence (Gly4Ser)2 indicated in SEQ ID NO: 104 or an amino acid sequence (Gly3 Ser)2 indicated in SEQ ID NO: 105;
preferably, the linker is a hinge; preferably, the hinge is an IgG1 hinge or an IgG4 hinge;
preferably, the IgG1 hinge includes an amino acid sequence having 80% or more identity to the amino acid sequence shown in SEQ ID NO: 106, preferably an amino acid sequence having an identity of 85%, 90%, 95%, 96%, 97%, 98%, or 99% or more, and more preferably an amino acid sequence having an identity of 98%, or 99% or more; and the amino acid sequence of the IgG1 hinge is shown as in SEQ ID NO: 106;
preferably, the IgG4 hinge includes an amino acid sequence having 80% or more identity to the amino acid sequence shown in SEQ ID NO: 107, preferably an amino acid sequence having an identity of 85%, 90%, 95%, 96%, 97%, 98%, or 99% or more, and more preferably an amino acid sequence having an identity of 98%, or 99% or more; and the amino acid sequence of the IgG4 hinge is shown as in SEQ ID NO: 107;
preferably, a cleavable peptide is included between the NK activating receptor module, CNK signal transduction module, and/or UT module comprises; and the cleavable peptide is selected from T2A peptide, GSG-T2A peptide, E2A peptide, GSG-E2A peptide, F2A peptide, GSG-F2A peptide, P2A peptide, or GSG-P2A peptide;
preferably, the T2A includes an amino acid sequence having 80% or more identity to the amino acid sequence shown in SEQ ID NO: 108, preferably an amino acid sequence having an identity of 85%, 90%, 95%, 96%, 97%, 98%, or 99% or more, and more preferably an amino acid sequence having an identity of 98%, or 99% or more; and the amino acid sequence of the T2Ais shown as in SEQ ID NO: 108;
preferably, the amino acid sequence of the GSG-T2A peptide is shown in SEQ ID NO: 109.
preferably, the P2A includes an amino acid sequence having 80% or more identity to the amino acid sequence shown in SEQ ID NO: 110, preferably an amino acid sequence having an identity of 85%, 90%, 95%, 96%, 97%, 98%, or 99% or more, and more preferably an amino acid sequence having an identity of 98%, or 99% or more; and the amino acid sequence of the P2A is shown as in SEQ ID NO: 110;
preferably, the amino acid sequence of the GSG-P2A peptide is shown in SEQ ID NO: 111;
preferably, the E2A includes an amino acid sequence having 80% or more identity to the amino acid sequence shown in SEQ ID NO: 112, preferably an amino acid sequence having an identity of 85%, 90%, 95%, 96%, 97%, 98%, or 99% or more, and more preferably an amino acid sequence having an identity of 98%, or 99% or more; and the amino acid sequence of the E2A is shown as in SEQ ID NO: 112;
preferably, the amino acid sequence of the GSG-E2A peptide is shown in SEQ ID NO:113;
preferably, the F2A includes an amino acid sequence having 80% or more identity to the amino acid sequence shown in SEQ ID NO: 114, preferably an amino acid sequence having an identity of 85%, 90%, 95%, 96%, 97%, 98%, or 99% or more, and more preferably an amino acid sequence having an identity of 98%, or 99% or more; and the amino acid sequence of the F2A is shown as in SEQ ID NO: 114;
preferably, the amino acid sequence of the GSG-F2A peptide is shown in SEQ ID NO: 115;
preferably, the multi-functional complex includes an amino acid sequence having 80% or more identity to the amino acid sequence shown in SEQ ID NO: 117, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 117;
preferably, the multi-functional complex includes an amino acid sequence having 80% or more identity to the amino acid sequence shown in SEQ ID NO: 121, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 121;
preferably, the multi-functional complex includes an amino acid sequence having 80% or more identity to the amino acid sequence shown in SEQ ID NO: 123, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 123;
preferably, the multi-functional complex includes an amino acid sequence having 80% or more identity to the amino acid sequence shown in SEQ ID NO: 125, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 125;
preferably, the multi-functional complex includes an amino acid sequence having 80% or more identity to the amino acid sequence shown in SEQ ID NO: 126, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 126;
preferably, the multi-functional complex includes an amino acid sequence having 80% or more identity to the amino acid sequence shown in SEQ ID NO: 127, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 127;
preferably, the multi-functional complex includes an amino acid sequence having 80% or more identity to the amino acid sequence shown in SEQ ID NO: 128, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 128;
preferably, the multi-functional complex includes an amino acid sequence having 80% or more identity to the amino acid sequence shown in SEQ ID NO: 129, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 129;
preferably, the multi-functional complex includes an amino acid sequence having 80% or more identity to the amino acid sequence shown in SEQ ID NO: 130, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 130;
preferably, the multi-functional complex includes an amino acid sequence having 80% or more identity to the amino acid sequence shown in SEQ ID NO: 131, preferably an amino acid sequence having an identity of 85%, 90%, 95%, 96%, 97%, 98%, or 99% or more, and more preferably an amino acid sequence having an identity of 98%, or 99% or more; and the amino acid sequence of the multi-functional complex is shown as in SEQ ID NO: 131.

7. A nucleic acid molecule encoding any of the multi-functional complexes of any one of claims 1-6; preferably, the nucleic acid molecule is DNA or RNA;
preferably, the RNA is mRNA;
preferably, the nucleic acid molecule includes a nucleotide sequence having 80% or more identity to the nucleotide sequence shown in SEQ ID NO: 118, preferably a nucleotide sequence having an identity of 85%, 90%, 95%, 96%, 97%, 98%, or 99% or more, and more preferably a nucleotide sequence having an identity of 98%, or 99% or more; and the nucleotide sequence of the nucleic acid molecule is shown in the SEQ ID NO: 118;
preferably, the nucleic acid molecule includes a nucleotide sequence having 80% or more identity to the nucleotide sequence shown in SEQ ID NO: 122, preferably a nucleotide sequence having an identity of 85%, 90%, 95%, 96%, 97%, 98%, or 99% or more, and more preferably a nucleotide sequence having an identity of 98%, or 99% or more; and the nucleotide sequence of the nucleic acid molecule is shown in the SEQ ID NO: 122;
preferably, the nucleic acid molecule includes a nucleotide sequence having 80% or more identity to the nucleotide sequence shown in SEQ ID NO: 124, preferably a nucleotide sequence having an identity of 85%, 90%, 95%, 96%, 97%, 98%, or 99% or more, and more preferably a nucleotide sequence having an identity of 98%, or 99% or more; and the nucleotide sequence of the nucleic acid molecule is shown in the SEQ ID NO: 124.

8. An expression vector containing the nucleic acid of claim 7;
preferably, the vector is selected from: plasmid, cosmid, viral vector, RNA vector, or linear or circular DNA or RNA molecules;
preferably, the viral vector is selected from: retrovirus, adenovirus, parvoviruse (e.g., adeno-associated virus), coronavirus, negative-strand RNA virus such as orthomyxovirus (e.g., influenza virus), rhabdovirus (e.g., rabies and vesicular stomatitis viruses), paramyxovirus (e.g., Machi and Sendai), positive-strand RNA virus such as picornavirus, alphavirus and double-stranded DNA virus, wherein the double-stranded DNA virus compirses adenovirus, herpesvirus (e.g., herpes simplex virus types 1 and 2, Epstein-Barr virus, cytomegalovirus) and pox Virus (e.g., vaccinia virus, fowlpox virus, and canarypox virus), Norwalk virus, togavirus, flavivirus, reovirus, papovavirus, hepadnavirus, baculovirus, and hepatitis virus;
preferably, the retroviral vector is selected from the group consisting of avian leukoproliferative-sarcoma, mammalian C-type virus, B-type virus, D-type virus, HTLV-BLV collection, Lentivirus, bubble virus;
preferably, the lentiviral vector is selected from HIV-1, HIV-2, SIV, FIV, BIV, EIAV, CAEV or ovine demyelinating leukoencephalitis lentivirus;
preferably, the NK activating receptor module, CNK signal transduction module and/or UT module may regulate expression under the same promoter of the same vector, or under different promoters, or in a plurality of vectors;
preferably, the vector is lentiviral vector, a cleavable peptide-coding gene is included between the gene encoding NK activating receptor module, CNK signal transduction module and/or UT module; preferably, the cleavable peptide is a 2A linker; the 2A linker is selected from T2A, P2A, E2A and F2A;
preferably, the vector further includes a promoter; and preferably, the promoter is an EF1α promoter or CMV promoter.

9. An immune cell, comprising the nucleic acid of claim 8 or the expression vector of claim 8; preferably, the immune cell is selected from T cell, NKT cell, NK cell, B cell, monocyte, macrophage, etc.

10. A method for producing immune cells, comprising introducing the nucleic acid of claim 8 or the expression vector of claim 8 into cells by methods selected from the following: electroporation, acoustic perforation, gene gun (e.g., gene gun with Au- particles), lipid transfection, polymer transfection, nanoparticles or polymer complexes.

11. A pharmaceutical composition comprising the multi-functional complex of any one of claims 1-6, the nucleic acid of claim 7, the expression vector of claim 8, the immune cell of claim 9 and/or the immune cell produced by the method of claim 10, and pharmaceutically acceptable carriers.

12. Use of the multi-functional complex of any one of claims 1-6, the nucleic acid of claim 7, the expression vector of claim 8, the immune cell of claim 9, the immune cell produced by the method of claim 10, and/or the pharmaceutical composition of claim 11, in the manufacture of a medicine for treating diseases.

13. A method of treating diseases, comprising administering the multi-functional complex of any one of claims 1-6, the nucleic acid of claim 7, the expression vector of claim 8, the immune cell of claim 9, and/or the pharmaceutical composition of claim 10 to a subject;
preferably, the diseases include various solid tumors and hematological tumors, viral infectious diseases, autoimmune diseases;
preferably, the solid tumor is selected from the group consisting of nervous system tumors, head and neck tumors, chest tumors, digestive system tumors, genitourinary system tumors, soft tissue and skin tumors, bone tumors, etc.;
preferably, the nervous system tumors comprise diffuse glioma, diffuse astrocytoma and anaplastic astrocytoma, glioblastoma, oligodendroglioma, oligoastrocytoma, diffuse Gliomas, other astrocytomas, ependymomas, neuronal and mixed neuronal-glial tumors, medulloblastoma, other embryonal tumors, schwannomas, meningiomas, solitary fibrous tumors and hemangiopericytoma, etc.;
preferably, the head and neck tumors comprise malignant tumors of the nasal cavity and sinuses, nasopharyngeal cancer, oral cancer, laryngeal cancer, salivary gland tumors, intracranial tumors, thyroid cancer, tongue cancer, etc.;
preferably, the thoracic tumors comprise lung cancer, esophageal cancer, cardia cancer, breast cancer, mediastinal tumors, etc.;
preferably, the digestive system tumors comprise gastric cancer, colorectal cancer, sigmoid colon and rectal cancer, liver cancer, pancreatic cancer and periampullary cancer, biliary tract cancer, malignant tumors of the small intestine, etc.;
preferably, the genitourinary system tumors comprise kidney cancer, prostate cancer, bladder cancer, testicular cancer, penile cancer, cervical cancer, endometrial cancer, ovarian cancer, etc.;
preferably, the soft tissue and skin tumors comprise malignant fibrous histiocytoma, rhabdomyosarcoma, synovial sarcoma, malignant melanoma of the skin, etc.;
preferably, the bone tumors comprise osteosarcoma, Ewing's sarcoma, etc.;
preferably, the colon cancer is colonic adenoma;
preferably, the breast cancer is a triple-negative breast cancer cell;
preferably, the liver cancer is hepatocellular carcinoma;
preferably, the disease is a hematological tumor selected from the group consisting of leukemia, lymphoma (HL), multiple myeloma (MM), myelodysplastic syndrome (MDS), etc.;
preferably, the leukemia is B-cell acute lymphoblastic leukemia, T-cell acute lymphoblastic leukemia, acute myeloid leukemia, etc.;
preferably, the viral infectious diseases comprise: respiratory viral diseases, gastrointestinal viral diseases, liver viral diseases, skin and mucous membrane viral diseases, ocular viral diseases, central nervous system viral diseases, lymphocytic viral diseases, insect-borne viral diseases, lentivirus infection diseases, etc.;
preferably, the respiratory viral diseases comprise infections of rhinovirus, adenovirus, respiratory syncytial virus, parainfluenza virus, and coronavirus; influenza; mumps, etc.;
preferably, the gastrointestinal viral diseases comprise polio; cooksackie virus infection; ECHO virus infection; and viral gastroenteritis including rotavirus gastroenteritis, Norwalk virus gastroenteritis, adenovirus gastroenteritis, astrovirus gastroenteritis, coronavirus gastroenteritis and calicivirus gastroenteritis, etc.;
preferably, the liver viral diseases comprise viral hepatitis A, viral hepatitis B, viral hepatitis C, viral hepatitis D, viral hepatitis E, Epstein-Barr viral hepatitis, and cytomegalovirus hepatitis;
preferably, the skin and mucous membrane viral diseases comprise measles, rubella, infantile acute rash, chickenpox and herpes zoster, smallpox, herpes simplex virus infection, rabies and foot-and-mouth disease, etc.;
preferably, the ocular viral diseases comprise epidemic keratoconjunctivitis, follicular conjunctivitis and herpetic keratoconjunctivitis, etc.;
preferably, the central nervous system viral diseases comprise Japanese encephalitis, western equine encephalitis, eastern equine encephalitis, St. Louis encephalitis, Venezuelan equine encephalitis, Murray Valley encephalitis, Californian encephalitis, forest encephalitis and lymphocytic choroid plexus meningitis, etc.;
preferably, the lymphocytic viral diseases comprise infectious mononucleosis, cytomegalovirus infection and acquired immunodeficiency syndrome, etc.;
preferably, the insect-borne viral diseases comprise viral hemorrhagic fevers including epidemic hemorrhagic fever, yellow fever, Crimean-Congo hemorrhagic fever, Rift Valley fever, Argentine hemorrhagic fever, Bolivian hemorrhagic fever, Lassa fever, Omu Scrubs hemorrhagic fever, Marburg disease and Ebola hemorrhagic fever, etc.; dengue fever and dengue hemorrhagic fever; West Nile fever; Colorado tick heat transfer; and sandfly fever, etc.;
preferably, the lentiviral infection diseases comprise subacute sclerosing panencephalitis, Kuru disease, progressive multifocal leukoencephalopathy and subacute spongiform encephalopathy (corticostriatal spinal cord degeneration), etc.;
preferably, the autoimmune diseases comprise organ-specific autoimmune diseases and systemic autoimmune diseases;
preferably, the organ-specific autoimmune diseases comprise chronic lymphocytic thyroiditis, hyperthyroidism, insulin-dependent diabetes mellitus, myasthenia gravis, ulcerative colitis, pernicious anemia with chronic atrophic gastritis, pulmonary hemorrhage nephritic syndrome, vulgaris Pemphigus, pemphigoid, primary biliary cirrhosis, multiple sclerosis, acute idiopathic polyneuritis, etc.;
preferably, the systemic autoimmune diseases comprise systemic lupus erythematosus, rheumatoid arthritis, systemic vasculitis, scleroderma, pemphigus, dermatomyositis, mixed connective tissue disease, autoimmune hemolytic anemia, thyroid autoimmune diseases, ulcerative colitis, etc.

14. A method of stimulating an immune response in a subject, comprising administering an effective amount of the multi-functional complex of any one of claims 1-6, the nucleic acid of claim 7, the expression vector of claim 8, the immune cell of claim 9, the immune cell produced by the method of claim 10, and/or the pharmaceutical composition of claim 11 to the subject.
